# FASCICULE DE BREVET EUROPEEN

(11) **EP 2 847 154 B1**
(45) Date de publication et mention de la délivrance du brevet: **30.05.2018**
(21) Numéro de dépôt: 13714296.4
(22) Date de dépôt: 04.04.2013
(51) Int. Cl.: A61K 51/04, C07C 67/03

(54) **RADIOTRACEUR NOTAMMENT POUR LE CANCER**
RADIOTRACER, INSBESONDERE FÜR KREBS
RADIOTRACER, IN PARTICULAR FOR CANCER

(30) Priorité: 04.04.2012 FR 1253109
(43) Date de publication de la demande: 18.03.2015
(73) Titulaire: Université de Lorraine, 54052 Nancy Cedex (FR); Centre National de la Recherche Scientifique (CNRS), 75794 Paris cedex 16 (FR)
(72) Inventeur: CHAPLEUR, Yves, F-54000 Nancy (FR); LAMANDE-LANGLE, Sandrine, F-54500 Vandoeuvre-lès-Nancy (FR); COLLET, Charlotte, F-54113 Gye (FR); CHRETIEN, Françoise, F-54500 Vandoeuvre-lès-Nancy (FR)
(74) Mandataire: Gevers SA
(86) Numéro de dépôt international: PCT/EP2013/057143
(87) Numéro de publication internationale: WO 2013/150117

(56) Documents cités:
- WO-A1-97/48711
- WO-A1-2009/014203
- WO-A2-2007/119108
- THEODORE POSTERNAK: "Recherches dans la série des cyclites VII. Sur la cyclite des moules (mytilite) et sur quelques substances voisines", HELVETICA CHIMICA ACTA, vol. 27, no. 1, 1944, pages 457-468, XP055040019, ISSN: 0018-019X, DOI: 10.1002/hlca.19440270156
- ANDRE; GIDDEY ET AL: "Recherches dans la s�rie des cyclitols XLV. Action des diazoalcanes sur la pentahydroxy-2,4,6/3,5-cyclohexanone et sur son dérivé penta-O-acétylé;. I. Etude des spiro-époxydes", HELVETICA CHIMICA ACTA, vol. 57, no. 7, 6 novembre 1974 (1974-11-06), pages 1963-1974, XP055040023, ISSN: 0018-019X, DOI: 10.1002/hlca.19740570709
- HORNE G ET AL: "First derivatives of myo-inositol 1,4,6-trisphosphate modified at positions 2 and 3: structural analogues of d-myo-inositol 1,4,5-trisphosphate", CARBOHYDRATE RESEARCH, PERGAMON, GB, vol. 339, no. 1, 2 janvier 2004 (2004-01-02), pages 51-65, XP004476656, ISSN: 0008-6215, DOI: 10.1016/J.CARRES.2003.09.008
- SHU SHU YANG ET AL.: "Synthesis of 2-C-fluoromethyl-myo-inositol", SYNTHETIC COMMUNICATIONS, vol. 19, no. 9, 1988, pages 899-905, XP009163374,
- MORIS MARC-ANTOINE ET AL: "SYNTHESIS OF (+/-)-2-O-[4'-(N-9''-PURINYL)BUTYL] MYO-INOSITOL 1,4,5-TRIS(PHOSPHATE), A POTENT FULL AGONIST AT THE D-MYO-INOSITOL 1,4,5-TRIS(PHOSPHATE) RECEPTOR", JOURNAL OF MEDICINAL CHEMISTRY, AMERICAN CHEMICAL SOCIETY, US, vol. 48, no. 4, 24 février 2005 (2005-02-24), pages 1251-1255, XP009076309, ISSN: 0022-2623, DOI: 10.1021/JM049458S
- TH. POSTERNAK ET AL: "Recherches dans la série des cyclitols XXIX. Synthèses du (+/-)-laminitol et du (-)-laminitol", HELVETICA CHIMICA ACTA, vol. 44, no. 7, 1961, pages 2080-2085, XP55040069, ISSN: 0018-019X, DOI: 10.1002/hlca.19610440733

## Description

### Domaine technique

La présente invention concerne de nouveaux radiotraceurs, en particulier pour la maladie d'Alzheimer ou pour les cancers.

Par « radiotraceur », on entend une molécule portant un atome radioactif émetteur de positions. Plus particulièrement, il s'agit d'une molécule portant un atome de fluor radioactif.

### Etat de la technique

La maladie d'Alzheimer est un désordre neurodégénératif progressif caractérisée par une accumulation anormale de plaques dans le cerveau, celles-ci étant composées en partie de peptides β-amyioïdes qui sont caractéristiques de cette maladie.

Afin de diagnostiquer la maladie d'Alzheimer, il a été développé des marqueurs non invasifs, spécifiques de ces plaques, comme un radiotraceur pour l'imagerie médicale par Tomographie par Emission de Positons (TEP). La TEP est une technique d'imagerie médicale non-invasive qui permet, grâce à une caméra spécifique, de suivre le devenir *in vivo* d'une molécule préalablement marquée avec un radioisotope, injectée à un patient. A ce jour plusieurs radiotraceurs sont en développement pour diagnostiquer la maladie d'Alzheimer par TEP. Cependant, afin de diagnostiquer les patients plus rapidement et avec une meilleure précision, il serait avantageux de proposer d'autres molécules qui seraient spécifiques des plaques β-amyloïdes et qui seraient plus efficace en imagerie TEP.

Il a été montré récemment que le *scyllo*-inositol ainsi que quelques uns de ses dérivés limitent les agrégats de l'amyloïde β.

La demande WO 2009/014203 décrit l'utilisation du 1-désoxy-1-fluoro-scyllo-inositol et certains de ses dérivés fluorés comme radiotraceurs potentiels de la maladie d'Alzheimer. Ces composés sont marqués avec du fluor-18. Toutefois des études récentes ont montré que ces composés franchissent difficilement la barrière hémato-encéphalique. Ils sont donc difficilement utilisables.

La demande WO2007/119108 décrit des dérivés d'inositol comprenant un radical de scyllo-inositol ainsi que d'autres substituants. Ces peuvent servir à prévenir et/ou traiter des maladies caractérisées par un repliement protéique anormal, une agrégation protéique, ou une formation, un dépôt, une accumulation ou une persistance d'amyloïde témoins de maladies dégénératives, Toutefois ce document ne décrit pas leur utilité comme radiotraceurs ni leur utilité dans le diagnostic des cancers.

Par conséquent, les inventeurs ont souhaité mettre au point des dérivés originaux des inositols, susceptibles de passer la barrière hématoencéphalique afin de les utiliser comme radiotraceurs, en particulier pour la maladie d'Alzheimer.

On recherche également des radiotraceurs efficaces pour diagnostiquer les cancers par TEP.

### Divulgation de l'invention

A cet effet, et conformément à la présente invention, il est proposé un composé de formule (I) : 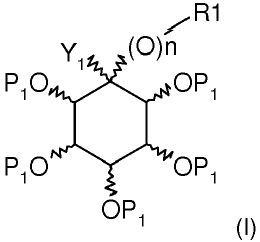 dans laquelle
- n est un entier égal à 0 ou 1,
- R1 représente
   ∘ quand n=0, une chaîne alkyle de 3 à 6 atomes de carbone de préférence linéaire, une chaîne alkènyle, alkylène, une chaîne hydroxyalkyle linéaire de 2 à 6 atomes de carbone, la fonction hydroxyle étant éventuellement protégée ou l'oxygène de cette fonction étant substitué de manière à former un groupement partant, une chaîne halogénoalkyle, l'halogène étant radioactif ou non, de 2 à 6 atomes de carbone, ou un groupe fluorométhyle, le fluor étant radioactif ou non
   ∘ quand n=1, une chaîne monohydroxyalkyle de 1 à 6 atomes de carbone, la fonction hydroxyle, en position terminale, étant éventuellement protégée ou l'oxygène de cette fonction étant substitué de manière à former un groupement partant, ou monohalogénoalkyle, l'halogène, en position terminale, étant radioactif ou non, de 1 à 6 atomes de carbone, l'halogène étant différent du brome
- P1 représente
   ∘ quand n=0 : chacun un hydrogène ou chacun un groupement protecteur identique de la fonction hydroxyle,
   ∘ quand n=1 et R1 est une chaîne monohydroxyalkyle : chacun un hydrogène ou chacun un groupement protecteur identique de la fonction hydroxyle,
- quand n=1 et R1 est une chaîne monohalogénoalkyle : chacun un hydrogène ou chacun un groupement protecteur identique de la fonction hydroxyle, Y1 représente :
   ∘ quand n=0, un groupement OP₁, où P₁ est tel que défini ci-avant, ou,
   ∘ quand n=1, un atome d'hydrogène,
à l'exception des composés 2-fluorométhyl-myo-inositols non radiomarqués pour lesquels P1, identique ou différent, représente un atome d'hydrogène ou un groupement protecteur benzyle.

La présente invention concerne aussi l'utilisation de ce composé comme médicament.

### Brève description des dessins

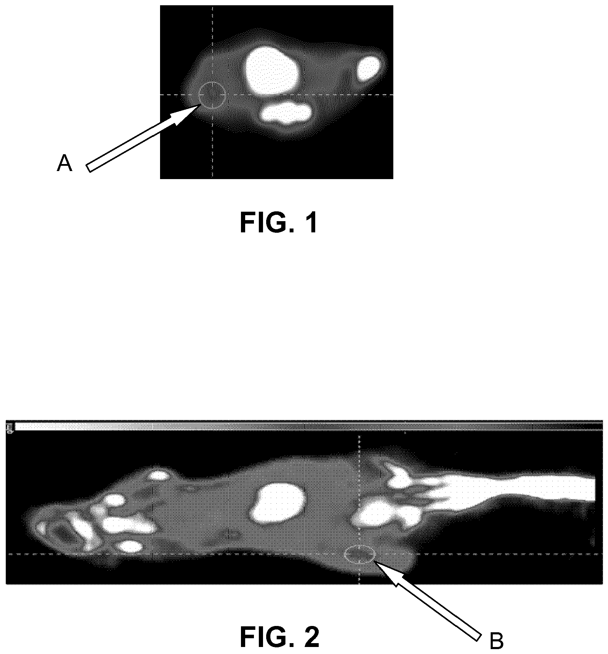

L'invention sera mieux comprise à la lecture de la description qui va suivre, de modes de réalisation, donnés à titre d'exemples et faits en référence aux dessins dans lesquels les figures 1 et 2 représentent des images, respectivement axiale et dans le plan sagital, acquises au MicroTEP après injection d'un composé selon l'invention chez une souris.

### Mode(s) de réalisation de l'invention

Les radiotraceurs selon l'invention, portent un bras espaceur de type alkyl ou éther d'une longueur variable de préférence de trois à quatre ou cinq atomes, fluoré à l'extrémité. Ils sont obtenus par substitution au fluor-18 de précurseurs de marquage tosylés. L'introduction de ce bras augmente le caractère lipophile des composés et permet un franchissement plus aisé de la barrière hématoencéphalique. De plus, les dérivés choisis sont estérifiés ou portent des hydroxyles libres afin de moduler leur solubilité.

Les composés synthétisés sont donc des dérivés d'inositols. De préférence, ils sont de configuration *myo-* ou *scyllo*-inositol. Ces deux diastéréoisomères diffèrent par la configuration relative d'un seul hydroxyle. Lorsque celui-ci est en position axiale, on parle de configuration *myo*-inositol et le carbone portant cet hydroxyle est le carbone n°2. Lorsque celui-ci est en position équatoriale, on parle de configuration *scyllo*-inositol et le carbone portant cet hydroxyle est le carbone n°1.

L'invention concerne donc des composés de formule (I) : 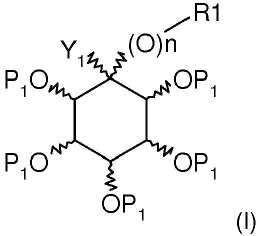 dans laquelle
- n est un entier égal à 0 ou 1,
- R1 représente
   ∘ quand n=0, une chaîne alkyle de 3 à 6 atomes de carbone de préférence linéaire, une chaîne alkényle, alkynyle, une chaîne hydroxyalkyle linéaire de 2 à 6 atomes de carbone, de préférence de 2 à 5 atomes de carbone, préférentiellement de 2 à 4 atomes de carbone, et plus préférentiellement de 2 à 3 atomes de carbone, la fonction hydroxyle étant éventuellement protégée ou l'oxygène de cette fonction étant substitué de manière à former un groupement partant, une chaîne halogénoalkyle, l'halogène étant radioactif ou non, de 2 à 6 atomes de carbone, de préférence de 2 à 5 atomes de carbone, préférentiellement de 2 à 4 atomes de carbone et plus préférentiellement de 2 à 3 atomes de carbone, ou un groupe fluorométhyle, le fluor étant radioactif ou non,
   ∘ quand n=1, une chaîne monohydroxyalkyle de 1 à 6 atomes de carbone, de préférence de 2 à 5 atomes de carbone, préférentiellement de 2 à 4 atomes de carbone, et plus préférentiellement de 2 à 3 atomes de carbone, la fonction hydroxyle, en position terminale, étant éventuellement protégée ou l'oxygène de cette fonction étant substitué de manière à former un groupement partant, ou monohalogénoalkyle, l'halogène, en position terminale, étant radioactif ou non, de 1 à 6 atomes de carbone, de préférence de 2 à 5 atomes de carbone, préférentiellement de 2 à 4 atomes de carbone, et plus préférentiellement de 2 à 3 atomes de carbone, l'halogène étant différent du brome,
- P₁, représente
   ∘ quand n=0 : chacun un hydrogène ou chacun un groupement protecteur identique de la fonction hydroxyle
   ∘ quand n=1 et R1 est une chaîne monohydroxyalkyle : chacun un hydrogène ou chacun un groupement protecteur identique de la fonction hydroxyle, quand n=1 et R1 est une chaîne monohalogénoalkyle : chacun un hydrogène ou chacun un groupement protecteur identique de la fonction hydroxyle
- Y1 représente :
   ∘ quand n=0, un groupement OP₁, où P₁ est tel que défini ci-avant, ou,
   ∘ quand n=1, un atome d'hydrogène,
à l'exception des composés 2-fluorométhyl-*myo*-inositols non radiomarqués pour lesquels P₁, identique ou différent, représente un atome d'hydrogène ou un groupement protecteur benzyle.

Ainsi, sont exclus de l'invention les composés penta-acétyl-iodo-isomytilite, penta-acétyl-bromo-isomytilite, chlorométhyl-C(2)-myo-inositol, bromométhyl-C(2)-myo-inositol, l'éthyle-C(2)-myo-inositol et son dérivé hexa-acétylé, l'hepta-O-actéyl-hydroxyéthyl C(2)-myo-inositol, l'hepta-O-actéyl-hydroxypropyl C(2)-myo-inositol, l'hepta-O-actéyl-hydroxy-n-butyl-C(2)-myo-inositol, l'hepta-O-actéyl-hydroxyéthyl C(2)-scyllo-inositol, l'hepta-O-actéyl-hydroxypropyl C(2)-scyllo-inositol, l'hepta-O-actéyl-hydroxy-n-butyl-C(2)-scyllo-inositol, _{DL}-2,5-di-O-benzyl-3-O-(2-benzyloxy-ethyl)-myo-inositol, _{DL}-2,5-di-O-benzyl-3-O-(3-benzyloxy-propyl)-myo-inositol, _{DL}-2,5-di-O-benzyl-3-O-(4-benzyloxy-butyl)-myo-inositol, L(-)-1-O-(6-bromo-hexyl)-2,3,4,5,6-penta-O-benzyl-myo-inositol, trifluorométhyl-scyllo-inositol, 1,4-di-O-benzyl-2-O-(4-hydroxy-butyl)-myo-inositol.

Dans toute la présente demande, les gammes de valeur indiquée s'entendent bornes incluses.

Le composé de formule (I) est de préférence un dérivé inositol, choisi parmi le groupe constitué par un *myo*-inositol, un *scyllo*-inositol, un neo-inositol, un *D-chiro-*inositol, un L-*chiro*-inositol, un *epi*-inositol, un *muco*-inositol, un *allo*-inositol et un *cis*-inositol.

Dans la présente description, une fonction « protégée » signifie une fonction chimique réactive pour laquelle tout ou partie de sa réactivité est masquée par un groupe fonctionnel « protecteur ». Un groupe « protecteur » est donc un groupe fonctionnel introduit dans une molécule à partir d'une fonction chimique réactive pour masquer tout ou partie de sa réactivité. Un groupe protecteur est choisi par exemple parmi le groupe comprenant des esters d'acides carboxyliques, des éthers d'alkyles ou de benzyles.

Un groupe partant ou nucléofuge est un substituant qui a la capacité de se détacher d'une molécule. Un groupe partant est choisi par exemple parmi le groupe comprenant les groupes tosyle, mésyle, un atome de chlore, brome et iode.

Avantageusement, le groupement OP₁ est un hydroxyle, un acétate ou un o-benzyle.

De préférence, la fonction halogène ou hydroxyle de R1 est terminale.

Il est bien entendu que le composé de formule (I) n'inclut pas les composés bicycliques.

Plus particulièrement, le composé selon l'invention est de formule (II): 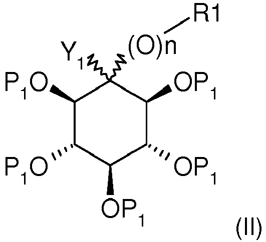 dans laquelle n, R1, P₁ et Y₁ sont tels que définis ci-avant.

Ce composé de formule (II) correspond à un dérivé de type *myo-* ou *scyllo*-inositol.

Selon un premier aspect de l'invention, le groupement R1 du composé de formule (I) ou (II) est une chaîne fluoroalkyle, le fluor étant en position terminale sur la chaîne alkyle et non radioactif. Préférentiellement, la chaîne alkyle comporte 1 à 3 atomes de carbone, ou de 2 à 6 atomes de carbone, de préférence de 2 à 5 atomes de carbone, plus préférentiellement de 2 à 4 atomes de carbone, et encore plus préférentiellement de 2 à 3 atomes de carbone.

Une chaine iodoalkyle répondant aux mêmes définitions peut également être utilisée. Plus généralement, pour tous les composés décrits dans la présente demande comprenant du fluor radioactif ou non, le fluor peut être remplacé par de l'iode radioactif ou non. L'utilisation d'iode permet le suivi du radiotraceur par une gamma caméra (SPECT).

Les composés selon le premier aspect correspondent aux composés obtenus en synthèse dite « froide » (sans radiomarquage).

Selon un second aspect de l'invention, le groupement R1 du composé de formule (I) ou (II) est une chaîne fluoroalkyle, le fluor étant en position terminale sur la chaîne alkyle et radioactif. Préférentiellement, la chaîne alkyle comporte 1 à 3 atomes de carbone ou de 2 à 6 atomes de carbone, de préférence de 2 à 5 atomes de carbone, plus préférentiellement de 2 à 4 atomes de carbone, et encore plus préférentiellement de 2 à 3 atomes de carbone.

Une chaine iodoalkyle répondant aux mêmes définitions peut également être utilisée.

Les composés selon le second aspect correspondent aux radiotraceurs obtenus en synthèse dite «chaude» (avec radiomarquage).

Avantageusement, selon ces deux aspects, P₁ est un hydrogène.

Selon un troisième aspect de l'invention, le groupement R1 du composé de formule (I) ou (II) est une chaîne monohydroxyalkyle de 1 à 6 carbones, de préférence de 2 à 6 atomes de carbone, de préférence de 2 à 5 atomes de carbone, plus préférentiellement de 2 à 4 atomes de carbone, et encore plus préférentiellement de 2 à 3 atomes de carbone, dans laquelle la fonction hydroxyle est terminale et l'oxygène de cette fonction est substitué de manière à former un groupement partant.

Les composés selon le troisième aspect correspondent aux précurseurs des radiotraceurs avant l'étape de radiomarquage.

Préférentiellement, le groupement partant est un tosylate et P₁ est un groupement protecteur, par exemple acétyle.

Le composé selon l'invention comporte un groupement (O)ₙ-R1 en position axiale. Alternativement, le groupement (O)ₙ-R1 est en position équatoriale.

Les composés préférés selon l'invention sont le 2-O-(fluoropropyl)-*myo*-inositol (composé 70) ou le 1-O-(fluoropropyl)-*scyllo*-inositol (composé 71), ainsi que les composés correspondant dans lesquels le fluor est radiomarqué 18F.

L'invention divulgue également les procédés d'obtention des composés selon l'invention, tels que définis dans les revendications ci-jointes. Toute description en dehors de la portée desdites revendications est donnée à titre illustratif uniquement.

La description concerne plus particulièrement un procédé de protection spécifique de la fonction hydroxyle tertiaire d'un composé de formule (III): 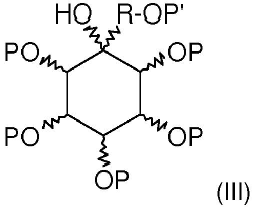 dans laquelle :
- R représente une chaîne alkyle ou alkényle de 2 à 6 atomes de carbone, de préférence une chaîne alkyle de 2 à 5 atomes de carbone, préférentiellement une chaîne alkyle de 2 à 4 atomes de carbone, et plus préférentiellement de 2 à 3 atomes de carbone,
- P' représente un groupement protecteur ou un substituant, qui associé à l'oxygène forme un groupement partant,
- P représente un groupement protecteur, préférentiellement un groupement acétyle,
- par acétylation à l'acétate d'isopropényle.

Préférentiellement, le groupement partant OP' est un groupement trityle ou tosyle. Avantageusement, lorsque la chaîne alkyle R comporte 3 atomes de carbone, OP' est un groupement trityle et lorsque la chaîne alkyle R comporte 2 atomes de carbone, OP' est un groupement tosyle.

De préférence, le composé protégé est le composé de formule (IV) : 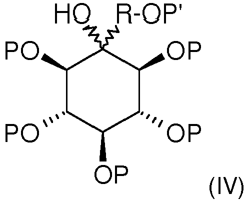 dans lequel R, P' et P sont tels que définis ci-avant.

Avantageusement, le solvant utilisé dans ce procédé est l'acétate d'isopropényle avec une quantité catalytique d'acide para-toluène sulfonique (ApTS). La réaction est avantageusement conduite à reflux pendant 1 à 5h, de préférence environ 2h.

La description concerne également un procédé de synthèse d'un composé de formule (V) : 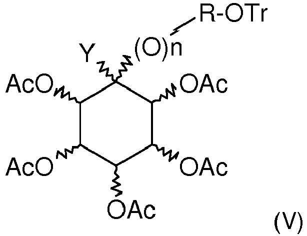 à partir d'un composé de formule (VI): 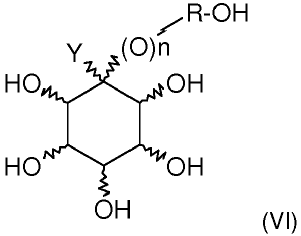 dans lesquelles :
- R représente une chaîne alkyle ou alkényle de 1 à 6 atomes de carbone, préférentiellement une chaîne alkyle de 1 à 3 atomes de carbone, ou préférentiellement une chaîne alkyle de 2 à 6 atomes de carbone, de préférence de 2 à 5 atomes de carbone, plus préférentiellement de 2 à 4 atomes de carbone, et encore plus préférentiellement de 2 à 3 atomes de carbone,
- n est un entier égal à 0 ou 1,
- Y représente :
   ∘ quand n=0, un groupement OH, ou,
   ∘ quand n=1, un atome d'hydrogène,
- Ac représente un groupement acétyle, et
- Tr représente un groupement trityle,
par tritylation de la fonction hydroxyle primaire puis acétylation des fonctions hydroxyles secondaires.

Plus particulièrement, le procédé permet la synthèse d'un composé de formule (VII) : 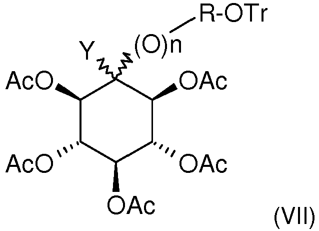 à partir d'un composé de formule (VIII) 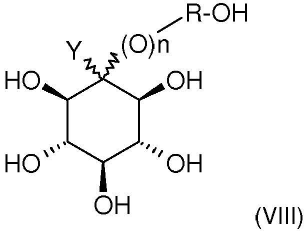 dans lesquelles R, n, Y, Ac et Tr sont tels que définis ci-avant.

Avantageusement, la tritylation est effectuée à l'aide de chlorure de trityle, dans un solvant organique avec un catalyseur, par exemple la pyridine et une quantité catalytique de 4-N,N-diméthylaminopyridine (DMAP), puis l'acétylation est effectuée avec de l'anhydride acétique.

Le composé de formule (VI) ci-avant, peut être synthétisé par déprotection d'un composé de formule (IX) 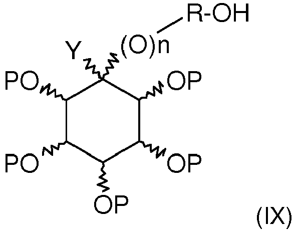 dans lesquelles :
- R représente une chaîne alkyle ou alkényle de 1 à 6 carbones, préférentiellement une chaîne alkyle de 1 à 3 atomes de carbone, ou préférentiellement une chaîne alkyle de 2 à 6 atomes de carbone, de préférence de 2 à 5 atomes de carbone, plus préférentiellement de 2 à 4 atomes de carbone, et encore plus préférentiellement de 2 à 3 atomes de carbone,
- n est un entier égal à 0 ou 1,
- Y représente :
   ∘ quand n=0, un groupement OH, ou,
   ∘ quand n=1, un atome d'hydrogène, et
- P représente un groupement protecteur, préférentiellement un groupement benzyle.

Plus particulièrement, ce procédé de déprotection permet la synthèse d'un composé de formule (VIII), à partir d'un composé de formule (X) : 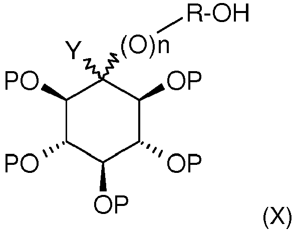 dans lesquelles R, n, Y et P sont tels que définis ci-avant.

Avantageusement, la déprotection est effectuée par réaction avec le dihydrogène H₂, en présence d'un catalyseur au palladium, tel que du Pd(OH)₂, dans un solvant. De préférence, le solvant est un mélange de dichlorométhane, de méthanol et d'eau. Avantageusement, la réaction est effectuée sous une pression de 2 à 5 bars, de préférence environ 3 bars.

Un composé de formule (XI) : 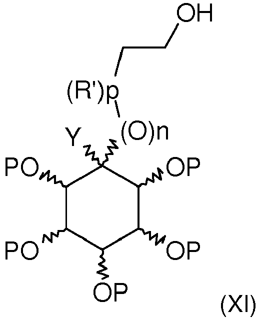 est synthétisé par hydroxylation d'un composé de formule (XII) 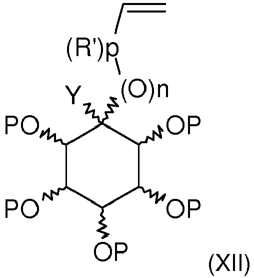 dans lesquelles :
- n est un entier égal à 0 ou 1,
- Y représente :
   ∘ quand n=0, un groupement OH, ou,
   ∘ quand n=1, un atome d'hydrogène,
- R' représente une chaîne alkyle de 1 à 4 atomes de carbone, préférentiellement une chaîne alkyle à 1 atome de carbone,
- p est un entier compris entre 0 et 1, et
- P représente un groupement protecteur, préférentiellement un groupement benzyle.

Plus particulièrement, le composé de formule (XIII) 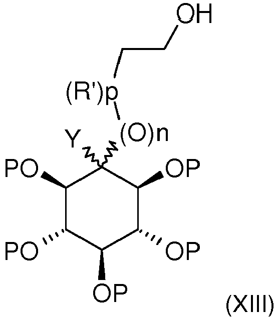 est synthétisé par hydroxylation d'un composé de formule (XIV) 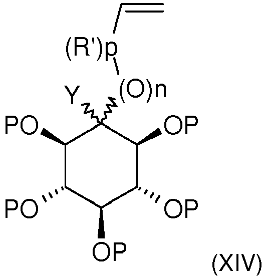 dans lesquelles n, Y, R', p et P sont tels que définis ci-avant.

Avantageusement, l'hydroxylation est préférentiellement effectuée par hydroboration, en présence de borane tel que du diborane, de H₂O₂, et d'une base forte telle que NaOH. De préférence, le solvant est le tétrahydrofurane (THF). La réaction d'hydroxylation peut également être effectuée par ozonolyse et dans ce cas, elle est accompagnée d'une étape réductrice (élimination d'un atome de carbone dans la chaîne alkyle).

L'invention concerne aussi un procédé de synthèse d'un composé de formule (XII), respectivement (XIV), dans lesquelles
- n est un entier égal à 0 ou 1,
- Y représente :
   ∘ quand n=0, un groupement OH, ou
   ∘ quand n=1, un atome d'hydrogène,
- R' représente une chaîne alkyle de 1 atome de carbone,
- p, un entier compris entre 0 et 1, et
- P représente un groupement protecteur, préférentiellement un groupement benzyle, par réaction d'un composé de formule (XV) ou (XV') : 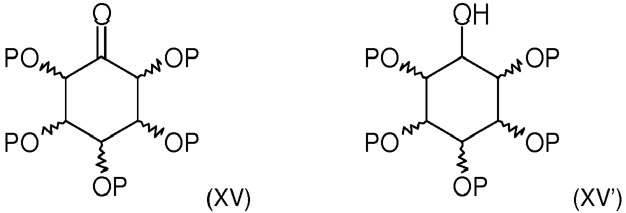 respectivement de formule (XVI) ou (XVI') : 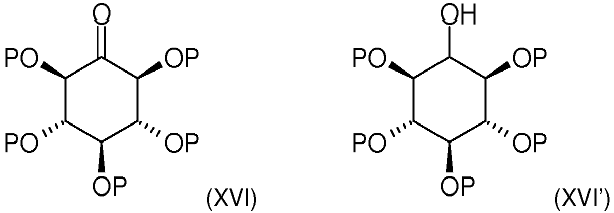
dans laquelle P est tel que décrit ci-avant, avec un organomagnésien de formule CH2=CH-(R')p-MgBr pour le composé (XV) respectivement (XVI), ou avec un bromure d'alkényle de formule CH2=CH-(R')p-Br pour le composé (XV') respectivement (XVI').

Cette réaction s'effectue avec le composé (XV) respectivement (XVI), de préférence dans un solvant organique anhydre tel que du THF anhydre, à température ambiante.

Cette réaction s'effectue avec le composé (XV') respectivement (XVI'), de préférence selon la réaction de Williamson en présence d'hydrure de sodium.

Le composé de formule (XVII) : 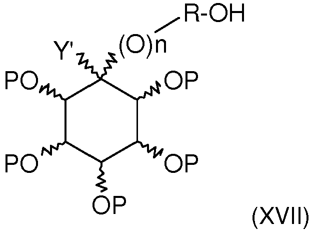 est préparé par detritylation d'un composé de formule (XVIII) : 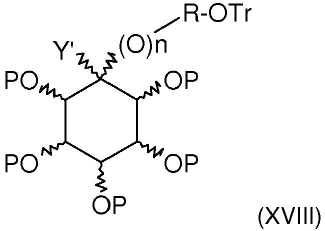 dans lesquelles :
- n est un entier égal à 0 ou 1,
- Y' représente :
   ∘ quand n=0, un groupement OH ou un oxygène portant un groupement protecteur, préférentiellement un groupement acétate, ou,
   ∘ quand n=1, un atome d'hydrogène,
- R représente une chaîne alkyle ou alkényle de 1 à 6 atomes de carbone, préférentiellement une chaîne alkyle de 1 à 3 atomes de carbone, ou préférentiellement une chaîne alkyle de 2 à 6 atomes de carbone, de préférence de 2 à 5 atomes de carbone, plus préférentiellement de 2 à 4 atomes de carbone, et encore plus préférentiellement de 2 à 3 atomes de carbone,
- P représente un groupement protecteur, préférentiellement un groupement acétyle, et
- Tr représente un groupement trityle.

Plus particulièrement, le composé de formule (XIX) : 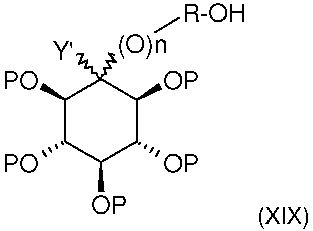 est préparé par detritylation d'un composé de formule (XX) :

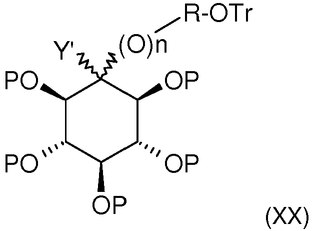

Avantageusement, la détritylation est effectuée en présence de FeCl₃, dans un solvant organique tel que du dichlorométhane.

Le composé de formule (XXI) : 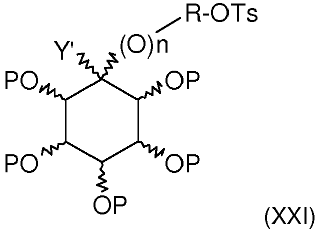 respectivement de formule (XXII) 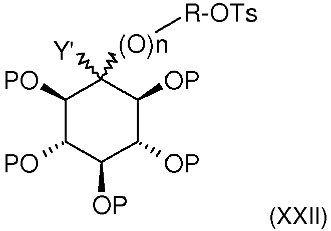 est préparé par tosylation d'un composé de formule (XVII), respectivement d'un composé de formule (XIX), dans lesquelles :
- n est un entier égal à 0 ou 1,
- Y' représente :
   ∘ quand n=0, un groupement OH ou un oxygène portant un groupement protecteur, préférentiellement un groupement acétate, ou,
   ∘ quand n=1, un atome d'hydrogène,
- R représente une chaîne alkyle ou alkényle de 1 à 6 atomes de carbone, préférentiellement une chaîne alkyle de 1 à 3 atomes de carbone, ou préférentiellement une chaîne alkyle de 2 à 6 atomes de carbone, de préférence de 2 à 5 atomes de carbone, plus préférentiellement de 2 à 4 atomes de carbone, et encore plus préférentiellement de 2 à 3 atomes de carbone,
- P représente un groupement protecteur, préférentiellement un groupement acétyle, et
- Ts représente un groupement tosyle.

Avantageusement, la réaction est effectuée en présence de chlorure de tosyle et de triéthylamine (Et₃N) dans un solvant organique tel que du dichlorométhane.

Les radiomarqueurs ont été préalablement préparés en synthèse froide.

Un composé de formule (XXIII) : 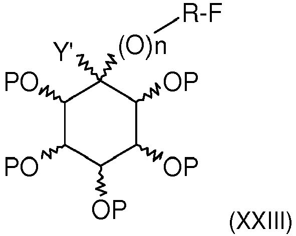 respectivement de formule (XXV) 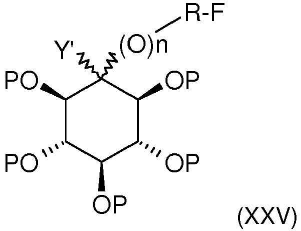 est obtenu par fluoration d'un composé de formule (XXIV) : 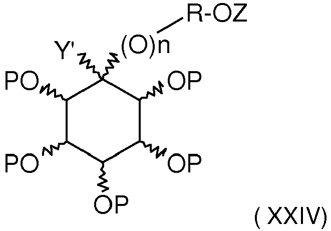 respectivement d'un composé de formule (XXVI) 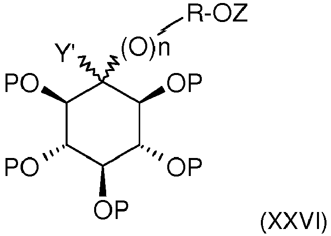 dans lesquelles :
- n est un entier égal à 0 ou 1,
- Y' représente :
   ∘ quand n=0, un oxygène portant un groupement protecteur, préférentiellement un groupement acétate, ou,
   ∘ quand n=1, un atome d'hydrogène,
- R représente une chaîne alkyle de 1 à 6 atomes de carbone, préférentiellement une chaîne alkyle de 1 à 3 atomes de carbone, ou préférentiellement une chaîne alkyle de 2 à 6 atomes de carbone, de préférence de 2 à 5 atomes de carbone, plus préférentiellement de 2 à 4 atomes de carbone, et encore plus préférentiellement de 2 à 3 atomes de carbone,
- F est un atome de fluor non radioactif,
- P représente un groupement protecteur, préférentiellement un groupement acétyle et
- Z représente un groupement tosyle ou un atome d'hydrogène.

Lorsque la chaîne alkyle R comporte 2 atomes de carbone, Y' représente de préférence OH ou OAc, et Z est un groupement tosyle. La réaction de fluoration est alors effectuée de préférence avec du fluorure de potassium, en présence d'éther couronne 18C6, dans un solvant organique tel que l'acétonitrile. De préférence, cette réaction s'effectue à température ambiante pendant 2 heures.

Lorsque la chaîne alkyle R comporte 3 atomes de carbone, Y' représente de préférence un acétate (OAc), et Z=H. La fluoration s'effectue alors par le trifluorure de diéthylaminosulfure (méthode DAST, voir l'exemple 57).

Le trifluorure de diéthylaminosulfure (DAST) est un des agents de fluoration les plus courant permettant de substituer directement un hydroxyle par un fluor par formation d'un groupe partant in situ. Les conditions classiques d'utilisation ont lieu dans le dichlorométhane à température modérée. Il existe d'autres dérivés.

Le procédé de radiomarquage selon l'invention vise la synthèse d'un composé de formule (XXVII) : 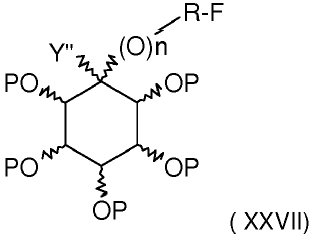 respectivement un composé de formule (XXIX) 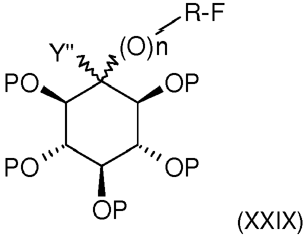 par fluoration d'un composé de formule (XXVIII), 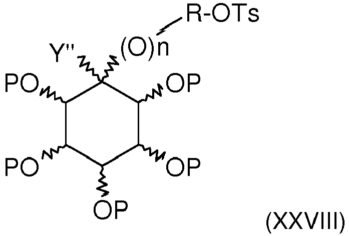 respectivement d'un composé de formule (XXX) 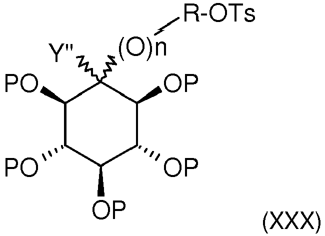 dans lesquelles :
- n est un entier égal à 0 ou 1,
- Y" représente :
   ∘ quand n=0, un groupement OP ou,
   ∘ quand n=1, un atome d'hydrogène,
- R représente une chaîne alkyle de 1 à 6 atomes de carbone, préférentiellement une chaîne alkyle de 1 à 3 atomes de carbone, ou préférentiellement une chaîne alkyle de 2 à 6 atomes de carbone, de préférence de 2 à 5 atomes de carbone, plus préférentiellement de 2 à 4 atomes de carbone, et encore plus préférentiellement de 2 à 3 atomes de carbone,
- F est un atome de fluor radioactif,
- P représente un groupement protecteur, préférentiellement un groupement acétyle et
- Ts représente un groupement tosyle.

La fluoration peut être effectuée par la méthode des bases organiques, des ammoniums, ou par Kryptofix® (voir l'exemple 60).

De préférence, la méthode utilisée est la méthode des bases organiques.

D'autres précurseurs que ceux décrits par la méthode précédente peuvent être utilisés, tel que les époxydes qui sont ouverts par des fluorures par une des trois méthodes citées précédemment, préférentiellement par la méthode des ammonium (voir exemple 60).

La méthode des ammoniums est décrite dans le document suivant : une publication d'Aerts, J et al. *Tetrahedron Lett.* **2010**, *51*, 64.

Une autre méthode consiste à piéger les fluorures sur une cartouche QMA préalablement modifiée avec un ammonium à longue chaîne. Ceci permet de former dans la cartouche une paire d'ion qui est soluble en phase organique. On élue ensuite cette paire d'ions avec un solvant organique qui peut-être de différente nature (DMSO, ACN, DMF). On récupère ainsi environ 90% de l'activité dans environ 1 mL de solvant.

Ces méthodes sont plus faciles et moins longues à mettre en oeuvre que la méthode Kryptofix® décrite ci-dessous. En effet avec la méthode Kryptofix® il était nécessaire pour décrocher les fluorures d'introduire de l'eau afin de pouvoir solubiliser le sel alcalin.

La méthode P₂Et/ **Base de Barton** est décrite dans la littérature : Lemaire, C. et Al. *Angew. Chem., Int. Ed.* **2010,** *49*, 3161, S3161/1-S3161/7.

Cette méthode consiste à éluer les fluorures piégés sur une QMA avec une base forte dans un solvant organiques. Les meilleurs résultats d'élution étant obtenu avec le P₂ET dans de l'acétonitrile à 5500 ppm. En utilisant cette technique l'élution est proche de 100% avec seulement 1mL de solution de ACN contenant 15 µL de P₂Et.

Pour que le marquage soit le plus efficace possible, il faut rajouter une autre base dans le milieu réactionnelle ainsi que de l'acétonitrile sec afin d'avoir une concentration en eau de l'ordre de 3000 ppm. Les meilleurs résultats de marquage ont été obtenus en co-additionnant la Base de Barton (15µL) qui est également une base forte.

La méthode au Kryptofix® est décrite dans la publication de Nickles, R. J.; Gatley, S. J.; Votaw, J. R.; Kornguth, M. L. Int. J. Radiat. Applic. Instrum., Part A 1986, 37, 649.

Elle consiste à éluer les fluorures par une solution aqueuse de K₂CO₃ vers un pilulier contenant Kryptofix K2.2.2. qui activera le fluor. La solution obtenue est ensuite séchée puis le solvant choisi est ensuite additionné (généralement ACN, DMSO).

On forme ainsi un fluorure dont le contre-ion potassium est crypté par le Kryptofix 2.2.2. Le complexe [¹⁸F]F-K2.2.2. est ainsi formé, le fluor-18 n'est plus associé à son contre-ion et est ainsi plus réactif.

Après l'insertion du fluor radioactif ou non, les composés selon l'invention sont déprotégés.

Le procédé de déprotection vise la synthèse d'un composé de formule (XXXI) : 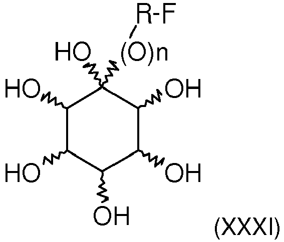 respectivement un composé de formule (XXXII) 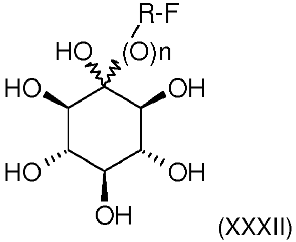 par déprotection d'un composé de formule (XXIII) ou (XXVII), respectivement d'un composé de formule (XXV) ou (XXIX) dans lesquelles :
- n est un entier égal à 0 ou 1,
- Y' représente :
   ∘ quand n=0, un oxygène portant un groupement protecteur, préférentiellement un groupement acétate, ou,
   ∘ quand n=1, un atome d'hydrogène,
- Y" représente :
   ∘ quand n=0, un groupement OP ou,
   ∘ quand n=1, un atome d'hydrogène,
- R est une chaîne alkyle de 1 à 6 atomes de carbone, préférentiellement une chaîne alkyle de 1 à 3 atomes de carbone, ou préférentiellement une chaîne alkyle de 2 à 6 atomes de carbone, de préférence de 2 à 5 atomes de carbone, plus préférentiellement de 2 à 4 atomes de carbone, et encore plus préférentiellement de 2 à 3 atomes de carbone,
- F est un atome de fluor radioactif ou non et
- P désigne un groupement protecteur, préférentiellement un groupement acétyle.

Pour l'obtention des composés radiomarqués ou non, la déprotection peut s'effectuer en présence d'une base forte ou d'un acide fort, en particulier NaOH ou HCI.

Avantageusement, pour l'obtention des composés non radiomarqués, cette déprotection s'effectue en présence d'une base forte dans un solvant, en particulier NaOH dans l'eau.

Enfin, les composés selon l'invention peuvent être utilisés comme médicament. Il s'agit plus particulièrement des composés de formule (I) 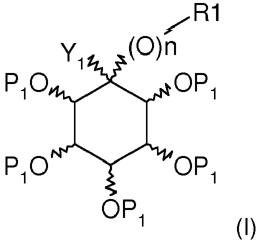 dans laquelle
- n est un entier égal à 0 ou 1,
- R1 représente
   ∘ quand n=0, une chaîne alkyle de 3 à 6 atomes de carbone, de préférence linéaire, une chaîne alkényle, alkynyle, une chaîne hydroxyalkyle linéaire de 2 à 6 atomes de carbone, la fonction hydroxyle étant éventuellement protégée ou l'oxygène de cette fonction étant substitué de manière à former un groupement partant, une chaîne halogénoalkyle, l'halogène étant radioactif ou non, de 2 à 6 atomes de carbone, ou un groupe fluorométhyle, le fluor étant radioactif ou non,
   ∘ quand n=1, une chaîne monohydroxyalkyle de 1 à 6 atomes de carbone, la fonction hydroxyle étant éventuellement protégée ou l'oxygène de cette fonction étant substitué de manière à former un groupement partant, ou monohalogénoalkyle, l'halogène étant radioactif ou non, de 1 à 6 atomes de carbone, l'halogène étant différent du brome,
- P1 représente
   ∘ quand n=0 : chacun un hydrogène ou chacun un groupement protecteur identique de la fonction hydroxyle
   ∘ quand n=1 et R1 est une chaîne monohydroxyalkyle : chacun un hydrogène ou chacun un groupement protecteur identique de la fonction hydroxyle,
- quand n=1 et R1 est une chaîne monohalogénoalkyle : chacun un hydrogène ou chacun un groupement protecteur identique de la fonction hydroxyle,
- Y1 représente :
   ∘ quand n=0, un groupement OP1, où P1 est tel que défini ci-avant, ou,
   ∘ quand n=1, un atome d'hydrogène.
et plus particulièrement, des composés de formule (II) : 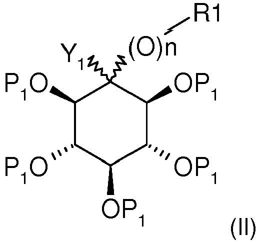 dans laquelle n, R1, P1, et Y1 sont tels que définis ci-avant, pour une utilisation comme médicament.

Avantageusement, les composés de formule (I) : 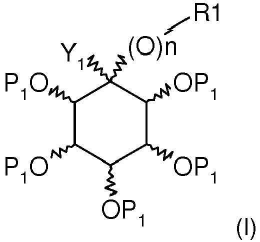 dans laquelle
- n est un entier égal à 0 ou 1,
- R1 représente
- quand n=0, une chaîne fluoroalkyle de 1 à 6 atomes de carbone, de préférence de 2 à 6 atomes de carbone, préférentiellement de 2 à 5 atomes de carbone, plus préférentiellement de 2 à 4 atomes de carbone, et encore plus préférentiellement de 2 à 3 atomes de carbone, le fluor étant en position terminale sur la chaîne alkyle et radioactif, quand n=1, une chaîne fluoroalkyle de 1 à 6 atomes de carbone, de préférence de 2 à 6 atomes de carbone, préférentiellement de 2 à 5 atomes de carbone, plus préférentiellement de 2 à 4 atomes de carbone, et encore plus préférentiellement de 2 à 3 atomes de carbone, le fluor étant en position terminale sur la chaîne alkyle et radioactif, P1 représente un groupement protecteur,
- Y1 représente :
   ∘ quand n=0, un groupement OH, ou,
   ∘ quand n=1, un atome d'hydrogène,
et plus particulièrement les composés de formule (II) 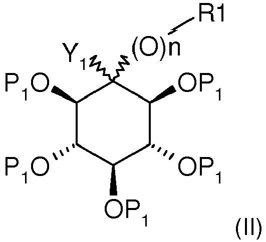 dans laquelle n, R1, P1, et Y1 sont tels que définis ci-avant, peuvent être utilisés comme radiotraceur pour diagnostiquer en particulier les cancers. Le fluor peut être remplacé par de l'iode.

Par « cancers » on entend de préférence, le cancer du sein ou le cancer du cerveau tel que le glyome.

### Mode(s) de réalisation de l'invention

L'invention sera mieux comprise au vu des exemples qui suivent, donnés à titre illustratif.

Les indications générales suivantes ont été appliquées à l'ensemble des exemples.

### Indications générales :

Les solvants ont été préalablement distillés avant usage : le méthanol et l'éthanol sur magnésium, le tétrahydrofurane (THF) sur sodium-benzophénone, le dichlorométhane sur P₂O₅.

L'avancement des réactions est suivi par chromatographie sur couches minces sur des plaques de silice Kieselgel 60 F254 (Merck), contenant un révélateur des groupements fluorescents chromophores dans l'ultraviolet (254 nm). La visualisation est faite sous lumière U.V. et par pulvérisation d'une solution d'acide sulfurique 18 N dans le méthanol suivie d'un chauffage à 270°C.

Les chromatographies sur colonne de silice ont été réalisées sur gel de silice SI 60 avec de la silice DAVISIL LC60 (70-200 µM) GRACE Davison pour les colonnes ouvertes. Certaines séparations ont été faites par chromatographie flash sur un système robotisé utilisant soit des colonnes en verre de 2,5 cm de diamètre et de 30 cm ou 23 cm de longueur Kontes Chromaflex remplie de silice 60 M (40-63 µM) soit des colonnes pré-remplies. Le système de gradient est généré par 2 pompes Gilson modèle 306. Ce dispositif est couplé à un collecteur de fraction et est piloté par le logiciel Gilson 712 HPLC avec de la silice 60 M.

Les points de fusion (Pf) ont été mesurés en tube capillaire à l'aide d'un appareil Tottoli et ne sont pas corrigés.

Les analyses élémentaires ont été réalisées par le service d'analyses de l'UMR 7565 sur un appareil Thermofinnigan FlashEA 1112.

Les spectres infrarouges (IR) ont été enregistrés sur spectromètre Perkin-Elmer Spectrum 100 soit en film sur pastille de NaCl soit en pastille de KBr. Les fréquences indiquées correspondent à des maxima d'absorption et sont exprimés en cm⁻¹.

Les spectres de résonance magnétique nucléaire (RMN) ont été enregistrés soit sur un appareil Bruker DPX 250 MHz pour le proton (1H) à 62.9 MHz pour le carbone (¹³C) et à 235 MHz pour le ¹⁹F, soit sur un appareil Bruker DRX 400 MHz pour le ¹H et à 100,6 MHz pour le ¹³C. Les déplacements chimiques (δ) sont donnés en partie par million (ppm) par rapport au pic résiduel des solvants deutérés (CDCl₃ = 7,26, D₂O = 4,79) pour le ¹H, ceux du ¹³C sont calibrés sur le signal du solvant (CDCl₃ = 77,16) et les spectres fluor sont calibrés par rapport à CFCl₃ comme référence externe. Les constantes de couplages sont données en Hertz (Hz). La multiplicité des signaux est notée avec les désignations : s (singulet), brs (broad singulet), brd (broad doublet), brt (broad triplet), brq (broad quadruplet), d (doublet), dd (doublet de doublet), ddt (doublet de doublet de triplet), dt (doublet de triplet), t (triplet), tt (triplet de triplet), q (quadruplet), qt (quintuplet), m (multiplet) et app (signal apparent). L'attribution des protons et carbones de certains composés a été déterminés par des spectres bidimensionnels (COSY, HMQC, NOESY).

Par ailleurs, dans la partie exemple, la description des spectres RMN est donnée selon la numérotation décrite ci-dessous :

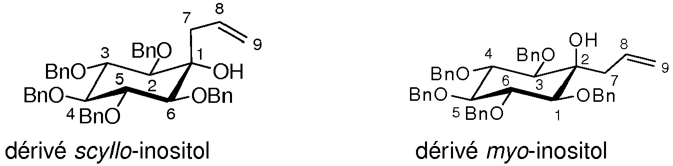

Les spectres de masse (MS) sont enregistrés sur un appareil trio-1000 Thermo Quest fonctionnant par la technique d'ionisation par electrospray, en mode positif (+) ou négatif (-). Les spectres hautes résolution notés HRMS sont enregistrés sur un appareil QTOF Micro Waters.

Pour les radiosynthèses, l'eau enrichie [¹⁸O] est achetée aux laboratoires Cambridge Isotope. Un scanner CCM Bioscan (modèle AR2000) est utilisé pour analyser les composés marqués au fluor-18. :

Les analyses HPLC sont réalisées sur une colonne Alltima NH₂ (GRACE) (250x4,6 mm, 5 µm) en 70/30 CH₃CN/H₂O en isocratique avec un débit de 1L/min :
- en chimie froide, sur une chaîne constituée d'un module de pompage modèle 321 Gilson, une vanne d'injection manuelle Rheodyne, un détecteur à diffusion de lumière ELS 1000 Polymer Lab et d'un détecteur à barrette de diodes SPD-M10A Shimatzu piloté par le logiciel LabSolution (Shimatzu).
- en chimie chaude, sur un système Waters constitué de pompe modèle 616, d'une vanne d'injection manuelle Rheodyne et d'un détecteur à barrette de diodes 996 et d'un détecteur à Scintillation Nal (TL) Eberline piloté par un logiciel Empower.

### Exemple 1 : Préparation du 1,3,4,5,6-penta-O-benzyl-2-désoxy-2-méthylène-myo-inositol 1

A une solution de bromure de méthyltriphénylphosphonium (0,57g, 1,6mmol) dans du THF anhydre (50mL) est ajouté goutte à goutte du butyl lithium (0,8mL d'une solution à 2,5M dans de l'éther diéthylique, 1,6mmol) sous argon à -5°C. Du 1,3,4,5,6-penta-O-benzyl-*myo*-2-inosose (0,5g, 0,8mmol) dilué dans du THF est ajouté goutte à goutte à -5°C. Le mélange réactionnel est agité pendant 1h à température ambiante puis évaporé sous pression réduite. Le produit brut est purifié par chromatographie sur colonne (gel de silice, Hex 100% à Hex/AcOEt: 90/10) pour donner **1** (0,45g, 79% de rendement (rdt)) sous forme de solide blanc.

| | |
|---|---|
| 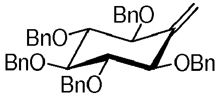 | Solide blanc |
| | **Formule** : C₄₂H₄₂O₅ |
| | **M. Mol.:** 626,78 g/mol |
| | **R_{f}** = 0,56 (Hex/AcOEt: 80/20) |
| | **pf =** 137-138°C |

**IR** film (v, cm⁻¹): 3029, 2902, 1654, 1360. **RMN ¹H (CDCl₃, 400 MHz):** δ (ppm) 3.48 (app t, 2H, *J*_{3,4} = *J*_{4,5} = *J*_{5,6} = *J*_{6,1} = 9.5 Hz, H-4 et H-6), 3.65 (app t, 1H, H-5), 3.92 (d, 2H, H-1 et H-3), 4.69 (d, 2H, *J* = 11.5 Hz, CH₂Ph), 4.80 (d, 2H, *J* = 11.0 Hz, CH₂Ph), 4.83 (d, 2H, *J* = 11.0 Hz, CH₂Ph), 4.91 (s, 2H, CH₂Ph), 4.98 (d, 2H, *J* = 11.0 Hz, CH₂Ph) 5.45-5.50 (brs, 2H, H-7), 7.25-7.45 (m, 25H, H-Ar). **RMN ¹³C (CDCl₃, 100.6 MHz):** 73.7 (2CH₂), 76.2 (2CH₂), 76.5 (CH₂), 81.4 (C-1 et C-3), 83.7 (C-5), 85.6 (C-4 et C-6), 108.6 (C-7), 127.9 (C-Ar), 128.0 (3C-Ar), 128.2 (2C-Ar), 128.2 (3C-Ar), 128.3 (2C-Ar), 128.5 (4C-Ar), 128.8 (7C-Ar), 128.9 (3C-Ar), 138.6 (2Cq-Ar), 138.1 (Cq-Ar), 138.1 (2Cq-Ar), 141.5 (C-2). **MS** (*m*/*z*, ESI), [M + Na]⁺ : 649.

### Exemple 2 : Préparation du 1,3,4,5,6-penta-O-acétyl-2-désoxy-2-méthylène myo-inositol 2

Cette molécule est décrite dans la référence bibliographique : Toyokuni, T. et al. Bulletin of the Chemical Society of Japan 1983, 56(2), 505.

A une solution de 1 (500mg, 0,80mmol) dissout dans du THF anhydre (10mL) est condensé de l'ammoniac dans le montage réactionnel à -78°C, jusqu'à ce que le mélange atteigne un volume d'environ 100mL. Une quantité catalytique de sodium est ajoutée jusqu'à ce que la solution devienne bleue profond. Après 30 min, la réaction est hydrolysée par ajout de méthanol (quelques gouttes). Le mélange réactionnel est laissé remonter à température ambiante dans le but d'évaporer l'ammoniac. De l'acide acétique est ajouté jusqu'à ce que le pH atteigne 4. Les solvants sont évaporés sous pression réduite. Le résidu est dissout dans de la pyridine (200mL), puis de l'anhydride acétique (200mL) et une quantité catalytique de DMAP est additionnée puis la solution est agitée pendant 2h à température ambiante. Le mélange est dilué avec du toluène et les solvants évaporés sous pression réduite. Le produit brut est purifié par flash chromatographie sur colonne (gel de silice, Hex/AcOEt: 60/40) pour donner 2 (305 mg, 98% rdt) sous forme de solide blanc.

| | |
|---|---|
| 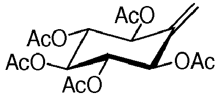 | Solide blanc |
| | **Formule** : C₁₇H₂₂O₁₀ |
| | **M. Mol.:** 386.35g/mol |
| | **R_{f}** = 0.33 (Hex/ AcOEt: 60/40) |
| | **pf** = 159-161 °C |

**IR** film (v, cm⁻¹): 2926, 2854,1760, 1666, 1224. **RMN ¹H (CDCl₃, 250 MHz)**: δ (ppm) 1.99 (s, 3H, Me), 2.01 (s, 6H, 2Me), 2.12 (s, 6H, 2Me), 5.05 (app t, 2H, *J*_{3,4} = *J*_{4,5} = *J*_{5,6} = *J*_{1,6} = 9.5 Hz, H-4 et H-6), 5.20 (app t, 2H, *J*₁₇ = *J*_{3,7} = 2.0 Hz, H-7), 5.30 (app t, 1H, H-5), 5.47 (dt, 2H, H-1 et H-3). **RMN ¹³C (CDCl₃, 62.9 MHz):** δ (ppm) 20.7 (5Me), 70.3 (C-1 et C-3), 70.9 (C-5), 72.6 (C-4 et C-6), 110.4 (C-7), 135.7 (C-2), 169.3 (2CO), 169.8 (CO), 169.9 (2CO). **HRMS** (*m*/*z*, ESI): calculé pour C₁₇H₂₂O₁₀Na, [M+Na]⁺ 409.1105. Trouvé: 409.1106.

### Exemple 3 : Préparation du 1-C-(hydroxyméthyl)-2,3,4,5,6-penta-O-acétyl-scyllo-inositol 3

A une solution de **2** (230mg, 0,59mmol) dans un mélange acétone/eau (6/1) (7mL) est additionné OsO₄ (32mg, cat) et NMO (220mg, cat). Le mélange réactionnel est agité pendant 7h à température ambiante, filtré sur de la Célite®, puis évaporé. Le filtrat est dissout dans AcOEt et de l'eau est additionnée. Le composé est extrait avec AcOEt, 3 fois, lavé avec de l'eau, avec une solution aqueuse saturée en sodium thiosulfate et avec de la saumure. Les phases organiques sont séchées sur MgSO₄, filtrées et les solvants évaporés sous pression réduite. Le produit brut est purifié par chromatographie sur colonne (gel de silice, CH₂Cl₂/MeOH: 98/2) pour donner 3 (150mg, 60% rdt) sous la forme d'une huile.

| | |
|---|---|
| 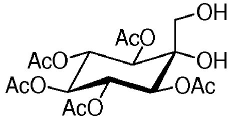 | Huile |
| | **Formule** : C₁₇H₂₄O₁₂ |
| | **M. Mol.:** 420.36 g/mol |
| | **R_{f}** = 0.32 (Hex/ AcOEt: 40/60) |

**IR** film (v, cm⁻¹): 3475, 2940, 1760, 1369, 1224. **RMN ¹H (CDCl₃, 400 MHz):** δ (ppm) 1.99 (s, 3H, Me), 2.01 (s, 6H, 2Me), 2.11 (s, 6H, 2Me), 3.88 (s, 2H, H-7), 5.27 (t, 1H, *J*_{3,4} = *J*_{4,5} = 10.0 Hz, H-4), 5.28 (d, 2H, *J*_{2,3} = *J*_{5,6} = 10.0 Hz, H-2 et H-6), 5.66 (t, 2H, H-3 et H-5). **RMN ¹³C (CDCl₃, 62.9 MHz):** δ (ppm) 20.7 (Me), 20.7 (2Me), 20.8 (2Me), 60.8 (C-7), 70.1 (C-3 et C-5), 71.2 (C-4), 74.6 (C-2), 74.7 (C-2 et C-6), 169.9 (CO), 170.1 (2CO), 170.6 (2CO). **HRMS** (*m*/*z*, ESI): calculé pour C₁₇H₂₄O₁₂Na, [M+Na]⁺ 443.1160. Trouvé: 443.1166.

### Exemple 4: Préparation du 1,1'-anhydro-1-C-(hydroxyméthyl)-2,3,4,5,6-penta-O-acétyl-scyllo-inositol 4

A une solution de **3** (100mg, 0,24mmol) dans du CH₂Cl₂ (3mL) est additionné du chlorure de tosyle (320mg, 1,68mmol), Et₃N (0,15mL) et une quantité catalytique de DMAP. Le mélange réactionnel est agité pendant 12h à température ambiante et les solvants sont évaporés. Le produit brut est purifié par chromatographie sur colonne (gel de silice, Hex/AcOEt : 50/50) pour donner 4 (68 mg, 72% rdt) sous forme d'un solide blanc.

| | |
|---|---|
| 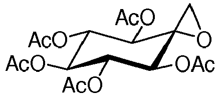 | Solide blanc |
| | Formule : C₁₇H₂₂O₁₁ |
| | M.Mol : 402.35 g/mol |
| | Rf = 0.45 (Hex/ EtOAc: 60/40) |
| | pf = 194-196°C |

**IR** film (v, cm⁻¹) : 1750, 1369, 1227. **NMR ¹H (CDCl₃, 250 MHz):** δ (ppm) 2.01 (s, 3H, Me), 2.01 (s, 6H, 2Me), 2.01 (s, 6H, 2Me), 2.88 (s, 2H, H-7), 5.22 (app t, 2H, *J*_{2,3} = *J*_{3,4} = *J*_{4,5} = *J*_{5,6} = 10.0 Hz, H-3 et H-5), 5.34 (t, 1H, H-4), 5.45 (d, 2H, H-2 et H-5). **NMR ¹³C(CDCl₃, 62.9 MHz):** δ (ppm) 20.5 (2Me), 20.6 (Me), 20.6 (2Me), 43.4 (C-7), 56.1 (C-1), 66.6 (C-2 et C-6), 70.8 (C-4), 71.1 (C-3 et C-5), 169.0 (2CO), 169.6 (CO), 169.8 (2CO). **HRMS** (*m*/*z*, ESI): calculé pour C₁₇H₂₂O₁₁Na, [M+Na]+ 425.1054. Trouvé: 425.1055.

### Exemple 5: Préparation du 1-C-(2-propèn-1-yl)-1,3,4,5,6-penta-O-benzyl-scyllo-inositol 5 et 2-C-(2-propèn-1-yl)-1,3,4,5,6-penta-O-benzyl-myo-inositol 6

A une solution de **1,3,4,5,6-penta-O-benzyl-*myo*-2-inosose** (10g, 15,9mmol) dans du THF anhydre (50mL) est additionné goutte à goutte du bromure d'allylmagnesium (127mL d'une solution 1M dans l'éther diéthylique, 127,2mmol) sous argon à 0°C. Le mélange réactionnel est agité pendant 2h à température ambiante, hydrolysé en ajoutant une solution aqueuse saturée de NH₄Cl (100mL) puis extrait avec AcOEt (200mL). La phase aqueuse restante est de nouveau extraite avec de l'acétate d'éthyle (2x50 mL) et les phases organiques combinées sont séchées sur MgSO₄. Les solvants sont évaporés sous pression réduite et le produit brut est purifié par chromatographie sur colonne (gel de silice, Hex 100% à Hex/AcOEt: 85/15) pour donner **5** (4.0 g, 38%) sous la forme d'un solide blanc et **6** (5.6 g, 52%) sous la forme d'une huile.

### 1-C-(2-propèn-1-yl)-2,3,4,5,6-penta-O-benzyl-scyllo-inositol 5:

| | |
|---|---|
| 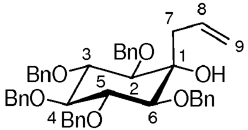 | Huile jaune |
| | **Formule** : C₄₄H₄₆O₆ |
| | **M. Mol.:** 670.83 g/mol |
| | **R_{f}** = 0.73 (Hex/AcOEt: 80/20) |

**IR** film (v, cm⁻¹): 3554, 3064, 3030, 2913, 1497,1066. **RMN ¹H (CDCl₃, 400 MHz):** δ (ppm) 2.57 (d, 2H, *J*_{7,8} = 7.5 Hz, H-7), 3.54 (d, 2H, *J*_{2,3} = *J*_{5,6} = 8.5 Hz, H-2 et H-6), 3.60-3.65 (m, 3H, H-3, H-4 et H-5), 4.88-4.95 (m, 10H, CH₂Ph), 5.20 (brd, 1H, *J* = 17.5 Hz, H-9), 5.21 (brd, 1H, *J* = 10.0 Hz, H-9'), 6.22 (ddt, 1H, H-8), 7.25-7.40 (m, 25H, H-Ar). **RMN ¹³C (CDCl₃, 100.6 MHz):** δ (ppm) 36.3 (C-7), 76.0 (2CH₂), 76.2 (2CH₂ et C-1), 76.4 (CH₂) 82.5 (C-3 et C-5), 83.8 (C-4), 85.5 (C-2 et C-6), 119.2 (C-9), 127.6 (2C-Ar), 127.7 (4C-Ar), 127.8 (3C-Ar), 128.0 (2C-Ar), 128.1 (4C-Ar), 128.5 (4C-Ar), 128.5 (4C-Ar), 128.6 (2C-Ar), 134.7 (C-8), 138.6 (2Cq-Ar), 138.6 (Cq-Ar), 139.0 (2Cq-Ar). **MS** (*m*/*z*, ESI), [M + Na]⁺ : 693.

### 2-C-(2-propèn-1-yl)-1,3,4,5,6-penta-O-benzyl-myo-inositol 6:

| | |
|---|---|
| 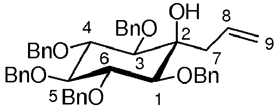 | Solide blanc |
| | **Formule** : C₄₄H₄₆O₆ |
| | **M. Mol.:** 670.84 g/mol |
| | **R_{f} =** 0.70 (Hex/ AcOEt: 80/20) |
| | **pf** = 96-97°C |

**IR** film (v, cm⁻¹): 3553, 3063, 3031, 2866. **RMN ¹H (CDCl₃, 400 MHz):** δ (ppm) 2.36 (brs, 1H, OH), 2.66 (d, 2H, *J*_{7,8} = 7.5 Hz, H7), 3.44 (d, 2H, *J*_{1,6} = *J*_{3,4} = 9.5 Hz, H-1 et H-3), 3.56 (app t, 1H, *J*_{4,5} = *J*_{5,6} = 9.5 Hz, H-5), 4.08 (app t, 2H, H-4 et H-6), 4.72 (d, 2H, *J* = 11.0 Hz, CH₂Ph), 4.87 (d, 2H, *J* = 11.0 Hz, CH₂Ph), 4.96 (s, 2H, CH₂Ph), 4.99 (d, 2H, *J* = 11.0 Hz, CH₂Ph), 5.06 (d, 2H, *J* = 11.0 Hz, CH₂Ph), 5.07-5.15 (m, 2H, H-9), 5.60 (ddt, 1H, *J*_{8,9} = 17.5 Hz, *J*_{8,9} = 9.5 Hz, H-8), 7.25-7.40 (m, 25H, H-Ar). **RMN ¹³C (CDCl₃, 100.6 MHz):** δ (ppm) 39.6 (C-7), 75.4 (2CH₂), 75.9 (3CH₂), 77.7 (C-2), 79.8 (C-3 et C-1), 83.3 (C-4, C-6 et C-5), 119.5 (C-9), 127.7 (3C-Ar), 127.8 (3C-Ar), 127.9 (5C-Ar), 127.9 (4C-Ar), 128.5 (10C-Ar), 132.9 (C-8), 138.3 (2Cq-Ar), 138.6 (3Cq-Ar). **MS** (*m*/*z*, ESI), [M + Na]⁺ : 693.

### Exemple 6: Préparation du 1-C-éthenyl-2,3,4,5,6-penta-O-benzyl-scyllo-inositol 7 et 2-C-éthenyl-1,3,4,5,6-penta-O-benzyl-myo-inositol 8

Ces composés sont préparés d'une manière similaire aux composés de l'exemple 5 à partir d'une solution de **1,3,4,5,6-penta-0-benzyl-myo-2-inosose** et de bromure de vinylmagnesium. On obtient les composés **7** (3,3 g, 32% rdt) sous la forme d'un solide blanc et **8** (5,0 g, 48% rdt) sous la forme d'une huile.

### 1-C-éthényl-2,3,4,5,6-penta-O-benzyl-scyllo-inositol 7:

| | |
|---|---|
| 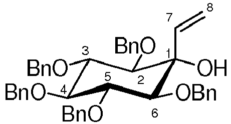 | Huile |
| | **Formule** : C₄₃H₄₄O₆ |
| | **M. Mol.:** 656.81 g/mol |
| | **R_{f}** = 0.62 (Hex/AcOEt: 80/20) |

**IR** film (v, cm⁻¹): 3552, 3030, 2910, 1497, 1066. **RMN ¹H (CDCl₃, 400 MHz):** δ (ppm) 3.61 (brd, 2H, *J*_{2,3} = *J*_{5,6} = 9.0 Hz, H-2 et H-6), 3.70-3.80 (m, 3H, H-3, H-4 et H-5), 4.89 (d, 2H, *J* = 11.0 Hz, CH₂Ph), 4.90 (d, 2H, *J* = 11.0 Hz, CH₂Ph), 4.93 (d, 2H, *J* = 11.0 Hz, CH₂Ph), 4.95 (d, 2H, *J* = 11.0 Hz, CH₂Ph), 4.98 (s, 2H, CH₂Ph), 5.56 (dd, 1H, *J*_{7,8} = 11.0 Hz et *J*_{8,8} = 1.5 Hz, H-8), 5.72 (dd, 1H, *J*_{7,8'} = 17.0 Hz, H-8'), 6.08 (dd, 1H, H-7), 7.31-7.40 (m, 25H, H-Ar). **RMN ¹³C (CDCl₃, 100.6 MHz):** δ (ppm) 75.9(4CH₂), 76.2 (CH₂), 78.6 (C-1), 82.5 (C-3 et C-5), 83.7 (C-4), 84.7 (C-2 et C-6), 119.2 (C-8), 127.8 (4C-Ar), 127.8 (5C-Ar), 128.0 (6C-Ar), 128.5 (4C-Ar), 128.5 (6CAr), 135.3 (C-7), 138.5 (3Cq-Ar), 138.8 (2Cq-Ar). **HRMS** (*m*/*z*, ESI): calculé pour C₄₃H₄₄O₆Na, [M+Na]⁺ 679.3030. Trouvé: 679.3050.

### 2-C-éthényl-1,3,4,5,6-penta-O-benzyl-myo-inositol 8:

| | |
|---|---|
| 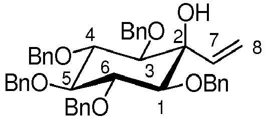 | Solide blanc |
| | **Formule** : C₄₃H₄₄O₆ |
| | **M. Mol.:** 656.81 g/mol |
| | **R_{f}** = 0.77 (Hex/ AcOEt: 80/20) |
| | **pf** = 119-120°C |

**IR** film (v, cm⁻¹): 3551, 3031, 2864, 1453, 1356, 1060. **RMN ¹H (CDCl₃, 400 MHz):** δ (ppm) 3.45 (d, 2H, *J*_{1,6} = *J*_{3,4} = 10.0 Hz, H-1 et H-3), 3.65 (app t, 1H, *J*_{4,5} = *J*_{5,6} = 10.0 Hz, H-5), 4.06 (app t, 2H, H-4 et H-6), 4.70 (d, 2H, *J* = 11.0 Hz, CH₂Ph), 4.85 (d, 2H, *J* = 10.0 Hz, CH₂Ph), 4.93 (d, 2H, *J* = 11.0 Hz, CH₂Ph), 4.98 (d, 2H, *J* = 10.0 Hz, CH₂Ph), 4.99 (s, 2H, CH₂Ph), 5.43 (dd, 1H, *J*_{7,8} = 10.5 Hz et *J*_{8,8} = 1.0 Hz, H-8), 5.64 (dd, 1H, *J*_{7,8} = 17.0 Hz, H-8'), 5.79 (dd, 1H, H-7), 7.30-7.40 (m, 25 H, H-Ar). **RMN ¹³C (CDCl₃, 100.6 MHz):** δ (ppm) 76.0 (3CH₂), 76.0 (2CH₂), 78.1 (C-2), 82.0 (C-1 et C-3), 82.6 (C-4 et C-6), 83.0 (C-5), 116.8 (C-8), 127.6 (3C-Ar), 127.9 (3CAr), 127.9 (4C-Ar), 128.3 (4C-Ar), 128.4 (6C-Ar), 128.5 (5C-Ar), 137.9 (2Cq-Ar), 138.6 (Cq-Ar), 138.7 (2Cq-Ar), 140.0 (C-7). **HRMS** (*m*/*z*, ESI): calculé pour C₄₃H₄₄O₆Na, [M+Na]⁺ 679.3030. Trouvé: 679.3022.

### Exemple 7: Préparation du 1-C-(hydroxyéthyl)-2,3,4,5,6-penta-O-benzyl-scyllo-inositol 9

A une solution de **7** (10,0g, 15,2mmol) dans du THF anhydre (250mL) est additionné goutte à goutte BH₃ (60mL d'une solution 1M dans l'hexane, 60mmol) sous argon à 0°C. Le mélange réactionnel est agité 8h à température ambiante, puis H₂O₂ 30% (500mL) et une solution aqueuse 2M de NaOH (600mL) sont additionnées lentement. Après 12h, le mélange réactionnel est dilué avec de l'acétate d'éthyle (200mL) puis extrait. La phase aqueuse restante est de nouveau extraite avec de l'acétate d'éthyle (2x100mL). Les phases organiques combinées sont séchées sur MgSO₄, filtrées et les solvants sont évaporés sous pression réduite. Le produit brut est purifié par chromatographie sur colonne (gel de silice, Hex 100% à Hex/AcOEt : 70/30) pour donner **9** (7,0g, 70% rdt) sous la forme d'une gomme.

| | |
|---|---|
| 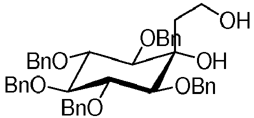 | Gomme |
| | **Formule** : C₄₃H₄₆O₇ |
| | **M. Mol.:** 674.83 g/mol |
| | **R_{f}** = 0.28 (Hex/AcOEt : 70/30) |

**IR** film (v, cm⁻¹): 3401, 3064, 2919, 1060. **RMN ¹H (CDCl₃, 400 MHz):** δ (ppm) 1.99 (t, 2H, *J*_{7,8} = 5.0 Hz, H-7), 3.50-3.55 (m, 2H, H-inositol), 3.60-3.70 (m, 3H, H-inositol), 3.89 (t, 2H, H-8), 4.85-4.95 (m, 10H, CH₂Ph), 7.25-7.38 (m, 25H, H-Ar). **RMN ¹³C (CDCl₃, 62.9 MHz):** δ (ppm) 34.3 (C-7), 58.3 (C-8), 75.9 (2CH₂), 76.1 (CH₂), 76.7 (2CH₂), 77.0 (C-1), 82.3 (C-3 et C-5), 84.2 (C-4), 85.6 (C-2 et C-6), 127.8 (C-Ar), 127.8 (2C-Ar), 127.9 (2C-Ar), 128.0 (4C-Ar), 128.1 (6C-Ar), 128.5 (6CAr), 128.7 (4C-Ar), 138.2 (2Cq-Ar), 138.4 (2Cq-Ar), 138.6 (Cq-Ar). **HRMS** (*m*/*z*, ESI): calculé pour C₄₃H₄₆O₇Na, [M+Na]⁺ 697.3136. Trouvé: 697.3150.

### Exemple 8: Préparation du 2-C-(hydroxyéthyl)-1,3,4,5,6-penta-O-benzyl-myo-inositol 10, du 1-C-(hydroxypropyl)-1,3,4,5,6-penta-O-benzyl-scyllo-inositol 11 et du 2-C-(hydroxypropyl)-1,3,4,5,6-penta-O-benzyl-myo-inositol 12

Ces composés sont préparés d'une manière similaire aux composés de l'exemple 7. On obtient les composés **10** (8,2 g, 80% rdt) sous la forme d'un solide blanc, **11** (7,20 g, 72% rdt) sous la forme d'un solide blanc et **12** (8,2 g, 80% rdt) sous la forme d'un solide blanc

### 2-C-(hydroxyéthyl)-1,3,4,5,6-penta-O-benzyl-myo-inositol 10

| | |
|---|---|
| 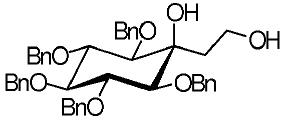 | Solide blanc |
| | **Formule** : C₄₃H₄₆O₇ |
| | **M. M₀I.:** 674.83 g/mol |
| | **R_{f}** = 0.55 (Hex/AcOEt: 60/40) |
| | **pf =** 140-141 °C |

**IR** film (v, cm⁻¹): 3448, 2927, 2875, 1065. **RMN ¹H (CDCl₃, 400 MHz):** δ (ₚpₘ) 1.94 (t, 2H, *J*_{7,8} = 6.5 Hz, H-7), 3.37 (d, 2H, *J*_{1,6} = *J*_{3,4} = 10.0 Hz, H-1 et H-3), 3.52 (t, 2H, H-8), 3.57 (app t, 1H, *J*_{4,5} = *J*_{5,6} = 10.0 Hz, H-5), 4.07 (app t, 2H, H-4 et H-6), 4.70 (d, 2H, *J* = 11.0 Hz, CH₂Ph), 4.85 (d, 2H, *J* = 11.0 Hz, CH₂Ph), 4.94 (s, 2H, CH₂Ph), 4.97 (d, 2H, *J* = 11.0 Hz, CH₂Ph), 5.05 (d, 2H, *J* = 1_{1.}0 Hz, CH₂Ph), 7.25-7.35 (m, 25H, H-Ar). **RMN ¹³C (CDCl₃, 100.6 MHz):** δ (ppm) 37.3 (C-7), 59.2 (C-8), 75.5 (2CH₂), 75.9 (3CH₂), 76.6 (C-2), 81.3 (C-1 et C-3), 83.2 (C-4 et C-6), 83.4 **(C**-5), 127.7 (3C-Ar), 127.8 (3C-Ar), 127.9 (4C-Ar), 128.0 (2C-Ar), 128.2 (4C-Ar), 128.6 (5C-Ar), *12*8.6 (4C-Ar), 138.2 (2Cq-Ar), 138.6 (3Cq-Ar). **HRMS** (*m*/*z*, ESI): calculé pour C₄₃H₄₆O₇Na, [M+Na]⁺ 697.3136. Trouvé: 697.3153.

### 1-C-(hydroxypropyl)-1,3,4,5,6-penta-O-benzyl-scyllo-inositol 11

| | |
|---|---|
| 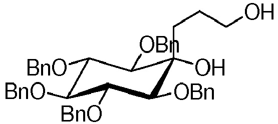 | Solide blanc |
| | **Formule** : C₄₄H₄₈O₇ |
| | **M. Mol.:** 688.86 g/mol |
| | **R_{f}** = 0.20 (Hex/ EtOAc: 70/30) |
| | **pf=** 152-153°C |

**IR** film (v, cm⁻¹): 3445, 2927, 1634, 1236. **RMN ¹H (CDCl₃, 400 MHz**): δ (ppm) 1.85 (t, 2H, *J*_{7,8} = 6.5 Hz, H-7), 1.95 (app qt, 2H, *J* = 6.5 Hz, H-8), 3.48 (d, 2H, *J*_{2,3} = *J*_{5,6} = 9.0 Hz, H-2 et H-6), 3.58-3.65 (m, 5H, H-3, H-4, H-5 et H-9), 4.75-4.95 (m, 10H, CH₂Ph), 7.25-7.35 (m, 25H, H-Ar). **RMN ¹³C (CDCl₃, 100.6 MHz)**: δ (ppm) 26.8 (C-8), 29.0 (C-7), 64.0 (C-9), 76.0 (2CH₂), 76.1 (C-1), 76.2 (CH₂), 76.3 (2CH₂), 82.7 (C-3 et C-5), 84.1 (C-4), 86.3 (C-2 et C-6), 127.8 (2C-Ar), 127.8 (3C-Ar), 127.9 (4C-Ar), 128.0 (2C-Ar), 128.1 (4C-Ar), 128.5 (4C-Ar), 128.6 (6C-Ar), 138.6 (2Cq-Ar), 138.6 (4Cq-Ar), 138.8 (2Cq-Ar). **HRMS** (*m*/*z*, ESI): calculé pour C₄₄H₄₃O₇Na, [M+Na]⁺ 711.3292. Trouvé : 711.3295.

### 2-C-(hydroxypropyl)-1,3,4,5,6-penta-O-benzyl-myo-inositol 12

| | |
|---|---|
| 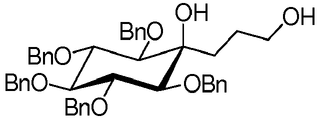 | Solide blanc |
| | **Formule** : C₄₄H₄₈O₇ |
| | **M. Mol.:** 688.86 g/mol |
| | **R_{f}** = 0.30 (Hex/ AcOEt: 70/30) |
| | pf = 121-122°C |

**IR** film (v, cm⁻¹): 3435, 2926, 2868, 1065. **RMN ¹H (CDCl₃, 400 MHz):** δ (ppm) 1.15-1.20 (m, 2H, H-8), 1.71-1.78 (m, 2H, H-7), 2.35 (brs, ¹H, OH), 3.29 (t, 2H, *J*_{8,9} = 6.5 Hz, H-9), 3.37 (d, 2H, *J*_{1,6} = *J*_{3,4} = 9.5 Hz, H-1 et H-3), 3.54 (app t, 1H, *J*_{4,5} = *J*_{5,6} = 9.5 Hz, H-5), 4.08 (app t, 2H, H-4 et H-6), 4.68 (d, 2H, *J* = 11.0 Hz, CH₂Ph), 4.87 (d, 2H, *J* = 11.0 Hz, CH₂Ph), 4.95 (s, 2H, CH₂Ph), 4.97 (d, 2H, *J* = 11.0 Hz, CH₂Ph), 5.02 (d, 2H, *J* = 11.0 Hz, CH₂Ph), 7.25-7.40 (m, 25H, H-Ar). **RMN ¹³C (CDCl₃, 100.6 MHz):** δ (ppm) 27.5 (C-8), 31.2 (C-7), 62.8 (C-9), 75.6 (2CH₂), 76.0 (3CH₂), 77.2 (C-2), 79.7 (C-1 et C-3), 83.4 (C-5), 83.5 (C-4 et C-6), 127.7 (C-Ar), 127.8 (2C-Ar), 127.8 (2C-Ar), 128.0 (6C-Ar), 128.4 (4C-Ar), 128.6 (10C-Ar), 138.4 (2Cq-Ar), 138.6 (2Cq-Ar), 138.7 (Cq-Ar). **MS** (*m*/*z*, ESI), [M + Na]⁺ : 711. [M+H]+ : 689. **Anal.** Calculé pour C₄₄H₄₈O₇: C, 76.71; H, 7.02. Trouvé: C, 76.16; H, 7.68.

### Exemple 9: Préparation du 1-C-(hydroxyéthyl)-scyllo-inositol 13

Le composé **9** (2g, 2,9mmol) est dissout dans MeOH (20mL), CH₂Cl₂ (20mL) et H₂O (2mL) puis Pd(OH)₂ (500mg, 20mol%) est additionné. La réaction est placée dans une bombe de Parr et agitée sous atmosphère d'hydrogène à 45Psi toute la nuit. Quand la réaction est finie, le mélange est filtré sur Célite, lavé avec de l'eau et le filtrat est concentré sous pression réduite. Le composé 13 (670mg, rdt quantitatif) est utilisé sans purification.

| | |
|---|---|
| 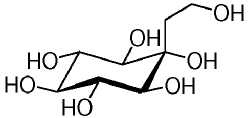 | Gomme |
| | **Formule** : C₃H₁₆O₇ |
| | **M. Mol.:** 224.20 g/mol |
| | **R_{f}** = 0 (CH₃CN/H₂O: 90/10) |

**IR** KBr (v, cm⁻¹): 3400, 2926, 1654, 1438. **RMN ¹H (D₂O, 400 MHz):** δ (ppm) 1.81 (t, 2H, *J*_{7,8} = 6.0 Hz, H-7), 3.27-3.48 (m, 5H, H-inositol), 3.83 (t, 2H, H-8). **RMN ¹³C (D₂O, 100.6 MHz):** δ (ppm) 32.1 (C-7), 57.3 (C-8), 73.1 (C-3 et C-5), 75.4 (C-4), 75.8 (C-2), 76.4 (C-2 et C-6). **HRMS** (*m*/*z*, ESI): calculé pour C₃H₁₆O₇Li, [M+Li]⁺ 231.1051. Trouvé: 231.1059.

### Exemple 10: Préparation du 2-C-(hydroxyéthyl)-myo-inositol 14, du 1-C-(hydroxypropyl)-scyllo-inositol 15 et du 2-C-(hydroxypropyl)-myo-inositol 16

Ces composés sont préparés d'une manière similaire aux composés de l'exemple 9. On obtient les composés **14** (670mg, rdt quantitatif), **15** (670mg, rdt quantitatif) et **16** (670mg, rdt quantitatif).

### 2-C-(hydroxyéthyl)-myo-inositol 14

| | |
|---|---|
| 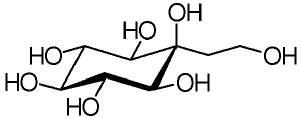 | Solide blanc |
| | **Formule** : C₃H₁₆O₇ |
| | **M. Mol.:** 224.21 g/mol |
| | **R_{f}** = 0 (CH₃CN/H₂O: 90/10) |
| | **pf** = 88-89°C |

**IR** : KBr (v, cm⁻¹) : 3398, 2923, 1654, 1458, 1121. **RMN ¹H, (D₂O, 400 MHz)** δ (ppm) : 2.00 (t, 2H, *J*_{7,8} = 7.0 Hz, H-7), 3.27 (app t, 1H, *J*_{4,5} = *J*_{5,6} = 9.5 Hz, H-5), 3.34 (d, 2H, *J*_{1,6} = *J*_{3,4} = 9.5 Hz, H-1 et H-3), 3.59 (app t, 2H, H-4 et H-6), 3.69 (t, 2H, H-8). **RMN ¹³C, (D₂O, 100.6 MHz)** δ (ppm) : 36.5 (C-7), 57.4 (C-8), 73.0 (C-1 et C-3), 73.4 (C-4 et C-6), 74.1 (C-5), 76.0 (C-2). **HRMS** (*m*/*z*, ESI): calculé pour C₃H₁₆O₇Na, [M+Na]⁺ 247.0788. Trouvé: 247.0798.

### 1-C-(hydroxypropyl)-scyllo-inositol 15

| | |
|---|---|
| 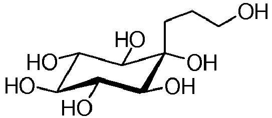 | Solide blanc |
| | **Formule** : C₉H₁₈O₇ |
| | **M. Mol.:** 238.23 g/mol |
| | **R_{f}** = 0 (CH₃CN/H₂O: 90/10) |
| | **pf** = 104-105°C |

**IR** KBr (v, cm⁻¹): 3400, 2922, 1654, 1458, 1080. **RMN ¹H (D₂O, 400 MHz):** δ (ppm) 1.57-1.62 (m, 2H, H-7), 1.78-1.87 (m, 2H, H-8), 3.30-3.47 (m, 5H, H-inositol), 3.68 (t, 2H, *J*_{8,9} = 5.0 Hz, H-9). **RMN ¹³C (D₂O, 100.6 MHz)** : δ (ppm) 26.4 (C-8), 26.4 (C-7), 63.0 (C-9), 73.2 (C-3 et C-5), 75.5 (C-4), 75.6 (C-1), 77.2 (C-2 et C-6). **MS** (*m*/*z*, ESI), [M + Na]⁺ : 261.

### 2-C-(hydroxypropyl)-myo-inositol 16

| | |
|---|---|
| 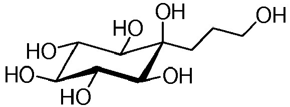 | Solide blanc |
| | **Formule** : C₉H₁₈O₇ |
| | **M. Mol.:** 238.23 g/mol |
| | **R_{f}** = 0 (CH₃CN/H₂O: 90/10) |
| | **pf** = 151-152°C |

**IR** KBr (v, cm⁻¹): 3467, 3060, 1490, 1445. **RMN ¹H (D₂O, 400 MHz):** δ (ppm) 1.43-1.55 (m, 2H, H-8), 1.68-1.75 (m, 2H, H-7), 3.25 (app t, 1H, *J*_{4,5} = *J*_{5,6} = 9.5 Hz, H-5), 3.36 (d, 2H, *J*_{1,6} = *J*_{3,4} = 9.5 Hz, H-1 et H-3), 3.59 (t, 2H, H-4 et H-6), 3.59 (app t, 2H, *J*_{8,9} = 7.0 Hz, H-9). **RMN ¹³C (D₂O, 100.6 MHz)** : δ (ppm) 26.0 (C-8), 29.9 (C-7), 62.1 (C-9), 71.6 (C-1 et C-3), 73.6 (C-4 et C-6), 74.2 (C-5), 76.9 (C-2). **HRMS** (*m*/*z*, ESI): calculé pour C₉H₁₈O₇Na, [M+Na]⁺ 261.0945. Trouvé: 261.0939.

### Exemple 11: Préparation du 1-C-(trityloxyéthyl)-2,3,4,5,6-penta-O-acétyl-scyllo-inositol 17

A une solution de **13** (670mg, 2,9mmol) dans de la pyridine (20mL) sont additionnés du chlorure de trityle (2,5g, 9mmol) et une quantité catalytique de DMAP. Le mélange réactionnel est agité à 70°C pendant 3 jours, puis la réaction est laissée revenir à température ambiante. De l'anhydride acétique (10mL) est additionné et la solution est agitée pendant 2h. Les solvants sont évaporés sous pression réduite et le résidu est purifié par chromatographie sur colonne (gel de silice, Hex/AcOEt: 80/20 à 60/40) pour donner **17** (1,4g, 70% rdt) sous la forme d'un solide blanc et **18** (345mg, 25% rdt) sous la forme d'un solide blanc.

### 1-C-(trityloxyéthyl)-2,3,4,5,6-penta-O-acétyl-scyllo-inositol 17

| | |
|---|---|
| 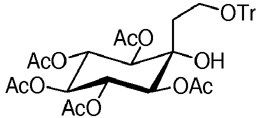 | Solide blanc |
| | **Formule** : C₃₇H₄₀O₁₂ |
| | **M. Mol.:** 676.71 g/mol |
| | **R_{f}** = 0.51 (Hex/AcOEt: 50/50) |
| | **pf** = 95-96°C |

**IR** film (v, cm⁻¹): 3467, 2107, 1759, 1644, 1368, 1224. **RMN ¹H (CDCl₃, 400 MHz):** δ (ppm) 1.92 (s, 6H, 2Me), 2.00 (s, 3H, Me), 2.01 (6H, 2Me), 2.21 (t, 2H, *J*_{7,8} = 6.5 Hz, H-7), 3.54 (t, 2H, H-8), 5.13-5.22 (m, 5H, H-inositol), 7.28 (brt, 3H, *J* = 8.0 Hz, H-Ar), 7.33 (brt, 6H, *J* = 8.0 Hz, H-Ar), 7.40 (brd, 6H, H-Ar). **RMN ¹³C (CDCl₃, 100.6 MHz)** : δ (ppm) 20.6 (2Me), 20.7 (3Me), 30.1 (C-7), 61.3 (C-8), 70.3 (C-3 et C-5), 71.5 (C-4), 74.3 (C-1), 74.6 (C-2 et C-6), 88.1 (Cq-Tr), 127.5 (3C-Ar), 128.2 (6C-Ar), 128.5 (6C-Ar), 143.5 (3Cq-Ar), 169.5 (2CO), 169.6 (CO), 170.0 (2CO). **HRMS** (*m*/*z*, ESI): calculé pour C₃₇H₄₀O₁₂Na [M+Na]⁺ 699.2412. Trouvé: 699.2400. **Anal.** calculé pour C₃₇H₄₀O₁₂ : C, 65.67 ; H, 5.95. Trouvé: C, 63.70 ; H, 5.88.

### 1-C-(acétoxyoéthyl)-2,3,4,5,6-penta-O-acétyl-scyllo-inositol 18

| | |
|---|---|
| 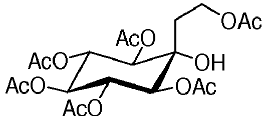 | Solide blanc |
| | **Formule** : C₂₀H₂₈O₁₃ |
| | **M. Mol.:** 476.43 g/mol |
| | **R_{f}** = 0.31 (Hex/AcOEt: 50/50) |
| | pf = 182-183°C |

**IR** film (v, cm⁻¹): 3492, 2937, 1755, 1737, 1371, 1218. **RMN ¹H (CDCl₃, 250 MHz)** : δ (ppm) 1.96 (s, 3H, Me), 2.00 (s, 6H, 2Me), 2.08 (s, 6H, 2Me), 2.14 (t, 2H, *J*_{7,8} = 7.5 Hz, H-7), 3.26 (brs, 1H, OH), 4.34 (t, 2H, H-8), 5.20-5.25 (m, 5H, H-inositol). **RMN ¹³C (CDCl₃, 62.9 MHz)** : δ (ppm) 20.5 (2Me), 20.6 (Me), 20.7 (2Me), 30.2 (C-7), 60.3 (C-8), 69.9 (C-3 et C-5), 71.5 (C-4), 74.0 (C-1), 74.5 (C-2 et C-6). **HRMS** (*m*/*z*, ESI): calculé pour C₂₀H₂₈O₁₃Na, [M+Na]⁺ 499.1422. Trouvé: 499.1425.

### Exemple 12: Préparation du 2-C-(trityloxyéthyl)-1,3,4,5,6-penta-O-acétyl-myo-inositol 19

Ce composé est préparé comme le composé 17 mais à partir d'une solution de **14** (680mg, 2,9mmol) pour donner **19** (1,45g, 74% rdt) sous la forme d'un solide blanc et **20** (275mg, 20% rdt) sous la forme d'un solide blanc.

### 2-C-(trityloxyéthyl)-1,3,4,5,6-penta-O-acétyl-myo-inositol 19:

| | |
|---|---|
| 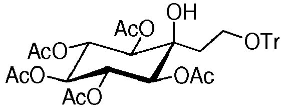 | Solide blanc |
| | **Formule** : C₃₇H₄₀O₁₂ |
| | **M. Mol.:** 676.71 g/mol |
| | **R_{f}** = 0.42 (Hex/AcOEt: 60/40) |
| | pf = 187-188°C |

**IR** : film (v, cm⁻¹) : 3482, 2936, 2857, 1758, 1227, 1036. **RMN ¹H, (CDCl₃, 400 MHz)** δ (ppm) : 1.88 (t, 2H, *J*_{7,8} = 6.5 Hz, H-7), 1.95 (s, 6H, 2Me), 1.98 (s, 6H, 2Me), 2.00 (s, 3H, Me), 3.26 (t, 2H, H-8), 4.96 (d, 2H, *J*_{1,6} = *J*_{3,4} = 9.5 Hz, H-1 et H-3), 5.17 (app t, 1H, *J*_{4,5} = *J*_{5,6} = 9.5 Hz, H-5), 5.54 (app t, 2H, H-4 et H-6), 7.29 (brt, 3H, *J* = 7.5 Hz, H-Ar), 7.33 (brt, 6H, *J* = 7.5 Hz, H-Ar), 7.38 (brd, 6H, H-Ar). **RMN ¹³C, (CDCl₃, 100.6 MHz)** δ (ppm) : 20.6 (2Me), 20.7 (3Me), 34.5 (C-7), 60.1 (C-8), 70.7 (C-5), 70.9 (C-4 et C-6), 73.5 (C-1 et C-3), 74.3 (C-2), 88.0 (Cq-Tr), 127.4 (3C-Ar), 128.1 (6C-Ar), 128.5 (6C-Ar), 143.5 (3Cq-Ar), 169.8 (2 CO), 169.8 (2CO), 169.9 (CO). **HRMS** (*m*/*z*, ESI): calculé pour C₃₇H₄₀O₁₂Na, [M+Na]⁺ 699.2412. Trouvé: 699.2441
**Anal.** calculé pour C₃₇H₄₀O₁₂: C : 65.67 ; H : 5.95. Trouvé: C : 65.75 ; H : 5.81.

### 2-C-(acétoxyoéthyl)-1,3,4,5,6-penta-O-acétyl-myo-inositol 20:

| | |
|---|---|
| 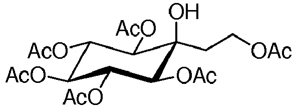 | Solide blanc |
| | **Formule** : C₂₀H₂₈O₁₃ |
| | **M. Mol.:** 476.43 g/mol |
| | **R_{f}** = 0.33 (Hex/AcOEt: 60/40) |
| | pf = 202-203°C |

**IR** film (v, cm⁻¹): 3481, 1752, 1229, 1037. **RMN ¹H (CDCl₃, 400 MHz)**: δ (ppm) 1.88 (t, 2H, *J*_{7,8} = 7.0 Hz, H-7), 1.98 (s, 6H, 2Me), 2.00 (s, 3H, Me), 2.03 (s, 3H, Me), 2.11 (s, 6H, 2Me), 2.64 (brs, 1H, OH), 4.06 (t, 2H, H-8), 5.12 (d, 2H, *J*_{1,6} = *J*_{3,4} = 10.0 Hz, H-1 et H-3), 5.25 (app t, 1H, *J*_{4,5} = *J*_{5,6} = 10.0 Hz, H-5), 5.53 (app t, 2H, H-4 et H-6). **RMN ¹³C (CDCl₃, 100.6 MHz)**: δ (ppm) 20.6 (Me), 20.7 (2Me), 20.7 (2Me), 21.0 (Me), 33.8 (C-7), 59.4 (C-8), 70.3 (C-5), 71.1 (C-4 et C-6), 71.9 (C-1 et C-3), 74.7 (C-2), 169.5 (2CO), 169.8 (CO), 170.0 (2CO), 171.0 (CO). **HRMS** (*m*/*z*, ESI): calculé pour C₂₀H₂₈O₁₃Na, [M+Na]⁺ 499.1422. Trouvé: 499.1422.

### Exemple 13: Préparation du 1-C-(trityloxypropyl)-2,3,4,5,6-penta-O-acétyl-scyllo-inositol 21

Ce composé est préparé comme le composé 17 mais à partir d'une solution de **15** (700mg, 2,9mmol) pour donner **21** (1,4g, 70% rdt) sous la forme d'un solide blanc et **22** (350mg, 25% rdt) sous la forme d'un solide blanc.

### 1-C-(trityloxypropyl)-2,3,4,5,6-penta-O-acétyl-scyllo-inositol 21:

| | |
|---|---|
| 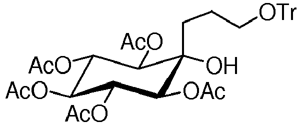 | Solide blanc |
| | **Formule** : C₃₃H₄₂O₁₂ |
| | **M. Mol.:** 690.74 g/mol |
| | **R_{f}** = 0.53 (Hex/ AcOEt: 60/40) |
| | pf = 128-130°C |

**IR** film (v, cm⁻¹): 3489, 2943, 1759, 1367, 1224, 1035. **RMN ¹H (CDCl₃, 400 MHz):** δ (ppm) 1.87-1.94 (m, 2H, H-7), 1.95-2.03 (m, 2H, H-8), 2.00 (s, 3H, Me), 2.05 (s, 6H, 2Me), 2.08 (s, 6H, 2Me), 3.15 (t, 2H, *J*_{8,9} = 5.5 Hz, H-9), 5.25-5.30 (m, 5H, H-inositol), 7.26 (brt, 3H, *J* = 7.5 Hz, H-Ar), 7.34 (brt, 6H, *J* = 7.5 Hz, H-Ar), 7.49 (brd, 6H, H-Ar). **RMN ¹³C (CDCl₃, 100.6 MHz):** δ (ppm) 20.5 (2Me), 20.6 (Me), 20.7 (2Me), 23.9 (C-8), 27.7 (C-7), 64.1 (C-9), 70.3 (C-3 et C-5), 71.6 (C-4), 74.0 (C-1), 75.0 (C-2 et C-6), 86.8 (Cq-Tr), 127.0 (3C-Ar), 127.9 (6C-Ar), 128.7 (6C-Ar), 144.3 (3Cq-Ar), 169.7 (CO), 169.8 (2CO), 170.3 (2CO). **HRMS** (*m*/*z*, ESI): calculé pour C₃₃H₄₂O₁₂Na, [M+Na]⁺ 713.2568. Trouvé: 713.2582.

### 1-C-(acétoxypropyl)-2,3,4,5,6-penta-O-acétyl-scyllo-inositol 22:

| | |
|---|---|
| 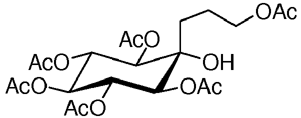 | Solide blanc |
| | **Formule** : C₂₁H₃₀O₁₃ |
| | **M. Mol.:** 490.45 g/mol |
| | **R_{f}** = 0.22 (Hex/ AcOEt: 60/40) |
| | **pf** = 121-122°C |

**IR** film (v, cm⁻¹): 3490, 2846, 1760, 1368, 1225. **RMN ¹H (CDCl₃, 250 MHz):** δ (ppm) 1.78-1.98 (m, 4H, H-7 et H-8), 1.99 (s, 3H, Me), 2.01 (s, 6H, 2Me), 2.09 (s, 9H, 3Me), 4.08 (t, 2H, *J*_{8,9} = 5.0 Hz, H-9), 5.13-5.30 (m, 5H, H-inositol). **RMN ¹³C (CDCl₃, 62.9 MHz):** δ (ppm) 20.6 (Me), 20.6 (2Me), 20.8 (2Me), 21.2 (Me), 22.7 (C-8), 27.6 (C-7), 65.1 (C-9), 70.0 (C-3 et C-5), 71.7 (C-4), 74.2 (C-1), 75.1 (C-2 et C-6), 169.8 (CO), 169.9 (2CO), 170.6 (2CO), 171.4 (CO). **HRMS** (*m*/*z,* ESI): calculé pour C₂₁H₃₀O₁₃Na, [M+Na]⁺ 513.1552. Trouvé: 513.1579.

### Exemple 14: Préparation du 2-C-(trityloxypropyl)-1,3,4,5,6-penta-O-acétyl-myo-inositol 23

Ce composé est préparé comme le composé 17 mais à partir d'une solution de **16** (700mg, 2,9mmol) pour donner **23** (1,3g, 67% rdt) sous la forme d'un solide blanc et **24** (430mg, 30% rdt) sous la forme d'un solide blanc.

### 2-C-(trityloxypropyl)-1,3,4,5,6-penta-O-acétyl-myo-inositol 23:

| | |
|---|---|
| 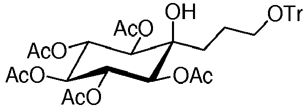 | Solide blanc |
| | **Formule** : C₃₈H₄₂O₁₂ |
| | **M. Mol.:** 690.74 g/mol |
| | **R_{f} =** 0.50 (Hex/ AcOEt: 60/40) |
| | **pf** = 136-137°C |

**IR** film (v, cm⁻¹): 3471, 1756, 1445, 1230, 1033. **RMN ¹H (CDCl₃, 400 MHz):** δ (ppm) 1.55-1.70 (m, 4H, H-8 et H-7), 2.01 (2Me), 2.02 (Me), 2.07 (2Me), 2.43 (brs, 1H, OH), 3.01 (t, 2H, *J*_{8,9} = 5.0 Hz, H-9), 5.19 (d, 2H, *J*_{1,6} = *J*_{3,4} = 10.0 Hz, H-1 et H-3), 5.25 (app t, 1H, *J*_{4,5} = *J*_{5,6} = 10.0 Hz, H-5), 5.59 (app t, 2H, H-4 et H-6), 7.24 (brt, 3H, *J* = 7.5 Hz, H-Ar), 7.30 (brt, 6H, *J* = 7.5 Hz, H-Ar), 7.39 (brd, 6H, H-Ar). **RMN ¹³C (CDCl₃, 100.6 MHz):** δ (ppm) 20.7 (5Me), 23.8 (C-7), 32.1 (C-8), 63.2 (C-9), 70.6 (C-5), 71.0 (C-1 et C-3), 71.3 (C-4 et C-6), 75.8 (C-2), 86.7 (Cq-Tr), 127.1 (3C-Ar), 127.9 (6 C-Ar), 128.8 (6C-Ar), 144.2 (3 Cq-Ar), 169.5 (2CO), 169.9 (CO), 170.0 (2 CO). **HRMS** (*m*/*z,* ESI): calculé pour C₃₃H₄₂O₁₂Na, [M+Na]⁺ 713.2568. Trouvé: 713.2654.

### 2-C-(acétoxypropyl)-1,3,4,5,6-penta-O-acétyl-myo-inositol 24:

| | |
|---|---|
| 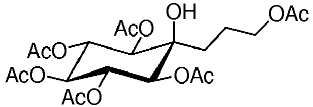 | Solide blanc |
| | **Formule** : C₂₁H₃₀O₁₃ |
| | **M. Mol.:** 490.45 g/mol |
| | **R_{f} =** 0.30 (Hex/ AcOEt: 60/40) |
| | **pf** = 196-197°C |

**IR** film (v, cm⁻¹): 3490, 2835, 1758, 1360, 1220. **RMN ¹H, (CDCl₃, 400 MHz)** δ (ppm) : 1.46-1.63 (m, 4H, H-7 et H-8), 1.98 (2Me), 1.99 (Me), 2.00 (Me), 2.10 (2Me), 2.42 (brs, 1H, OH), 3.93 (t, 2H, *J*_{8,9} = 5.5 Hz, H-9), 5.13 (d, 2H, *J*_{1,6} = *J*_{3,4} = 10.0 Hz, H-1 et H-3), 5.21 (app t, 1H, *J*_{4,5} = *J*_{5,6} = 10.0 Hz, H-5), 5.53 (app t, 2H, H-4 et H-5). **RMN ¹³C, (CDCl₃, 100.6 MHz)** δ (ppm) : 20.6 (2Me), 20.6 (Me), 20.6 (2Me), 21.0 (Me), 22.5 (C-8), 31.5 (C-7), 63.9 (C-9), 70.5 (C-5), 70.8 (C-1 et C-3), 71.1 (C-4 et C-6), 75.6 (C-2), 169.5 (2CO), 169.9 (CO), 169.9 (2CO), 171.0 (CO). **HRMS** (*m*/*z,* ESI): calculé pour C₂₁H₃₀O₁₃Na, [M+Na]⁺ 513.1579. Trouvé: 513.1576.

### Exemple 15: Préparation du 1-C-(hydroxvéthyl)-2,3,4,5,6-penta-O-acétyl-scyllo-inositol 25

Une solution de **17** (700mg, 1,03mmol) et FeCl₃ (1,5g, 10,3mmol) dans du CH₂Cl₂ anhydre (10mL) est agitée à température ambiante toute la nuit. Le mélange réactionnel est ensuite hydrolysé en ajoutant de l'eau puis extrait avec CH₂Cl₂ (3x100mL). Les phases organiques combinées sont séchées sur MgSO₄, filtrées et les solvants sont évaporés sous pression réduite. Le produit brut est purifié par chromatographie sur colonne (gel de silice, Hex/AcOEt: 80/20) pour donner **25** (320mg, 72% rdt) sous la forme d'un solide blanc.

| | |
|---|---|
| 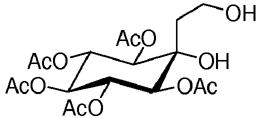 | Solide blanc |
| | **Formule** : C₁₈H₂₆O₁₂ |
| | **M. Mol.:** 434.39 g/mol |
| | **R_{f}** = 0.20 (Hex/AcOEt: 30/70) |
| | **pf** = 188-189°C |

**IR** film (v, cm⁻¹): 3484, 2942, 1757, 1362, 1224. **RMN ¹H (CDCl₃, 400 MHz):** δ (ppm) 1.98 (s, 3H, Me), 2.01 (s, 6H, 2Me), 2.10 (s, 6H, 2Me), 2.02-2.12 (m, 2H, H-7), 3.98-4.04 (m, 2H, H-8), 5.20-5.25 (app brs, 5H, H-inositol). **RMN ¹³C (CDCl₃, 100.6 MHz):** δ (ppm) 20.6 (3Me), 20.8 (2Me), 32.3 (C-7), 59.7 (C-8), 70.1 (C-3 et C-5), 71.6 (C-4), 74.9 (C-2 et C-6), 75.4 (C-1), 169.7 (CO), 169.9 (2CO), 170.2 (2CO). **HRMS** (*m*/*z,* ESI): calculé pour C₁₈H₂₆O₁₂Na, [M+Na]⁺ 457.1316. Trouvé: 457.1319.

### Exemple 16: Préparation du 2-C-(hydroxyéthyl)-1,3,4,5,6-penta-O-acétyl-myo-inositol 26

Ce composé est préparé d'une manière similaire au composé 25 mais à partir d'une solution de **19** (100mg, 0,15mmol) et FeCl₃ (200mg, 1,5mmol) dans du CH₂Cl₂ anhydre (5mL) pour donner **26** (29mg, 45% rdt) sous la forme d'un solide blanc, **27** sous forme d'un solide blanc (5mg, 10% rdt) et **28** sous forme d'un solide blanc.

### 2-C-(hydroxyéthyl)-1,3,4,5,6-penta-O-acétyl-myo-inositol 26:

| | |
|---|---|
| 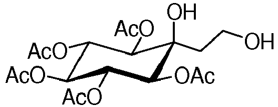 | Solide blanc |
| | **Formule** : C₁₈H₂₆O₁₂ |
| | **M. Mol.:** 434.39 g/mol |
| | **R_{f}** = 0.25 (Hex/AcOEt: 60/40) |
| | **pf** = 168-169°C |

**IR** film (v, cm⁻¹): 3403, 1729, 1370, 1227. **RMN ¹H (CDCl₃, 400 MHz):** δ (ppm) 1.78 (t, 2H, *J*_{7,8} = 6.0 Hz, H-7), 1.98 (s, 6H, 2Me), 1.99 (s, 3H, Me), 2.11 (s, 6H, 2Me), 3.73 (t, 2H, H-8), 5.16 (d, 2H, *J*_{1,6} = *J*_{3,4} = 10.0 Hz, H-1 et H-3), 5.23 (app t, 1H, *J*_{5,4} = *J*_{5,6} = 10.0 Hz, H-5), 5.54 (app t, 2H, H-4 et H-6). **RMN ¹³C (CDCl₃, 100.6 MHz):** 20.7 (3Me), 20.8 (2Me), 36.3 (C-7), 58.3 (C-8), 70.5 (C-5), 71.0 (C-4 et C-6), 72.9 (C-1 et C-3), 75.0 (C-2) 169.9 (3CO), 170.0 (2CO). **MS** (*m*/*z,* ESI): [M+Na]⁺ 457.

### 2-C-(acétoxyyéthyl)-3,4,5,6-tetra-O-acétyl-myo-inositol 27:

| | |
|---|---|
| 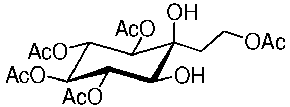 | Solide blanc |
| | **Formule** : C₁₈H₂₆O₁₂ |
| | **M. Mol.:** 434.39 g/mol |
| | **R_{f}** = 0.34 (Hex/ AcOEt: 40/60) |
| | **pf** = 183-184°C |

**IR** film (v, cm⁻¹): 3456, 1753, 1369, 1234. **RMN ¹H (CDCl₃, 400 MHz)** : δ (ppm) 1.90-2.10 (m, 2H, H-7), 1.98 (s, 3H, Me), 2.01 (s, 3H, Me), 2.06 (s, 3H, Me), 2.09 (s, 3H, Me), 2.11 (s, 3H, Me), 3.64 (d, 1H, *J*_{1,6} = 10.0 Hz, H-1), 4.17 (t, 2H, *J*_{7,8} = 6.5 Hz, H-8), 5.05 (d, 1H, *J*_{3,4} = 10.0 Hz, H-3), 5.17 (app t, 1H, *J*_{5,4} = *J*_{5,6} = 10.0 Hz, H-5), 5.33 (app t, 1H, H-6), 5.48 (app t, 1H, H-4). **RMN ¹³C (CDCl₃, 100.6 MHz)** : δ (ppm) 20.9 (2Me), 21.0 (Me), 21.2 (Me), 21.4 (Me), 30.1 (C-7), 60.3 (C-8), 70.4 (C-5), 71.4 (C-4), 72.6 (C-3), 72.7 (C-1), 73.6 (C-6), 75.5 (C-2). **MS** (*m*/*z,* ESI): [M+Na]⁺ 457.

### 1,2'-anhydro-2-C-(hydroxyéthyl)-2,3,4,5,6-penta-O-acétyl-myo-inositol 28:

| | |
|---|---|
| 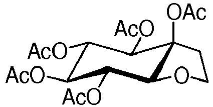 | Solide blanc |
| | **Formule** : C₁₈H₂₄O₁₁ |
| | **M. Mol.:** 416.38 g/mol |
| | **R_{f}** = 0.73 (Hex/AcOEt: 40/60) |

**IR** film (v, cm⁻¹): 2936, 1758, 1369, 1223. **RMN ¹H (CDCl₃, 400 MHz):** δ (ppm) 1.37 (dd , 1H, *J*_{7,7'} = 13.5 Hz, *J*_{7,8} = 4.0 Hz, H-7), 1.99 (s, 3H, Me), 2.00 (s, 3H, Me), 2.06-2.12 (m, 1H, H-7'), 2.08 (s, 3H, Me), 2.14 (s, 3H, Me), 3.83 (dd, 1H, *J*_{7,8'}= 7.0 Hz, *J*_{8,8'} = 11.0 Hz, H-8'), 4.07 (dt, 1H, *J*_{8,8'} = 11.0 Hz, *J*_{7,8} = 4.0 Hz, H-8), 4.26 (d, 1H, *J*_{1,6} = 6.5 Hz, H-1), 5.10 (app t, 1H, *J*_{4,5} = *J*_{5,6} = 10.0 Hz, H-5), 5.13-5.18 (m, 1H, H-6), 5.22 (d, 1H, *J*_{3,4} = 10.5 Hz, H-3), 5.45 (app t, 1H, H-4). **RMN ¹³C (CDCl₃, 100.6 MHz)** : δ (ppm) 20.4 (2Me), 20.5 (Me), 20.7 (Me), 28.9 (C-7), 58.1 (C-8), 69.3 (C-4), 69.7 (C-5), 70.9 (C-3), 73.8 (C-6), 79.8 (C-1), 80.9 (C-2), 169.2 (CO), 169.4 (CO), 169.7 (CO), 170.1 (CO). **MS** (*m*/*z,* ESI): [M+Na]⁺ 439.

### Exemple 17: Préparation du 1-C-(hydroxypropyl)-2,3,4,5,6-penta-O-acétyl-scyllo-inositol 29

Ce composé est préparé d'une manière similaire au composé 25 mais à partir d'une solution de **21** (100mg, 0,22mmol) et FeCl₃ (325mg, 2,2mmol) dans du CH₂Cl₂ anhydre (5mL) pour donner **29** (78mg, 78 % rdt) sous la forme d'un solide blanc.

| | |
|---|---|
| 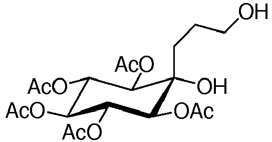 | Solide blanc |
| | **Formule** : C₁₉H₂₈O₁₂ |
| | **M. Mol.:** 448.42 g/mol |
| | **R_{f}** = 0.20 (Hex/ AcOEt: 40/60) |
| | **pf** = 150-151°C |

**IR** film (v, cm⁻¹): 3489, 2939, 1759, 1369, 1225. **RMN ¹H (CDCl₃, 250 MHz):** δ (ppm) 1.82-1.94 (m, 4H, H-7 et H-8), 1.96 (s, 3H, Me), 1.99 (s, 6H, 2Me), 2.06 (s, 6H, 2Me), 3.01 (brs, 2H, OH), 3.65 (t, 2H, *J*_{8,9} = 5.0 Hz, H-9), 5.10-5.24 (m, 5H, H-inositol). **RMN ¹³C (CDCl₃, 62.9 MHz):** δ (ppm) 20.6 (2Me), 20.6 (Me), 20.8 (2Me), 26.1 (C-8), 28.4 (C-7) 63.3 (C-9), 70.4 (C-3 et C-5), 71.6 (C-4), 73.8 (C-1), 75.0 (C-2 et C-6), 169.8 (CO), 170.0 (2CO), 170.4 (2CO). **HRMS** (*m*/*z,* ESI): calculé pour C₂₁H₂₉FO₁₂Na, [M+Na]⁺ 515.1535. Trouvé: 515.1515.

### Exemple 18: Préparation du 2-C-(hydroxypropyl)-1,3,4,5,6-penta-O-acétyl-myo-inositol 30

Ce composé est préparé d'une manière similaire au composé 25 mais à partir d'une solution de **23** (100mg, 0,22mmol) et FeCl₃ (325mg, 2,2mmol) dans du CH₂Cl₂ anhydre (5mL) pour donner **30** (80 mg, 81% rdt) sous la forme d'un solide blanc.

| | |
|---|---|
| 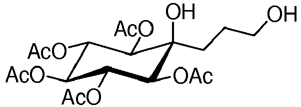 | Solide blanc |
| | **Formule** : C₁₉H₂₈O₁₂ |
| | **M. Mol.:** 448.42 g/mol |
| | **R_{f}** = 0.40 (Hex/ AcOEt: 20/80) |
| | **pf** = 121-122°C |

**IR** film (v, cm⁻¹): 3480, 1756, 1368, 1228, 1036. **RMN ¹H (CDCl₃, 400 MHz):** δ (ppm) 1.45-1.55 (m, 2H, H-8), 1.55-1.65 (m, 2H, H-7), 1.99 (s, 6H, 2Me), 2.00 (s, 3H, Me), 2.11 (s, 6H, 2 Me), 3.54 (t, 2H, *J*_{8,9} = 5.5 Hz, H-9), 5.12 (d, 2H, *J*_{1,6} = *J*_{3,4} = 10.0 Hz, H-1 et H-3), 5.21 (app t, 1H, *J*_{4,5} = *J*_{5,6} = 10.0 Hz, H5), 5.56 (app t, 2H, H-4 et H-6). **RMN ¹³C (CDCl₃, 100.6 MHz):** δ (ppm) 20.7 (3Me), 20.7 (2Me), 26.1 (C-8), 31.4 (C-7), 62.2 (C-9), 70.6 (C-5), 71.1 (C-4 et C-6), 71.2 (C-1 et C-3), 75.6 (C-2), 169.9 (2CO), 169.9 (3CO). **HRMS** (*m*/*z,* ESI): calculé pour C₁₉H₂₈O₁₂Na, [M+Na]⁺ 471.1473. Trouvé: 471.1471.

### Exemple 19: Préparation du 1-C-(tosyloxvéthyl)-2,3,4,5,6-penta-O-acétyl-scyllo-inositol 31

Une solution de **25** (100mg, 0,23mmol), chlorure de tosyle (107mg, 0,56 mmol) et triéthylamine (0,2mL, 0,03mmol) dans du CH₂Cl₂ anhydre (3mL) est agitée à température ambiante pendant 3h. Le mélange réactionnel est hydrolysé par ajout d'eau et extrait avec CH₂Cl₂ (3x50mL). Les phases organiques combinées sont séchées sur MgSO₄, filtrées et les solvants sont évaporés sous pression réduite. Le produit brut est purifié par chromatographie sur colonne (gel de silice, Hex/ AcOEt : 60/40) pour donner **31** (88 mg, 70% rdt) sous forme de solide blanc.

| | |
|---|---|
| 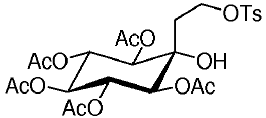 | Solide blanc |
| | **Formule** : C₂₅H₃₂O₁₄S |
| | **M. Mol.:** 588.58 g/mol |
| | **R_{f}** = 0.28 (Hex/AcOEt: 50/50) |
| | **pf** = 168-169°C |

**IR** film (v, cm⁻¹): 3485, 2945, 1760, 1368, 1222. **RMN ¹H (CDCl₃, 250 MHz):** δ (ppm) 1.96 (s, 3H, Me), 1.99 (s, 6H, 2Me), 2.04 (s, 6H, 2Me), 2.11 (t, 2H, *J*_{7,8} = 7.5 Hz, H-7), 2.45 (Me Ts), 4.34 (t, 2H, H-8), 5.05-5.25 (m, 5H, H-inositol), 7.36 (d, 2H, *J* = 8.0 Hz ,H-Ar), 7.82 (d, 2H, H-Ar). **RMN ¹³C (CDCl₃, 62.9 MHz):** δ (ppm) 20.5 (2Me), 20.6 (Me), 20.7 (2Me), 21.8 (Me-Ts), 30.3 (C-7), 66.4 (C-8), 70.7 (C-3 et C-5), 71.3 (C-1), 73.9 (C-4), 74.5 (C-2 et C-6), 128.1 (2C-Ar), 130.1 (2C-Ar), 133.3 (Cq-Ar), 145.1 (Cq-Ar), 169.7 (2CO), 169.7 (CO), 170.4 (2CO). **HRMS** (*m*/*z,* ESI): calculé pour C₂₅H₃₂O₁₄SK, [M+K]⁺ 627.1144. Trouvé: 627.1146.

### Exemple 20: Préparation du 2-C-(tosyloxyéthyl)-1,3,4,5,6-penta-O-acétyl-myo-inositol 32

Ce composé est préparé d'une manière similaire au composé 31 mais à partir d'une solution de **26** (100mg, 0,21 mmol), chlorure de tosyle (107mg, 0,63 mmol) pour donner **32** (86mg, 70% rdt) sous forme de solide blanc.

| | |
|---|---|
| 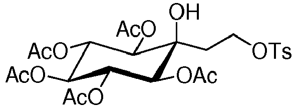 | Solide blanc |
| | **Formule** : C₂₅H₃₂O₁₄S |
| | **M. Mol.:** 588.58 g/mol |
| | **R_{f}** = 0.55 (Hex/AcOEt: 60/40) |
| | **pf** = 88-89°C |

**IR** film (v, cm⁻¹): 3483, 2942, 1757, 1227, 1037. **RMN ¹H, (CDCl₃, 400 MHz)** δ (ppm) : 1.96-2.00 (m, 2H, H-7), 1.97 (s, 6H, 2Me), 1.98 (s, 3H, Me), 2.09 (s, 6H, 2Me), 2.47 (s, 3H, MeTs), 3.98 (t, 2H, *J*_{7,8} = 7.0 Hz, H-8), 4.90 (d, 2H, *J*_{1,6} = *J*_{3,4} = 9.5 Hz, H-1 et H-3), 5.12 (app t, 1H, *J*_{4,5} = *J*_{5,6} = 9.5 Hz, H-5), 5.48 (app t, 2H, H-4 et H-6), 7.39 (d, 2H, *J* = 8.0 Hz, H-Ar), 7.79 (d, 2H, *J* = 8.0 Hz, H-Ar). **RMN ¹³C, (CDCl₃, 100.6 MHz)** δ (ppm) : 20.6 (5Me), 21.8 (MeTs), 34.5 (C-7), 65.1 (C-8), 70.3 (C-5), 70.8 (C-4 et C-6), 71.7 (C-1 et C-3), 74.3 (C-2), 128.2 (2C-Ar), 130.2 (2C-Ar), 132.6 (Cq-Ar), 145.4 (Cq-Ar), 169.7 (2CO), 169.8 (3CO). **HRMS** (*m*/*z,* ESI): calculé pour C₂₅H₃₂O₁₄SNa, [M+Na]⁺ 611.1405. Trouvé: 611.1400.

### Exemple 21: Préparation du 2-C-(tosyloxypropyl)-1,3,4,5,6-penta-O-acétyl-myo-inositol 33

Ce composé est préparé d'une manière identique au composé 31 mais à partir d'une solution de **30** (92mg, 0,19mmol), pour donner **33** (80mg, 66% rdt) sous forme de solide blanc.

| | |
|---|---|
| 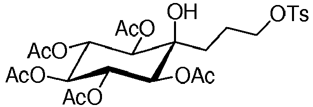 | Solide blanc |
| | **Formule** : C₂₆H₃₄O₁₄S |
| | **M. Mol.:** 602.61 g/mol |
| | **R_{f}** = 0.45 (Hex/ EtOAc: 60/40) |
| | **pf** = 121-122°C |

**IR** : film (v, cm⁻¹) : 3490, 3000, 1756, 1227. **RMN ¹H (CDCl₃, 250 MHz):** δ (ppm) 1.55-1.63 (m, 4H, H-7 et H-8), 1.97 (2Me), 1.99 (Me), 2.10 (2Me), 2.45 (MeTs), 3.89 (brs, 2H, H-9), 5.06 (d, 2H, *J*_{1,6} = *J*_{3,4} = 10.0 Hz, H-1 et H-3), 5.18 (app t, 1H, *J*_{4,5} = *J*_{5,6} = 10.0 Hz, H-5), 5.54 (app t, 2H, H-4 et H-6), 7.35 (d, 2H, *J* = 8.0 Hz, H-Ar), 7.75 (d, 2H, *J* = 8.0 Hz, H-Ar).

### Exemple 22: Préparation du 2-C-(fluoroéthyl)-1,3,4,5,6-penta-O-acétyl-myo-inositol 34

A une solution de **32** (70mg, 0,044mmol) dans du CH₃CN anhydre (8mL) est additionné KF (220mg, 0,44mmol) et 18C6 (300mg, 0,50mmol). Le mélange réactionnel est agité à température ambiante pendant 2h après évaporation. Le résidu est dilué dans CH₂Cl₂, lavé avec de l'eau, séché sur MgSO₄ et filtré. Les solvants sont évaporés sous pression réduite. Le produit brut est purifié par chromatographie sur colonne (gel de silice, Hex/AcOEt: 60/40) pour donner **34** (45 mg, 70%), **28** (10 mg, 15%), **35** (10 mg, 6%) et **36** (7 mg, 10%).

### 2-C-(fluoroéthyl)-1,3,4,5,6-penta-O-acétyl-myo-inositol 34:

| | |
|---|---|
| 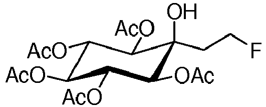 | huile |
| | **Formule** : C₁₈H₂₅FO₁₁ |
| | **M. Mol.:** 436.38 g/mol |
| | **R_{f} =** 0.35 (Hex/ AcOEt: 40/60) |

**IR** film (v, cm⁻¹): 3403, 2955, 1729, 1370, 1227, 1036. **RMN ¹H (CDCl₃, 400 MHz):** δ (ppm) 1.92 (dt, 2H, *J*_{H,F} = 28.0 Hz et *J*_{7,8} = 5.5 Hz, H-7), 1.99 (s, 6H, 2Me), 2.00 (s, 3H, Me), 2.10 (s, 6H, 2Me), 2.51 (brs, 1H, OH), 4.50 (dt, 2H, *J*_{H,F} = 47.0 Hz, H-8), 5.13 (d, 2H, *J*_{1,6} = *J*_{3,4} = 10.0 Hz, H-1 et H-3), 5.25 (app t, 1H, *J*_{4,5} = *J*_{5,6} = 10.0 Hz, H-5), 5.53 (app t, 2H, H-4 et H-6). **RMN ¹³C (CDCl₃, 100.6 MHz):** δ (ppm) 20.7 (5Me), 35.6 (d, *J*_{C,F} = 20.0 Hz, C-7), 70.3 (C-5), 71.0 (C-4 et C-6), 72.3 (C-1 et C-3), 74.6 (d, *J*_{C,F} = 1.5 Hz, C-2), 79.3 (d, *J*_{C,F} = 165 Hz, C-8), 169.5 (2CO), 169.8 (CO), 169.9 (2CO). **RMN ¹⁹F (CDCl₃, 235 MHz):** δ (ppm) -217.0 (tt, *J*_{H,F} =.48.0 Hz, *J*_{H,F} = 28.0 Hz). **HRMS** (*m*/*z,* ESI) calculé pour C₁₈H₂₅FO₁₁ Na, [M+Na]⁺: 459.1273. Trouvé: 459.1300.

### 2,2'-anhydro-2-C-(hydroxyéthyl)-1,3,4,5,6-penta-O-acétyl-myo-inositol 35:

| | |
|---|---|
| 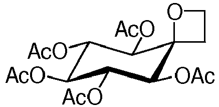 | Solide blanc |
| | **Formule** : C₁₈H₂₄O₁₁ |
| | **M. Mol.:** 416.37 g/mol |
| | **R_{f}** = 0.42(Hex/ AcOEt: 40/60) |
| | **pf** = 244-246°C |

**IR** film (v, cm⁻¹): 2939, 2896, 1757, 1375, 1223, 1042. **RMN ¹H (CDCl₃, 400 MHz):** δ (ppm) 1.98 (s, 3H, Me), 1.99 (s, 6H, 2Me), 2.19 (s, 6H, 2Me), 2.42 (t, 2H, *J*_{7,8} = 7.0 Hz, H-7), 4.48 (t, 2H, H-8), 5.09 (d, 2H, *J*_{1,6} = *J*_{3,4} = 10.0 Hz, H-1 et H-3), 5.16 (app t, 1H, *J*_{4,5} = *J*_{5,6} = 10.0 Hz, H-5), 5.38 (app t, 2H, H-4 et H-6). **RMN ¹³C (CDCl₃, 100.6 MHz):** δ (ppm)20.6 (3Me), 20.8 (2Me), 24.9 (C-7), 68.1 (C-8), 69.6 (C-4 et C-6), 70.7 (C-5), 72.3 (C-1 et C-3), 85.8 (C-2), 169.6 (2CO), 169.9 (CO), 170.3 (2CO). **HRMS** (*m*/*z,* ESI): calculé pour C₁₈H₂₄O₁₁Na, [M+Na]⁺ 439.1211. Trouvé: 439.1201.

### 2-C-(acétyloxyoéthyl)-3,4,5,6-tétra-O-acétyl-1-p-toluènesulfonate-myo-inositol 36:

| | |
|---|---|
| 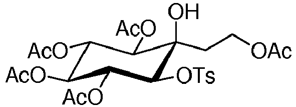 | Solide blanc |
| | **Formule** : C₂₅H₃₂O₁₄S |
| | **M. Mol.:** 588.58 g/mol |
| | **R_{f} =** 0.36 (Hex/ AcOEt: 40/60) |
| | **pf** = 88-89°C |

**IR** film (v, cm⁻¹): 3375, 2916, 1750, 1367, 1230, 1037. **RMN ¹H, (CDCl₃, 400 MHz)** δ (ppm) : 1.85 (s, 3H, Me), 1.97 (s, 3H, Me), 1.99 (s, 3H, Me), 1.98-2.02 (m, 2H, H-7), 2.02 (s, 3H, Me), 2.09 (s, 3H, Me), 2.45 (s, 3H, Me Ts), 4.06 (dt, 1H, *J*_{8,8'} = 19.0Hz, *J*_{8,7} = 6.0 Hz, H-8), 4.16 (dt, 1H, *J*_{8',7} = 6.5 Hz, H-8'), 4.82 (d, 1H, *J*_{1,6} = 10.0 Hz, H-1), 5.06 (d, 1H, *J*_{3,4} = 10.0 Hz, H-3), 5.20 (app t, 1H, *J*_{4,5} = *J*_{5,6} = 10.0 Hz, H-5), 5.50 (app t, 1H, H-4), 5.62 (t, 1H, H-6), 7.35 (d, 2H, *J* = 8.0 Hz, H-Ar), 7.77 (d, 2H, *J* = 8.0 Hz, H-Ar). **RMN ¹³C (CDCl₃, 100.6 MHz):** δ (ppm) 20.6 (Me), 20.7 (Me), 20.7 (Me), 20.7 (C-7), 21.0 (Me), 21.8 (Me-Ts), 59.4 (C-8), 70.0 (C-6), 70.3 (C-5), 70.8 (C-4), 71.9 (C-3), 75.1 (C-2), 80.0 (C-1), 127.8 (2C-Ar), 130.1 (2C-Ar), 133.9 (Cq-Ar), 145.5 (Cq-Ar), 169.5 (CO), 169.8 (CO), 169.9 (CO), 170.0 (CO), 171.0 (CO). **HRMS** (*m*/*z,* ESI): calculé pour C₂₅H₃₂O₁₄SNa, [M+Na]⁺ 611.1405. Trouvé: 611.1434.

### Exemple 23: Préparation du 2-C-(fluoroéthyl)-myo-inositol 37

A une solution de **34** (50mg, 0,097mmol) dans MeOH anhydre (10mL) est additionnée à 0°C, sous argon, une quantité catalytique de sodium. Le mélange réactionnel est agité pendant 2h à température ambiante, neutralisé avec de l'Amberlite IR-120 et de l'eau est additionnée pour solubiliser le composé. La solution est filtrée et évaporée sous pression réduite pour donner **37** (25mg, quantitatif) sous l'aspect d'une huile.

| | |
|---|---|
| 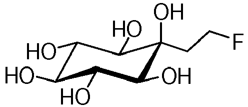 | huile |
| | **Formule :** C₈H₁₅FO₆ |
| | **M. Mol.:** 226.20 g/mol |
| | **R_{f} =** 0.30 (CH₃CN/H₂O: 90/10) |

**IR** KBr (v, cm⁻¹): 3400, 1637, 1384. **RMN ¹H (D₂O, 400 MHz)** : δ (ppm) 2.21 (dt, 2H, *J*_{H,F} = 28.0 Hz, *J*_{7,8} = 6.0 Hz, H-7), 3.33 (app t, 1H, *J*5,4 = *J*5,6 = 9.5 Hz, H-5), 3.46 (d, 2H, *J*1,6 = *J*3,4 = 9.5 Hz, H-1 et H-3), 3.64 (app t, 2H, H-4 et H-6), 4.67 (dt, 2H, *J*_{H,F} = 47.0 Hz, H-8). **RMN ¹³C (D₂O, 100.6 MHz)** : 34.5 (d, *J*_{C,F} = 19.0 Hz, C-7), 72.7 (C-4 et C-6), 73.4 (C-1 et C-3), 74.0 (C-5), 75.9 (d, *J*_{C,F} = 2.5 Hz, C-2), 81.5 (d, *J*_{C,F} = 158.0 Hz, C-8). **RMN ¹⁹F (D₂O, 235 MHz)** : δ (ppm) -216.7 (tt, *J*_{H,F} = 47.0 Hz, *J*_{H,F} = 28.0 Hz). **HRMS** (*m*/*z,* ESI): calculé pour C₃H₁₅FO₆Na, [M+Na]⁺ 249.0745. Trouvé: 249.0745.

### Exemple 24: Préparation du 2-C-(acétoxypropyl)-1,3,4,5,6-penta-O-benzyl-myo-inositol 38

A une solution de **12** (150mg, 0,22mmol) dans de la pyridine (5mL) est additionné sous argon, de l'anhydride acétique (2mL) et une quantité catalytique DMAP, puis la réaction est agitée à 100°C pendant 1h. Le mélange réactionnel est hydrolysé par ajout d'eau (50mL) et extrait avec AcOEt (3x75mL). La phase organique est lavée avec de l'eau, de la saumure, de l'eau glacée puis est séchée sur MgSO₄. Les solvants sont évaporés sous pression réduite. Le produit brut est purifié par flash chromatographie sur colonne (gel de silice, Hex/AcOEt: 60/40) pour donner **38** (143mg, 89% rdt) sous forme de solide blanc.

| | |
|---|---|
| 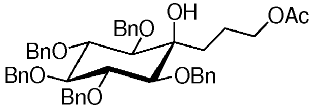 | Solide blanc |
| | **Formule** : C₄₆H₅₀O₈ |
| | **M. Mol.:** 730.89 g/mol |
| | **R_{f}** = 0.40 (Hex/ AcOEt: 70/30) |
| | **pf** = 137-138°C |

**IR** film (v, cm⁻¹): 3529-3030-3927-1735, 1240, 1063. **RMN ¹H (CDCl₃, 400 MHz):** δ (ppm) 1.20-1.30 (m, 2H, H-8), 1.75-1.83 (m, 2H, H-7), 1.88 (s, 3H, Me), 2.24 (brs, 1H, OH), 3.35 (d, 2H, *J*_{1,6} = *J*_{3,4} = 9.5 Hz, H-1 et H-3), 3.52 (app t, 1H, *J*_{4,5} = *J*_{5,6} = 9.5 Hz, H-5), 3.73 (t, 2H, *J*_{8,9} = 6.0 Hz, H-9), 4.06 (app t, 2H, H-4 et H-6), 4.66 (d, 2H, *J* = 11.0 Hz, CH₂Ph), 4.85 (d, 2H, *J* = 11.0 Hz, CH₂Ph), 4.93 (s, 2H, CH₂Ph), 4.96 (d, 2H, *J* = 11.0 Hz, CH₂Ph), 5.01 (d, 2H, *J* = 11.0 Hz, CH₂Ph), 7.24-7.35 (m, 25H, H-Ar). **RMN ¹³C (CDCl₃, 100.6 MHz):** δ (ppm) 20.9 (Me), 23.6 (C-8), 31.3 (C-7), 64.4 (C-9), 75.5 (2CH₂), 76.0 (3CH₂), 77.3 (C-2), 79.3 (C-1 et C-3), 83.4 (C-4, C-5 et C-6), 127.7 (C-Ar), 127.8 (2C-Ar), 127.8 (2C-Ar), 127.9 (4C-Ar), 128.1 (2C-Ar), 128.3 (4C-Ar), 128.6 (6C-Ar), 128.6 (4C-Ar),138.2 (2Cq-Ar), 138.6 (3Cq-Ar), 171.1 (CO). **HRMS** (*m*/*z,* ESI): calculé pour C₄₆H₅₀O₈Na, [M+Na]⁺ 753.3398. Trouvé: 753.3361.

### Exemple 25: Préparation du 2-C-(2-propèn-1-yl)-1,3,4,5,6-penta-O-benzyl-2-O-acétyl-myo-inositol 39

A une solution de **6** (150mg, 0,22 mmol) dans de l'acétate d'isopropényle (50mL) est additionné de l'ApTS (120mg, 0,70mmol). Le mélange réactionnel est agité à 80°C pendant 2h. Les solvants sont évaporés sous pression réduite, le résidu est dilué dans de l'eau et extrait avec CH₂Cl₂ (3x 50mL). La phase organique est séchée sur MgSO₄, filtrée et les solvants sont évaporés sous pression réduite. Le produit brut est purifié par flash chromatographie sur colonne (gel de silice, Hex/AcOEt: 80/20) pour donner **39** (133 mg, 85% rdt) sous forme d'un solide blanc.

| | |
|---|---|
| 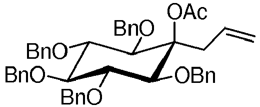 | Solide blanc |
| | **Formule** : C₄₆H₄₈O₇ |
| | **M. Mol.:** 712.87 g/mol |
| | **R_{f} =** 0.75 (Hex/ AcOEt: 80/20) |
| | **pf** = 87-88°C |

**IR** film (v, cm⁻¹): 3031, 2925, 1750, 1223. **RMN ¹H (CDCl₃, 400 MHz):** δ (ppm) 2.14 (s, 3H, Me), 3.54 (d, 2H, *J*_{1,6} = *J*_{3,4} = 9.5 Hz, H-1 et H-3), 3.60 (app t, 1H, *J*_{4,5} = *J*_{5,6} = 10.0 Hz, H-5), 3.59 (d, 2H, *J*_{7,8} = 7.0 Hz, H-7), 4.01 (app t, 2H, H-4 et H-6), 4.83 (d, 2H, *J* = 11.5 Hz, CH₂Ph), 4.85 (d, 2H, *J* = 10.5 Hz, CH₂Ph), 4.97 (d, 2H, *J* = 10.5 Hz, CH₂Ph), 4.98 (s, 2H, CH₂Ph), 5.03 (d, 2H, *J* = 11.5 Hz , CH₂Ph), 5.10 (dd, 1H, *J*_{8,9} = 10.0 Hz, *J*_{9,9'}= 1.5 Hz, H-9) 5.13 (dd, 1H, *J*_{8,9} = 17.0 Hz, H9') 5.69 (ddt, 1H, H-8), 7.25-7.42 (m, 25H, H-Ar). **RMN ¹³C (CDCl₃, 100.6 MHz):** δ (ppm) 23.3 (Me), 35.5 (C-7), 75.3 (2CH₂), 75.9 (2CH₂), 76.2 (CH₂), 80.1 (C-1 et C-3), 83.1 (C-4 et C-6), 83.4 (C-5), 89.8 (C-2), 119.9 (C-9), 127.2 (4C-Ar), 127.5 (3C-Ar), 127.8 (2C-Ar), 127.9 (4C-Ar), 128.1 (2C-Ar), 128.4 (4C-Ar), 128.5 (4C-Ar), 128.6 (2C-Ar), 133.0 (C-8), 138.5 (Cq-Ar), 138.4 (2Cq-Ar), 138.7 (2Cq-Ar), 169.4 (CO). **MS** (*m*/*z,* ESI), [M + Na]⁺ : 735.

### Exemple 26: Préparation de 1-C-(acétoxyéthyl)-1,2,3,4,5,6-hexa-O-acétyl-scyllo-inositol 40

Ce composé est préparé d'une manière similaire au composé 39 mais à partir d'une solution de **17** (1,0g, 1,45mmol) dans de l'acétate d'isopropényle (200mL) est additionné de l'ApTS (200mg, 1,16mmol) pour donner **40** (190mg, 25% rdt) sous forme d'un solide blanc et **18** (345mg, 50% rdt).

| | |
|---|---|
| 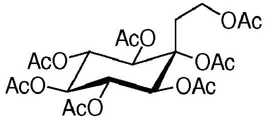 | Solide blanc |
| | **Formule** : C₂₂H₃₀O₁₄ |
| | **M. Mol.:** 518.47 g/mol |
| | **R_{f}** = 0.75 (Hex/AcOEt: 50/50) |
| | **pf** = 102-103°C |

**IR** film (v, cm⁻¹): 2991, 2943, 1760, 1369, 1223. **RMN ¹H (CDCl₃, 400 MHz)** : δ (ppm) 1.94 (s, 3H, Me), 1.98 (s, 3H, Me), 2.00 (s, 6H, 2Me), 2.06 (s, 9H, 3Me), 2.36 (t, 2H, *J*_{7,8} = 7.5 Hz, H-7), 4.27 (t, 2H, H-8), 5.23-5.32 (m, 3H, H-3, H-4 et H-5), 6.16 (d, 2H, *J*_{2,3} = *J*_{6,5} = 8.5 Hz, H-2 et H-6). **RMN ¹³C (CDCl₃, 100.6 MHz)** : δ (ppm) 20.6 (3Me), 20.6 (2Me), 21.1 (Me), 22.0 (Me), 29.9 (C-7), 59.8 (C-8), 70.4 (C-3 et C-5), 70.6 (C-2 et C-6), 70.9 (C-4), 81.9 (C-1), 169.0 (2CO), 169.5 (CO), 169.6 (CO), 169.8 (2CO), 171.0 (CO). **MS**(*m*/*z*, ESI), [M + NH₄]⁺ : 536.

### Exemple 27: Préparation du 1-C-(trityloxypropyl)-1,2,3,4,5,6-hexa-O-acétyl-scyllo-inositol 41

Ce composé est préparé d'une manière similaire au composé 39 mais à partir d'une solution de **21** (1,0g, 1,45mmol) dans de l'acétate d'isopropényle (200mL) est additionné de l'ApTS (200mg, 1,16mmol) pour donner **41** (550mg, 52% rdt) sous forme d'un solide blanc et **22** (285mg, 40% rdt).

| | |
|---|---|
| 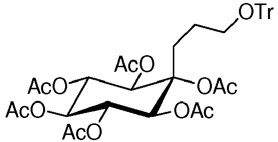 | Solide blanc |
| | **Formule** : C₄₀H₄₄O₁₃ |
| | **M. Mol.:** 732.77 g/mol |
| | **R_{f}** = 0.60 (Hex/ AcOEt: 60/40) |
| | **pf** = 158-159°C |

**IR** film (v, cm⁻¹): 2947, 1761, 1368, 1223. **RMN ¹H (CDCl₃, 400 MHz):** δ (ppm) 1.73-1.85 (m, 2H, H-8), 1.96 (s, 3H, Me), 1.99-2.04 (m, 2H, H-7), 1.99 (s, 3H, Me), 2.00 (s, 6H, 2Me), 2.03 (s, 6H, 2Me), 3.08 (t, 2H, *J*_{8,9} = 6.5 Hz, H-9), 5.23 (app t, 2H, *J*_{2,3} = *J*_{3,4} = *J*_{4,5} = *J*_{5,6} = 10.0 Hz, H-3 et H-5), 5.34 (app t, 1H, H-4), 6.05 (d, 2H, H-2 et H-6), 7.24 (tt, 3H, *J* = 7.5 Hz, *J* = 2.0 Hz, H-Ar), 7.31 (brt, 6H, *J* = 7.5 H, H-Ar), 7.44 (brd, 6H, H-Ar). **RMN ¹³C, (CDCl₃, 100.6 MHz)** δ (ppm) : 20.7 (3Me), 20.7 (2Me), 22.0 (Me), 24.2 (C-8), 27.3 (C-7), 63.6 (C-9), 70.8 (C-4), 70.9 (C-3 et C-5), 71.3 (C-2 et C-6), 82.7 (C-1), 86.6 (Cq-Tr), 127.1 (3C-Ar), 127.9 (6C-Ar), 128.8 (6C-Ar), 144.3 (3Cq-Ar), 169.1 (2CO), 169.6 (CO), 169.7 (CO), 169.8 (2CO). **HRMS** (*m*/*z,* ESI): calculé pour C₄₀H₄₄O₁₃Na, [M+Na]⁺ 755.2674. Trouvé: 755.2674.

### Exemple 28: Préparation du 2-C-(tritylloxypropyl)-1,2,3,4,5,6-hexa-O-acétyl-myo-inositol 42

Ce composé est préparé d'une manière similaire au composé 39 mais à partir d'une solution de **23** (1,0g, 1,45mmol) dans de l'acétate d'isopropényle (200mL) est additionné de l'ApTS (200mg, 1,16mmol) pour donner **42** (595mg, 56% rdt) sous forme d'un solide blanc et **24** (255mg, 36% rdt).

| | |
|---|---|
| 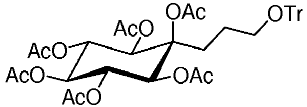 | Solide blanc |
| | **Formule** : C₄₀H₄₄O₁₃ |
| | **M. Mol.:** 732.77 g/mol |
| | **R_{f}** = 0.55 (Hex/ AcOEt: 60/40) |
| | **pf** = 121-122°C |

**IR** film (v, cm⁻¹): 3050, 1756, 1368, 1224. **RMN ¹H (CDCl₃, 400 MHz):** δ (ppm) 1.56-1.66 (m, 2H, H-8), 2.01 (s, 6H, 2Me), 2.03 (s, 3H, Me), 2.05 (s, 6H, 2Me), 2.33-2.42 (m, 2H, H-7), 2.99 (t, 2H, *J*_{8,9} = 6.0 Hz, H-9), 5.23 (app t, 1H, *J*_{4,5} = *J*_{5,6} = 10.0 Hz, H-5), 5.26 (d, 2H, *J*_{1,6} = *J*_{3,4} = 10.0 Hz, H-1 et H-3), 5.49 (app t, 2H, H-4 et H-6), 7.23 (bt, 3H, *J* = 7.5 Hz, H-Ar), 7.30 (brt, 6H, *J* = 7.5 Hz, H-Ar), 7.39 (brd, 6H, H-Ar). **RMN ¹³C (CDCl₃, 100.6 MHz):** δ (ppm) 20.6 (3Me), 20.6 (2Me), 22.9 (Me), 24.3 (C-8), 27.9 (C-7), 63.4 (C-9), 70.5 (C-1 et C-3), 70.7 (C-5), 70.8 (C-4 et C-6), 77.5 (C-2) 86.6 (Cq-Tr), 127.0 (3C-Ar), 127.8 (6C-Ar), 128.9 (6C-Ar), 144.3 (3Cq-Ar), 168.7 (CO), 169.5 (2CO), 169.8 (CO), 169.9 (2CO). **HRMS** (*m*/*z,* ESI): calculé pour C₄₀H₄₄O₁₃Na, [M+Na]⁺ 755.2674. Trouvé: 755.2671.

### Exemple 29: Préparation du 1-C-(hydroxypropyl)-1,2,3,4,5,6-hexa-O-acétyl-myo-inositol 43

Ce composé est préparé d'une manière similaire au composé 25 mais à partir d'une solution de 41 (500mg, 0,68mmol) et FeCl₃ (1,01g, 6,8mmol) dans du CH₂Cl₂ anhydre (10mL), pour donner 43 (233mg, 70% rdt) sous forme d'un solide blanc.

| | |
|---|---|
| 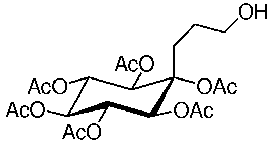 | Solide blanc |
| | **Formule** : C₂₁H₃₀O₁₃ |
| | **M. Mol.:** 490.45 g/mol |
| | **R_{f}** = 0.30 (Hex/ AcOEt: 40/60) |
| | **pf =** 147-149°C |

**IR** film (v, cm⁻¹): 3490, 2948, 1760, 1369, 1224. **RMN ¹H, (CDCl₃, 400 MHz)** δ (ppm) : 1.68-1.78 (m, 2H, H-8), 1.97 (s, 3H, Me), 1.99 (s, 3H, Me), 2.00 (s, 6H, 2Me), 2.06 (s, 6H, 2Me), 2.03-2.13 (m, 2H, H-7), 3.65 (t, 2H, *J*_{8,9} = 5.5 Hz, H-9), 5.26 (app t, 2H, *J*_{2,3} = *J*_{3,4} = *J*_{4,5} = *J*_{5,6} = 9.0 Hz, H-3 et H-5), 5.31 (app t, 1H, H-4), 6.00 (d, 2H, H-2 et H-6). **RMN ¹³C, (CDCl₃, 100.6 MHz)** δ (ppm) : 20.7 (3Me), 20.7 (2Me), 22.0 (Me), 26.5 (C-8), 26.9 (C-7), 62.9 (C-9), 70.9 (C-3 et C-5), 70.9 (C-4), 71.4 (C-2 et C-6), 82.8 (C-1), 169.1 (2CO), 169.7 (CO), 169.7 (CO), 169.9 (2CO). **HRMS** (*m*/*z,* ESI) calculated. for C₂₁H₃₀O₁₃Na, [M+Na]⁺: 513.1579. Trouvé: 513.1582.

### Exemple 30: Préparation du 2-C-(hydroxypropyl)-1,2,3,4,5,6-hexa-O-acétyl-myo-inositol 44

Ce composé est préparé d'une manière similaire au composé 25 mais à partir d'une solution de **42** (500mg, 0,68mmol) et FeCl₃ (1,01g, 6,8mmol) dans du CH₂Cl₂ anhydre (10mL), pour donner **44** (210mg, 63% rdt) sous forme d'un solide blanc.

| | |
|---|---|
| 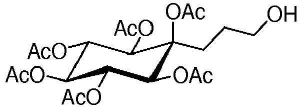 | Solide blanc |
| | **Formule** : C₂₁H₃₀O₁₃ |
| | **M. Mol.:** 490.45 g/mol |
| | **R_{f}** = 0.20 (Hex/ AcOEt: 60/40) |
| | **pf** = 212-213°C |

**IR** film (v, cm⁻¹): 3483, 2962, 1754, 1370, 1224. **RMN ¹H, (CDCl₃, 400 MHz)** δ (ppm) : 1.43-1.53 (m, 2H, H-7), 1.96 (s, 6H, 2Me), 1.97 (s, 3H, Me), 2.05 (s, 6H, 2Me), 2.16 (s, 3H, Me), 2.26-2.33 (m, 2H, H-8), 3.49 (t, 2H, *J*_{8,9} = 6.0 Hz, H-9), 5.25 (app t, 1H, *J*_{4,5} = *J*_{5,6} = 10.0 Hz, H-5), 5.27 (d, 2H, *J*_{1,6} = *J*_{3,4} = 10.0 Hz, H-1 et H-3), 5.44 (app t, 2H, H-4 et H-6). **RMN ¹³C, (CDCl₃, 100.6 MHz)** δ (ppm) : 20.6 (3 Me), 20.6 (2Me), 22.8 (Me), 26.8 (C-8), 27.1 (C-7), 62.2 (C-9), 70.6 (C-5), 70.6 (C-1 et C-3), 70.7 (C-4 et C-6), 86.5 (C-2), 168.8 (CO), 169.8 (2CO), 169.9 (CO), 170.0 (2CO). **HRMS** (*m*/*z,* ESI): calculé pour C₂₁H₃₀O₁₃Na, [M+Na]⁺ 513.1579. Trouvé: 513.1586.

### Exemple 31: Préparation du 1-C-(tosyloxypropyl)-1,2,3,4,5,6-hexa-O-acétyl-scyllo-inositol 45

Ce composé est préparé d'une manière similaire au composé 31 mais à partir d'une solution de **43** (50mg, 0,10mmol), chlorure de tosyle (60mg, 0,30mmol) et triéthylamine (0,1 mL, 0,05mmol) pour donner **45** (46mg, 73% rdt) sous la forme d'un solide blanc.

| | |
|---|---|
| 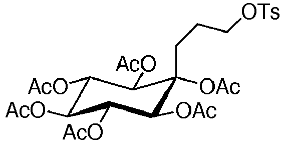 | Solide blanc |
| | **Formule** : C₂₈H₃₆O₁₅S |
| | **M. Mol.:** 644.64 g/mol |
| | **R_{f}** = 0.33 (Hex/ AcOEt: 60/40) |
| | **pf** = 110-111 °C |

**IR** film (v, cm⁻¹): 2925, 1761, 1368, 1223, 1035. **RMN ¹H (CDCl₃, 400 MHz):** δ (ppm) 1.80-1.90 (m, 2H, H-8), 1.94 (s, 3H, Me), 1.95-2.01 (m, 2H, H-7), 1.98 (s, 3H, Me), 1.99 (s, 6H, 2Me), 2.04 (s, 6H, 2Me), 2.46 (s, 3H, MeTs), 4.00 (t, 2H, *J*_{8,9} = 6.5 Hz, H-9), 5.17 (app t, 2H, *J*_{2,3} = *J*_{3,4} = *J*_{4,5} = *J*_{5,6} = 10.0 Hz, H-3 et H-5), 5.30 (app t, 1H, H-4), 6.02 (d, 2H, H-2 et H-6), 7.38 (d, 2H, *J* = 8.0 Hz, H-Ar), 7.80 (d, 2H, H-Ar). **RMN ¹³C (CDCl₃, 100.6 MHz):** δ (ppm) 20.7 (2Me), 20.7 (Me), 20.7 (2Me), 21.8 (Me), 22.0 (Me Ts), 23.3 (C-8), 26.6 (C-7), 70.3 (C-9), 70.6 (C-3 et C-5), 70.8 (C-4), 71.0 (C-2 et C-6), 82.2 (C-1), 128.1 (2C-Ar), 130.1 (2C-Ar), 132.9 (Cq-Ts), 145.1 (Cq-Ts), 169.0 (2CO), 169.5 (CO), 169.7 (CO), 169.8 (2CO). **HRMS** (*m*/*z,* ESI); calculé pour C₂₈H₃₆O₁₅SNa, [M+Na]⁺:667.1667. Trouvé: 667.1633.

### Exemple 32: Préparation du 2-C-(tosyloxypropyl)-1,2,3,4,5,6-hexa-O-acétyl-myo-inositol 46

Ce composé est préparé d'une manière similaire au composé 31 mais à partir d'une solution de **44** (50mg, 0,10mmol), chlorure de tosyle (60mg, 0,30mmol) et triéthylamine (0,1mL, 0,05mmol) pour donner **46** (46mg, 70% rdt) sous la forme d'un solide blanc.

| | |
|---|---|
| 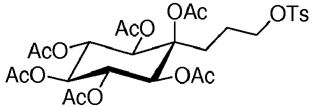 | Solide blanc |
| | **Formule** : C₂₈H₃₆O₁₅S |
| | **M. Mol.:** 644.64 g/mol |
| | **R_{f} =** 0.50 (Hex/ AcOEt: 60/40) |
| | **pf** = 182-183°C |

**IR** film (v, cm⁻¹): 2924, 1757, 1368, 1225. **RMN ¹H, (CDCl₃, 400 MHz)** δ (ppm) : 1.50-1.65 (m, 2H, H-8), 1.98 (s, 6H, 2Me), 2.00 (s, 3H, Me), 2.06 (s, 6H, 2Me), 2.19 (s, 3H, Me), 2.25-2.32 (m, 2H, H-7), 2.45 (s, 3H, Me Ts), 3.89 (t, 2H, *J*_{8,9} = 6.0 Hz, H-9), 5.10 (d, 2H, *J*_{1,6} = *J*_{3,4} = 10.0 Hz, H-1 et H-3), 5.16 (app t, 1H, *J*_{4,5} = *J*_{5,6} = 10.0 Hz, H-5), 5.44 (app t, 2H, H-4 et H-6), 7.35 (d, 2H, *J* = 8.5 Hz, H-Ar), 7.76 (d, 2H, *J* = 8.5 Hz, H-Ar). **RMN ¹³C, (CDCl₃, 100.6 MHz)** δ (ppm) : 20.6 (2Me), 20.6 (3Me), 21.8 (MeTs), 22.8 (Me), 23.2 (C-8), 27.0 (C-7), 69.9 (C-9), 70.4 (C-1 et C-3), 70.5 (C-4 et C-6), 70.7 (C-5), 85.7 (C-2), 128.1 (2C-Ar), 130.0 (2C-Ar), 132.9 (Cq-Ts), 144.9 (Cq-Ts), 168.6 (CO), 169.7 (2CO), 169.8 (CO), 169.8 (2CO). **HRMS** (*m*/*z,* ESI): calculé pour C₂₈H₃₆O₁₅Na, [M+Na]⁺ 667.1667. Trouvé: 667.1694

### Exemple 33: Préparation du 1-C-(tosyloxypropyl)-1,2,3,4,5,6-hexa-O-acétyl-scyllo-inositol 47

Ce composé est préparé d'une manière similaire au composé 39 mais à partir d'une solution de **31** (200mg, 0,34mmol) dans de l'acétate d'isopropényle (100mL) est additionné de l'ApTS (100mg, 0,34mmol) pour donner **47** (210mg, quantitatif) sous forme d'un solide blanc.

| | |
|---|---|
| 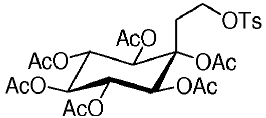 | Solide blanc |
| | **Formule** : C₂₇H₃₄O₁₅S |
| | **M. Mol.:** 630.62 g/mol |
| | **R_{f}** = 0.36 (Hex/AcOEt: 50/50) |
| | **pf** = 102-104°C. |

**IR** film (v, cm⁻¹): 2946, 1763, 1432, 1368, 1221. **RMN ¹H (CDCl₃, 400 MHz):** δ (ppm) 1.90 (s, 3H, CH₃), 1.98 (s, 3H, CH₃), 1.99 (s, 6H, 2 CH₃), 2.02 (s, 6H, 2 CH₃), 2.31 (t, 2H, *J*_{7,8} = 7.5 Hz, H-7), 2.46 (s, 3H, CH₃-Ts), 4.29 (t, 2H, *J*_{7,8} = 7.5 Hz, H-8), 5.11 (t, 2H, *J*_{2,3}= *J*_{3,4}= *J*_{4,5}= *J*_{5,6}= 10.0 Hz, H-3 et H-5), 5.26 (t, 1H, *J*_{3,4}= *J*_{4,5}= 10.0 Hz, H-4), 6.15 (d, 2H, *J*_{2,3}= *J*_{5,6}= 10.0 Hz, H-2 et H-6), 7.39 (d, 2H, *J* = 8.5 Hz, H-Ts), 7.82 (d, 2H, *J* = 8.5 Hz, H-Ts). **RMN ¹³C (CDCl₃, 100.6 MHz):** δ (ppm) 20.6 (5Me), 21.8 (Me), 21.9 (Me-Ts), 30.3 (C-7), 65.5 (C-8), 70.1 (C-3 et C-5), 70.3 (C-2 et C-6), 70.6 (C-4), 81.6 (C-1), 128.1 (2C-Ar), 130.1 (2C-Ar), 133.3 (Cq-Ar), 145.2 (Cq-Ar), 168.8 (2CO), 169.4 (CO), 169.5 (CO), 169.7 (2CO). **HRMS** (*m*/*z,* ESI): calculé pour C₂₇H₃₄O₁₅SNa, [M+Na]⁺ 653.1511. Trouvé: 653.1530.

### Exemple 34: Préparation du 2-C-(tosyloxyéthyl)-1,2,3,4,5,6-hexa-O-acétyl-myo-inositol 48

Ce composé est préparé d'une manière similaire au composé 39 mais à partir d'une solution de **32** (200mg, 0,34mmol) dans de l'acétate d'isopropényle (100mL) est additionné de l'ApTS (100mg, 0,34mmol) pour donner **48** (170mg, 78% rdt) sous forme d'un solide blanc.

| | |
|---|---|
| 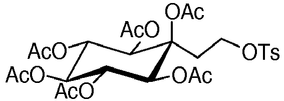 | Solide blanc |
| | **Formule** : C₂₇H₃₄O₁₅S |
| | **M. Mol.:** 630.62 g/mol |
| | **R_{f}** = 0.31 (Hex/AcOEt: 60/40) |
| | **pf** = 110-111 °C |

**IR** film (v, cm⁻¹): 2928, 1760, 1369, 1178. **RMN ¹H (CDCl₃, 400 MHz):** δ (ppm) 1.96 (s, 6H, 2Me), 1.97 (s, 3H, Me), 2.05 (s, 6H, 2Me), 2.17 (s, 3H, Me), 2.45 (s, 3H, MeTs), 2.64 (t, 2H, *J*_{7,8} = 7.0 Hz, H-7), 3.98 (t, 2H, H-8), 4.97 (d, 2H, *J*_{1,6} = *J*_{3,4} = 10.0 Hz, H-1 et H-3), 5.10 (appt, 1H, *J*_{4,5} = *J*_{5,6} = 10.0 Hz, H-5), 5.39 (app t, 2H, H-4 et H-6), 7.36 (d, 2H, *J* = 8.0 Hz, H-Ar), 7.78 (d, 2H, *J* = 8.0 Hz, H-Ar). **RMN ¹³C (CDCl₃, 100.6 MHz):** δ (ppm)20.6 (5 Me), 21.8 (Me), 22.6 (MeTs), 31.0 (C-7), 65.0 (C-8), 70.3 (C-4 et C-6), 70.4 (C-5), 71.2 (C-1 et C-3), 83.7 (C-2), 128.2 (2C-Ar), 130.1 (2C-Ar), 132.7 (Cq-Ar), 145.2 (Cq-Ar), 168.7 (CO), 169.6 (2CO), 169.7 (3CO). **HRMS** (*m*/*z,* ESI) calculated. for C₂₇H₃₄O₁₅SNa, [M+Na]⁺: 653.1511. Trouvé: 653.1527

### Exemple 35: Préparation du 1-C-(fluoropropyl)-1,2,3,4,5,6-hexa-O-acétyl-scyllo-inositol 49

A une solution de **43** (50mg, 0,1mmol) dans du CH₂Cl₂ anhydre (8mL) est additionné du DAST (0,03mL, 0,24mmol). Le mélange réactionnel est agité à température ambiante pendant 5 minutes puis hydrolysé par ajout de MeOH et dilué dans CH₂Cl₂ (20mL). La solution est lavée par une solution aqueuse saturée en hydrogénocarbonate de sodium et par de l'eau. La phase organique est séchée sur MgSO₄, filtrée et les solvants sont évaporés sous pression réduite. Le produit brut est purifié par flash chromatographie sur colonne (gel de silice Hex/AcOEt: 80/20) pour donner **49** (45mg, 91% rdt) sous la forme d'un solide blanc.

| | |
|---|---|
| 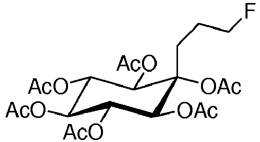 | Solide blanc |
| | **Formule** : C₂₁H₂₉FO₁₂ |
| | **M. Mol.:** 492.44 g/mol |
| | **R_{f} =** 0.25 (Hex/ AcOEt: 60/40) |
| | **pf** = 151-152°C |

**IR** film (v, cm⁻¹): 2963, 1760, 1369, 1224, 1036. **RMN ¹H (CDCl₃, 400 MHz):** δ (ppm) 1.77-1.94 (m, 2H, H-8), 1.96 (s, 3H, Me), 1.97 (s, 3H, Me), 1.99 (s, 6H, 2Me), 2.05 (s, 6H, 2Me), 2.09-2.15 (m, 2H, H-7), 4.43 (dt, 2H, *J*_{H,F} = 47.0 Hz et *J*_{8,9} = 6.0 Hz, H-9), 5.23 (app t, 2H, *J*_{2,3} = *J*_{3,4} = *J*_{4,5} = *J*_{5,6} = 9.5 Hz, H-3 et H-5), 5.32 (app t, 1H, H-4), 6.02 (d, 2H, H-2 et H-6). **RMN ¹³C (CDCl₃, 100.6 MHz):** δ (ppm) 20.6 (3Me), 20.7 (2Me), 22.0 (Me), 24.7 (d, *J*_{C,F} = 20.0 Hz, C-8), 26.5 (d, *J*_{C,F} = 5.0 Hz, C-7), 70.8 (C-3, C-4 et C-5), 71.2 (C-2 et C-6), 82.5 (C-1), 83.8 (d, *J*_{C,F} = 166.0 Hz, C-9), 169.0 (2CO), 169.5 (CO), 169.7 (CO), 169.8 (2CO). **RMN ¹⁹F (D₂O, 235 MHz):** δ (ppm) -219.1 (tt, *J*_{H,F} = 47.0 Hz et *J*_{H,F} = 25.0 Hz). **HRMS** (*m*/*z,* ESI): calculé pour C₂₁H₂₉FO₁₂Na, [M+Na]⁺ 515.1535. Trouvé: 515.1515.

### Exemple 36: Préparation du 2-C-(fluoropropyl)-1,2,3,4,5,6-hexa-O-acétyl-myo-inositol 50

Ce composé est préparé d'une manière similaire au composé 49 mais à partir d'une solution de **44** (50mg, 0,1mmol) dans du CH₂Cl₂ anhydre (8mL) est additionné du DAST (0,03mL, 0,24mmol) pour donner **50** (48mg, 95% rdt) sous la forme d'un solide blanc.

| | |
|---|---|
| 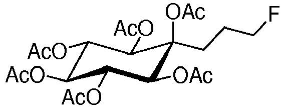 | Solide blanc |
| | **Formule** : C₂₁H₂₉FO₁₂ |
| | **M. Mol.:** 492.44 g/mol |
| | **R_{f}** = 0.50 (Hex/ AcOEt: 60/40) |
| | **pf =** 209-210°C |

**IR** film (v, cm⁻¹): 2923, 1754, 1370, 1223, 1041. **RMN ¹H (CDCl₃, 400 MHz):** δ (ppm) 1.55-1.67 (m, 2H, H-8), 1.99 (s, 6H, 2Me), 2.00 (s, 3H, Me), 2.07 (s, 6H, 2Me), 2.19 (s, 3H, Me), 2.33-2.37 (m, 2H, H-7), 4.33 (dt, 2H, *J*_{H,F} = 47.0 Hz et *J*_{8,9} = 5.5 Hz, H-9), 5.18 (d, 2H, *J*_{1,6} = *J*_{3,4} = 10.0 Hz, H-1 et H-3), 5.20 (app t, 1H, *J*_{4,5} = *J*_{5,6} = 10.0 Hz, H-5), 5.47 (app t, 2H, H-4 et H-6). **RMN ¹³C (CDCl₃, 100.6 MHz):** δ (ppm) 20.5 (2Me), 20.6 (3Me), 22.8 (Me), 24.7 (d, *J* = 18.0 Hz, C-8), 26.9 (d, *J*_{C,F} = 5.0 Hz, C-7), 70.6 (C-1 et C-3), 70.6 (C-4 et C-6), 70.7(C-5), 83.4 (d, *J*_{C,F} = 167.0 Hz, C-9), 86.1 (C-2), 168.8 (CO), 169.7 (2CO), 169.8 (3CO). **RMN ¹⁹F (CDCl₃, 235 MHz):** δ (ppm) -219.7 (tt, *J*_{H,F} = 47.0 Hz et *J*_{H,F} = 21.0 Hz). **HRMS** (*m*/*z,* ESI): calculé pour C₂₁H₂₉FO₁₂Na, [M+Na]⁺ 515.1535. Trouvé: 515.1546.

### Exemple 37: Préparation du 2-C-(fluoropropyl)-myo-inositol 51

Ce composé est préparé d'une manière similaire au composé 37 mais à partir d'une solution de **45** (48mg, 0,097mmol) pour donner **51** (23mg, quantitatif) sous forme d'une huile.

| | |
|---|---|
| 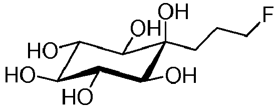 | Huile |
| | **Formule** : C₉H₁₇FO₆ |
| | **M. Mol.:** 240.23 g/mol |
| | **R_{f}** = 0.38 (CH₃CN/H₂O: 90/10) |

**IR** : KBr (v, cm⁻¹) : 3436, 2924, 1384, 1124. **RMN ¹H, (D₂O, 400 MHz)** δ (ppm) : 1.61-1.78 (m, 2H, H-9), 1.78-1.85 (m, 2H, H-7), 3.29 (app t, 1H, *J*_{4,5} = *J*_{5,6} = 10.0 Hz, H-5), 3.38 (d, 2H, *J*_{1,6} = *J*_{3,4} = 10.0 Hz, H-1 et H-3), 3.61 (app t, 2H, H-4 et H-6), 4.54 (dt, 2H, *J*_{H,F} = 47.0 Hz et *J*_{8,9} = 6.0 Hz, H-9). **RM7N ¹³C, (D₂O, 62.9 MHz)** δ (ppm) : 24.5 (d, *J*_{C,F} = 20.0 Hz, C-8), 29.2 (d, *J*_{C,F} = 5.0 Hz, C-7), 71.6 (C-1 et C-3), 73.5 (C-4 et C-6), 74.1 (C-5), 76.6 (C-2), 85.5 (d, *J*_{C,F} = 159.0 Hz, C-9). **RMN ¹⁹F (D₂O, 235 MHz):** δ (ppm) -216.6 (tt, *J*_{H,F} = 47.0 Hz et *J*_{H,F} = 25.0 Hz). **HRMS** (*m*/*z,* ESI): calculé pour C₉H₁₇FO₆Na, [M+Na]⁺ 263.0901. Trouvé: 263.0896.

### Exemple 38: Préparation du 1-C-(fluoropropyl)-scyllo-inositol 52

Ce composé est préparé d'une manière similaire au composé 37 mais à partir d'une solution de **49** (100mg, 0,097mmol) pour donner **52** (50mg, quantitatif) sous forme d'une huile.

| | |
|---|---|
| 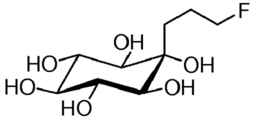 | Huile |
| | **Formule :** C₉H₁₇FO₆ |
| | **M. Mol.:** 240.22 g/mol |
| | **R_{f} =** 0.42 (CH₃CN/H₂O : 90/10) |

**IR** KBr (v, cm⁻¹): 3394, 2923, 1654, 1005. **RMN ¹H (D₂O, 250 MHz) :** δ (ppm) 1.63-1.75 (m, 2H, H-7), 1.85-2.16 (m, 2H, H-8), 3.35-3.55 (m, 5H, H-inositol), 4.55 (dt, 2H, *J*_{H,F} = 47 Hz, *J*_{9,8} = 6.0 Hz, H-9). **RMN ¹³C (D₂O, 62.9 MHz)** : δ (ppm) 25.0 (d, *J*_{C,F} = 19.0 Hz, C-8), 25.6 (d, *J*_{C,F} = 5.0 Hz, C-7), 73.1 (C-3 et C-5), 75.4 (C-1 et C-4), 77.1 (C-2 et C-6), 100.0 (d, *J*_{C,F} = 158.0 Hz, C-9). **RMN ¹⁹F (D₂O, 235 MHz)** :δ (ppm) -216.9 (tt, *J*_{H,F} = 47.0 Hz, *J*_{H,F} = 27.0 Hz). **HRMS** (*m*/*z,* ESI): calculé pour C₉H₁₇FO₆Na, [M+Na]⁺ 263.0901. Trouvé: 263.0905.

### Exemple 39: Préparation du 2,3,4,5,6-penta-O-benzyl-scyllo-inositol 53

Ce composé est décrit dans la publication Martin, S. et al. Journal of Organic Chemistry 1994, 59(17), 4805.

A une solution de **1,3,4,5,6-penta-*O*-benzyl-*myo*-2-inosose** (10g, 16mmol) dans iPrOH anhydre (300mL) est additionné NaBH4 (900mg, 16mmol).

Le mélange réactionnel est agité à température ambiante pendant 2h puis hydrolysé par ajout d'eau et dilué dans CH₂Cl₂ (500mL). La solution est lavée avec de l'eau, séchée sur MgSO₄ et filtrée. Les solvants sont évaporés sous pression réduite. Le produit brut est purifié par chromatographie sur colonne (gel de silice, Hex/AcOEt: 80/20) pour donner **53** (2,8g, 30% rdt) sous la forme d'un solide blanc et **1,3,4,5,6-penta-*O*-benzyl-*myo*-inositol** (5,5g, 55% rdt).

| | |
|---|---|
| 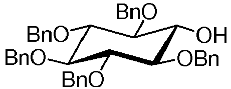 | Solide blanc |
| | **Formule** : C₄₁H₄₂O₆ |
| | **M. Mol.:** 630.77 g/mol |
| | **R_{f}** = 0.68 (Hex/AcOEt: 80/20) |
| | **pf** = 75-76°C |

**IR** film (v, cm⁻¹): 3448, 3030, 2907, 1453, 1060. **RMN ¹H (CDCl₃, 400 MHz):** δ (ppm) 3.45 (app t, 2H, *J*_{3,2} = *J*_{3,4} = *J*_{5,4} = *J*_{5,6} = 9.0 Hz, H-3 et H-5), 3.55-3.62 (m, 3H, H-4, H-2 et H-6), 3.64 (app t, 1H, H-1), 4.83 (d, 2H, *J* = 11.0 Hz, CH₂Ph), 4.89 (s, 4H, CH₂Ph), 4.90 (s, 2H, CH₂Ph), 4.92 (d, 2H, *J* = 11.0 Hz, CH₂Ph), 7.27-7.39 (m, 25H, H-Ar). **RMN ¹³C (CDCl₃, 100.6 MHz):** δ (ppm) 74.6 (C-1), 75.7 (2CH₂), 76.0 (2CH₂), 76.1 (CH₂), 82.6 (C-3 et C-5), 83.0 (C-2 et C-6), 83.3 (C-4), 127.8 (3C-Ar), 128.0 (3C-Ar), 128.0 (3C-Ar), 128.1 (3C-Ar), 128.5 (3C-Ar), 128.6 (5C-Ar), 138.6 (3Cq-Ar), 138.6 (2Cq-Ar). **HRMS** (*m*/*z,* ESI): calculé pour C₄₁H₄₂O₆Na, [M+Na]⁺ 653.2864. Trouvé: 653.2874.

### Exemple 40: Préparation du 1,3,4,5,6-penta-O-benzyl-2-O-(2-propèn-1-yl)-myo-inositol 54

Ce composé est décrit dans la publication de Florence, G. J. et al. *Synlett* **2009,** 3099.

A une solution de **1,3,4,5,6-penta-*O*-benzyl-*myo*-inositol** (8g, 12,7mmol) dans du bromure d'allyle (200mL) est additionné NaH (3g, 12,7mmol).

Le mélange réactionnel est agité pendant 2h à 70°C. Les solvants sont évaporés sous pression réduite et le résidu est dilué dans de l'eau et extrait avec CH₂Cl₂ (3x 50mL). Les phases organiques combinées sont séchées sur MgSO₄, filtrées et les solvants sont évaporés sous pression réduite. Le produit brut est purifié par flash chromatographie sur colonne (gel de silice, Hex/AcOEt: 80/20 à 40/60) pour donner **54** (6,8g, 80% rdt) sous la forme d'un solide blanc.

| | |
|---|---|
| 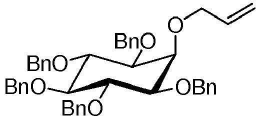 | Solide blanc |
| | **Formule** : C₄₄H₄₆O₆ |
| | **M. Mol.:** 670.83 g/mol |
| | **R_{f}** = 0.80 (Hex/AcOEt: 80/20) |
| | **pf** = 82-83°C |

**IR** film (v, cm⁻¹): 3030, 2868, 1454, 1071. **RMN ¹H (CDCl₃, 400 MHz):** δ (ppm) 3.34 (dd, 2H, *J*_{3,4} = *J*_{1,6} = 9.5 Hz, *J*_{3,2} = *J*_{1,2} = 2.0 Hz, H-1 et H-3), 3.48 (app t, 1H, *J*_{5,4} = *J*_{5,6} = 9.5 Hz, H-5), 3.98 (app t, 1H, H-2), 4.05 (app t, 2H, H-4 et H-6), 4.35 (bd, 2H, *J*_{7,8} = 5.5 Hz, H-7), 4.69 (s, 4H, CH₂Ph), 4.84 (d, 2H, *J* = 10.5 Hz, CH₂Ph), 4.89 (s, 2H, CH₂Ph), 4.93 (d, 2H, *J* = 10.5 Hz, CH₂Ph), 5.19 (dd, 1H, *J*_{9,8} = 10.5 Hz, *J*_{9,9'} = 2.0 Hz, H-9), 5.31 (dd, 1H, *J*_{9',8} = 16.0 Hz, *J*_{9,9'} = 2.0 Hz, H-9'), 6.00 (ddt, 1H, H-8), 7.24-7.42 (m, 25H, H-Ar). **RMN ¹³C (CDCl₃, 100.6 MHz):** δ (ppm) 72.9 (2CH₂), 73.6 (C-7), 74.1 (C-2), 76.0 (2CH₂), 76.0 (CH₂), 80.9 (C-3 et C-1), 81.8 (C-4 et C-6), 83.8 (C-5), 116.9 (C-9), 127.6 (3C-Ar), 127.8 (5C-Ar), 127.9 (2C-Ar), 128.2 (3C-Ar), 128.4 (3C-Ar), 128.5 (1C-Ar), 128.5 (3C-Ar), 135.9 (C-8), 138.5 (2Cq-Ar), 139.0 (Cq-Ar), 139.0 (2Cq-Ar). **HRMS** (*m*/*z,* ESI): calculé pour C₄₄H₄₆O₆Na, [M+Na]⁺ 693.3187. Trouvé: 693.3177.

### Exemple 41: Préparation du 2,3,4,5,6-penta-O-benzyl-1-O-(2-propèn-1-yl)-scyllo-inositol 55

A une solution de **53** (8g, 12,7mmol) dans du bromure d'allyle (200mL) est additionné NaH (3g, 12,7mmol). Le mélange réactionnel est agité pendant 2h à 70°C. Les solvants sont évaporés sous pression réduite et le résidu dilué dans de l'eau et extrait avec CH₂Cl₂ (3x 50mL). Les phases organiques combinées sont séchées sur MgSO₄, filtrées et les solvants sont évaporés sous pression réduite. Le produit brut est purifié par flash chromatographie sur colonne (gel de silice, AcOEt/Hex: 80/20 à 40/60) pour donner **55** (7,0g, 82% rdt) sous forme d'une huile.

| | |
|---|---|
| 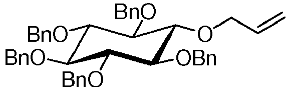 | huile |
| | **Formule** : C₄₄H₄₆O₆ |
| | **M. Mol.:** 670.83 g/mol |
| | **R_{f}** = 0.71 (Hex/AcOEt: 80/20) |

**IR** film (v, cm⁻¹): 2925, 1638, 1067. **RMN ¹H (CDCl₃, 400 MHz):** δ (ppm) 3.39-3.45 (m, 1H, H-4), 3.58-3.57 (m, 5H, H-inositol), 4.36 (bd, 2H, *J*_{7,8} = 5.5 Hz, H-7), 4.82-4.90 (m, 10H, 5CH₂Ph), 5.17 (dd, 1H, *J*_{9,8} = 10.5 Hz, *J*_{9,9'} = 1.5 Hz, H-9), 5.28 (dd, 1H, *J*_{9',8} = 17.0 Hz, *J*_{9,9'} = 1.5 Hz, H-9'), 5.97 (ddt, 1H, H-8), 7.18-7.42 (m, 25H, H-Ar). **RMN ¹³C (CDCl₃, 100.6 MHz):** δ (ppm) 74.8 (C-7), 76.1 (3CH₂), 76.2 (3CH₂), 82.7 (C-4), 83.0 (C-2 et C-6 ou C-3 et C-5), 83.1 (C-1, C-2 et C-6 ou C-3 et C-5), 117.0 (C-9), 127.8 (3C-Ar), 127.8 (2C-Ar), 128.0 (4C-Ar), 128.2 (3C-Ar), 128.5 (8CAr), 135.2 (C-8), 138.7 (5Cq-Ar). **HRMS** (*m*/*z,* ESI): calculé pour C₄₄H₄₆O₆Na, [M+Na]⁺ 693.3187. Trouvé: 693.3185.

### Exemple 42: Préparation du 2-O-(hydroxypropyl)-1,3,4,5,6-penta-O-benzyl-myo-inositol 56

A une solution de **54** (7g, 10mmol) dans du THF anhydre (250mL) est additionné goutte à goutte BH₃ (120mL d'une solution 1M dans l'hexane, 120mmol) sous argon à 0°C. Le mélange réactionnel est agité pendant 6h à température ambiante, puis H₂O₂ 30% (500mL) et une solution aqueuse 2M de NaOH (600mL) sont additionnées lentement. Après 12h, le mélange réactionnel est dilué avec de l'acétate d'éthyle (200mL) puis extrait. La phase aqueuse restante est de nouveau extraite avec de l'acétate d'éthyle (2x100mL). Les phases organiques combinées sont séchées sur MgSO₄, filtrées et les solvants sont évaporés sous pression réduite. Le produit brut est purifié par chromatographie sur colonne (gel de silice, Hex 100% à Hex/AcOEt: 70/30) pour donner **56** (5,6g, 78% rdt) sous la forme d'un solide blanc.

| | |
|---|---|
| 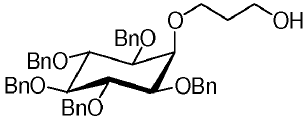 | Solide blanc |
| | **Formule** : C₄₄H₄₈O₇ |
| | **M. Mol.:** 688.85 g/mol |
| | **R_{f}** = 0.30 (Hex/AcOEt: 70/30) |
| | **pf** = 70-71°C |

**IR** film (v, cm⁻¹): 3463, 3030, 2871, 1070. **RMN ¹H (CDCl₃, 400 MHz):** δ (ppm) 1.78 (qt, 2H, *J*_{8,7} = *J*_{8,9} = 5.5 Hz, H-8), 3.32 (dd, 2H, *J*_{3,4} = *J*_{1,6} = 9.5 Hz, *J*_{3,2} = *J*_{1,2} = 2.0 Hz, H-1 et H-3), 3.43 (app t, 1H, *J*_{5,4} = *J*_{5,6} = 9.5 Hz, H-5), 3.74 (app t, 1H, H-2), 3.78 (t, 2H, H-7), 3.92 (t, 2H, H-9), 3.94 (app t, 2H, H-4 et H-6), 4.65 (d, 2H, *J* = 11.5 Hz, CH₂Ph), 4.71 (d, 2H, *J* = 11.5 Hz, CH₂Ph), 4.83 (d, 2H, *J* = 10.5 Hz, CH₂Ph), 4.86 (s, 2H, CH₂Ph), 4.87 (d, 2H, *J* = 11.5 Hz, CH₂Ph), 7.24-7.38 (m, 25H, H-Ar). **RMN ¹³C (CDCl₃, 100.6 MHz):** δ (ppm) 32.2 (C-8), 60.8 (C-9), 72.2 (C-7), 73.4 (2CH₂), 76.1 (2CH₂), 76.1 (CH₂), 76.8 (C-2), 80.5 (C-3 et C-1), 81.8 (C-4 et C-6), 83.5 (C-5), 127.7 (3C-Ar), 128.0 (2C-Ar), 128.1 (5C-Ar), 128.2 (3C-Ar), 128.5 (3C-Ar), 128.5 (1C-Ar), 128.7 (3C-Ar), 138.0 (2Cq-Ar), 138.8 (3Cq-Ar). **HRMS** (*m*/*z,* ESI): calculé pour C₄₄H₄₈O₇Na, [M+Na]⁺ 711.3292. Trouvé: 711.3292.

### Exemple 43: Préparation du 1-O-(hydroxypropyl)-2,3,4,5,6-penta-O-benzyl-scyllo-inositol 57

Ce composé est préparé d'une manière similaire au composé 56 mais à partir d'une solution de **55** (10g, 15mmol) pour donner **57** (7,7g, 75% rdt) sous la forme d'un solide blanc.

| | |
|---|---|
| 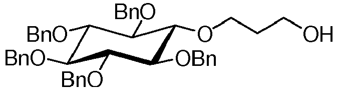 | Solide blanc |
| | **Formule** : C₄₄H₄₈O₇ |
| | **M. Mol.:** 688.85 g/mol |
| | **R_{f}** = 0.18 (Hex/AcOEt: 70/30) |
| | **pf** = 108-109°C |

**IR** film (v, cm⁻¹): 3468, 3030, 2823, 1070. **RMN ¹H (CDCl₃, 400 MHz):** δ (ppm) 1.83 (qt, 2H, *J*_{8,7} = *J*_{8,9} = 6.0 Hz, H-8), 3.37 (app t, 1H, *J*_{3,4} = *J*_{4,5} = 9.5 Hz, H-4), 3.46-3.59 (m, 5H, H-inositol), 3.71 (t, 2H, H-9), 4.00 (t, 2H, H-7), 4.86 (d, 2H, *J* = 11.0 Hz, CH₂Ph), 4.87 (d, 2H, *J* = 11.0 Hz, CH₂Ph), 4.89 (s, 2H, CH₂Ph), 4.90 (d, 2H, *J =* 11.0 Hz, CH₂Ph), 4.92 (d, 2H, *J* = 11.0 Hz, CH₂Ph), 7.26-7.40 (m, 25H, H-Ar). **RMN ¹³C (CDCl₃, 100.6 MHz):** δ (ppm) 33.0 (C-8), 61.5 (C-9), 72.7 (C-7), 75.9 (2CH₂), 75.9 (2CH₂), 76.0 (CH₂), 82.8 (C-2 et C-6 ou C-3 et C-5), 82.9 (C-1), 83.0 (C-2 et C-6 ou C-3 et C-5), 83.1 (C-4), 127.7 (3C-Ar), 127.7 (2C-Ar), 127.8 (4C-Ar), 127.8 (4C-Ar), 127.9 (2C-Ar), 128.4 (5C-Ar), 128.5 (5C-Ar), 138.4 (3Cq-Ar), 138.5 (2Cq-Ar). **HRMS** (*m*/*z,* ESI): calculé pour C₄₄H₄₈O₇Na, [M+Na]⁺ 711.3292. Trouvé: 711.3259.

### Exemple 44: Préparation du 2-O-(hydroxypropyl)-myo-inositol 58

Le composé **56** (2g, 2,9mmol) est dissout dans MeOH (20mL), CH₂Cl₂ (20mL) et H₂O (2mL), puis Pd(OH)₂ (500mg, 20mol%) est additionné. La réaction est placée dans une bombe de Parr et agitée sous atmosphère d'hydrogène à 45Psi toute la nuit. Quand la réaction est finie, le mélange est filtré sur Célite, lavé avec de l'eau et le filtrat est concentré sous pression réduite. Le composé **58** (700mg, rdt quantitatif) est utilisé sans purification.

| | |
|---|---|
| 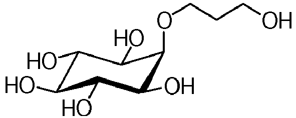 | huile |
| | **Formule** : C₉H₁₈O₇ |
| | **M. Mol.:** 238.23 g/mol |
| | **R_{f}** = 0 (CH₃CN/H₂O: 90/10) |

**IR** film (v, cm⁻¹): 3400, 2901, 1049. **RMN ¹H (D₂O**, **400 MHz):** δ (ppm) 1.85 (qt, 2H, *J*_{8,7} = *J*_{8,9} = 6.0 Hz, H-8), 3.25 (app t, 1H, *J*_{5,4} = *J*_{5,6} = 9.5 Hz, H-5), 3.56 (dd, 2H, *J*_{3,4} = *J*_{1,6} = 9.5 Hz, *J*_{3,2} = *J*_{1,2} = 2.0 Hz, H-1 et H-3), 3.60 (app t, 2H, H-4 et H-6), 3.73 (t, 2H, H-9), 3.86 (m, 3H, H-7 et H-2). **RMN ¹³C (D₂O**, **100.6 MHz):** δ (ppm) 32.0 (C-8), 59.6 (C-9), 71.9 (C-7), 72.0 (C-3 et C-1), 73.1 (C-4 et C-6), 74.8 (C-5), 81.5 (C-2). **HRMS** (*m*/*z,* ESI): calculé pour C₉H₁₈O₇Na, [M+Na]⁺ 261.0945. Trouvé: 261.0903.

### Exemple 45: Préparation du 1-O-(hydroxypropyl)-scyllo-inositol 59

Ce composé est préparé d'une manière similaire au composé 58 mais à partir du composé **57** (2g, 2,9mmol). Le composé **59** (700mg, rdt quantitatif) est utilisé sans purification.

| | |
|---|---|
| 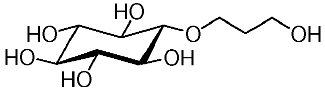 | huile |
| | **Formule** : C₉H₁₈O₇ |
| | **M. Mol.:** 238.23 g/mol |
| | **R_{f}** = 0 (CH₃CN/H₂O: 90/10) |

**IR** film (v, cm⁻¹): 3400, 2923, 1105, 996. **RMN ¹H (D₂O**, **400 MHz):** δ (ppm) 1.92 (qt, 2H, *J*_{7,8} = *J*_{8,9} = 6.5 Hz, H-8), 3.27 (app t, 1H, *J*_{1,2} = *J*_{1,6} = 9.5 Hz, H-1), 3.36-3.50 (m, 5H, H-inositol), 3.78 (t, 2H, H-9), 3.95 (t, 2H H-7). **RMN ¹³C (D₂O**, **100.6 MHz):** δ (ppm) 32.2 (C-8), 59.4 (C-9), 70.6 (C-7), 73.6 (C-2 et C-6 ou C-3 et C-5), 73.9 (C-4), 73.9 (C-2 et C-6 ou C-3 et C-5), 82.8 (C-1). **HRMS** (*m*/*z,* ESI): calculé pour C₉H₁₈O₇Na, [M+Na]⁺ 261.0945. Trouvé: 261.0904.

### Exemple 46: Préparation du 2-O-trityloxypropyl)-1,3,4,5,6-penta-O-acétyl-myo-inositol 60

A une solution de **58** (190mg, 0,8mmol) dans de la pyridine (20mL) sont additionnés du chlorure de trityle (675mg, 2,42mmol) et une quantité catalytique de DMAP. Le mélange réactionnel est agité à 40°C pendant 3 jours, puis la réaction est laissée revenir à température ambiante. De l'anhydride acétique (10mL) est additionné et la solution est agitée pendant 2h. Les solvants sont évaporés sous pression réduite et le résidu est purifié par chromatographie sur colonne (gel de silice, Hex/AcOEt: 80/20 à 60/40) pour donner **60** (440mg, 80% rdt) sous la forme d'un solide blanc et **61** (63mg, 16% rdt) sous la forme d'un solide blanc.

### 2-O-(trityloxypropyl)-1,3,4,5,6-penta-O-acétyl-myo-inositol 60

| | |
|---|---|
| 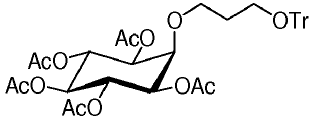 | Solide blanc |
| | **Formule** : C₃₈H₄₂O₁₂ |
| | **M. Mol.:** 690.73 g/mol |
| | **R_{f}** = 0.55 (Hex/AcOEt 50/50) |
| | **pf** = 128-130°C |

**IR** film (v, cm⁻¹): 2936, 1756, 1368, 1230. **RMN ¹H (CDCl₃, 400 MHz):** δ (ppm) 1.84 (qt, 2H, *J*_{8,7} = *J*_{8,9} = 6.5 Hz, H-8), 1.95 (s, 6H, 2Me), 1.99 (s, 3H, Me), 1.99 (s, 6H, 2Me), 3.19 (t, 2H, H-9), 3.72 (t, 2H, H-7), 3.94 (app t, 1H, *J*_{2,1} = *J*_{2,3} = 2.5 Hz, H-2), 4.93 (dd, 2H, *J*_{3,4} = *J*_{1,6} = 10.0 Hz, *J*_{3,2} = *J*_{1,2} = 2.5 Hz, H-1 et H-3), 5.10 (app t, 1H, *J*_{5,4} = *J*_{5,6} = 10.0 Hz, H-5), 5.48 (app t, 2H, H-4 et H-6), 7.22 (brt, 3H, *J* = 7.5 Hz, H-Ar), 7.29 (brt, 6H, *J* = 7.5 Hz, H-Ar), 7.41 (brd, 6H, H-Ar). **RMN ¹³C (CDCl₃, 100.6 MHz):** δ (ppm) 20.6 (Me), 20.7 (4Me), 30.8 (C-8), 60.5 (C-9), 70.0 (C-4 et C-6), 71.0 (C-1 et C-3), 71.2 (C-5), 71.4 (C-7), 76.0 (C-2), 127.0 (3C-Ar), 127.9 (6C-Ar), 128.7 (6C-Ar), 144.3 (3Cq-Ar), 169.7 (2CO), 169.9 (2CO), 170.3 (CO). **HRMS** (*m*/*z,* ESI): calculé pour C₃₃H₄₂O₁₂Na, [M+Na]⁺ 713.2568. Trouvé: 713.2568.

### 2-O-(acétoxypropyl)-1,3,4,5,6-penta-O-acétyl-myo-inositol 61:

| | |
|---|---|
| 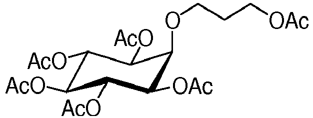 | Solide blanc |
| | **Formule** : C₂₁H₃₀O₁₃ |
| | **M. Mol.:** 490.45 g/mol |
| | **R_{f}** = 0.35 (Hex/AcOEt 50/50) |
| | **pf** = 97-98°C |

**IR** film (v, cm⁻¹): 2935, 1755, 1228. **RMN ¹H (CDCl₃**, **400 MHz):** δ (ppm) 1.90 (t, 2H, *J*_{7,8} = *J*_{8,9} = 6.0 Hz, H-8), 1.98 (s, 3H, Me), 1.98 (s, 6H, 2Me), 2.03 (s, 3H, Me), 2.05 (s, 6H, 2Me), 3.68 (t, 2H, H-7), 3.96 (t, 1H, *J*_{1,2} = *J*_{2,3} = 2.5 Hz, H-2), 4.21 (t, 2H, H-9), 4.96 (dd, *J*_{3,4} = *J*_{1,6} = 10.0 Hz, H-1 et H-3), 5.11 (t, 1H, *J*_{4,5} = *J*_{5,6} = 10.0 Hz, H-5), 5.52 (t, 2H, H-4 et H-6). **RMN ¹³C (CDCl₃, 100.6 MHz):** δ (ppm) 20.6 (Me), 20.7 (2Me), 20.7 (2 Me), 21.0 (Me), 29.6 (C-8), 61.4 (C-9), 69.9 (C-4 et C-6), 70.7 (C-7), 71.1 (C-1 et C-3), 71.3 (C-5), 76.7 (C-2), 169.8 (2CO), 169.9 (2CO), 169.9 (CO), 171.1 (CO). **HRMS** (*m*/*z,* ESI): calculé pour C₂₁H₃₀O₁₃Na, [M+Na]⁺ 513.1579. Trouvé: 513.1598

### Exemple 47: Préparation du 1-O-(trityloxypropyl)-2,3,4,5,6-penta-O-acétyl-scyllo-inositol 62

Ce composé est préparé d'une manière similaire au composé 60 mais à partir d'une solution de **59** (190mg, 0,8mmol) pour donner **62** (418mg, 76% rdt) sous la forme d'un solide blanc et **63** (78mg, 20% rdt) sous la forme d'un solide blanc.

### 1-O-(trityloxypropyl)-2,3,4,5,6-penta-O-acétyl-scyllo-inositol 62:

| | |
|---|---|
| 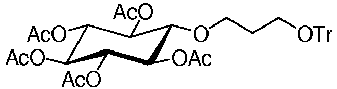 | Solide blanc |
| | **Formule** : C₃₈H₄₂O₁₂ |
| | **M. Mol.:** 690.73 g/mol |
| | **R_{f}** = 0.62 (Hex/AcOEt 50/50) |
| | **pf** = 134-135°C |

**IR** film (v, cm⁻¹): 3061, 1755, 1446, 1228. **RMN ¹H (CDCl₃**, **400 MHz):** δ (ppm) 1.75 (qt, 2H, *J*_{7,8} = *J*_{8,9} = 6.5 Hz, H-8), 1.95 (s, 3H, Me), 1.98 (s, 6H, 2Me), 2.00 (s, 3H, Me), 2.05 (s, 3H, Me), 3.09 (t, 2H, H-9) 3.50 (app t, 1H, *J*_{1,2} = *J*_{1,6} = 7.0 Hz, H-1), 3.68 (t, 2H, H-7), 5.08-5.21 (m, 5H, H-inositol), 7.22 (brt, 3H, *J* = 7.5 Hz, H-Ar), 7.29 (brt, 6H, *J* = 7.5 Hz, H-Ar), 7.38 (brd, 6H, H-Ar). **RMN ¹³C (CDCl₃, 100.6 MHz):** δ (ppm) 20.5 (Me), 20.6 (2Me), 20.7 (2Me), 31.0 (C-8), 60.5 (C-9), 70.2 (C-7), 70.6 (C-2 et C-6 ou C-3 et C-5), 70.8 (C-4), 71.7 (C-2 et C-6 ou C-3 et C-5), 78.1 (C-1), 86.7 (Cq-Tr), 127.1 (3C-Ar), 127.9 (6C-Ar), 128.7 (6C-Ar), 144.3 (3Cq-Ar), 169.3 (2CO), 169.5 (CO), 169.9 (2CO). **HRMS** (*m*/*z,* ESI): calculé pour C₃₃H₄₂O₁₂K, [M+K]⁺ 729.2308. Trouvé: 729.2307.

### 1-O-(acétoxypropyl)-2,3,4,5,6-penta-O-acétyl-scyllo-inositol 63:

| | |
|---|---|
| 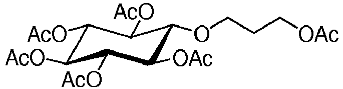 | Solide blanc |
| | **Formule** : C₂₁H₃₀O₁₃ |
| | **M. Mol.:** 490.45 g/mol |
| | **R_{f}** = 0.42 (Hex/AcOEt 50/50) |
| | **pf** = 122-123°C |

**IR** film (v, cm⁻¹): 3061, 1755, 1446, 1228. **RMN ¹H (CDCl₃, 400 MHz):** δ (ppm) 1.79 (qt, 2H, *J*_{7,8} = *J*_{8,9} = 6.0 Hz, H-8), 1.99 (s, 3H, Me), 2.00 (s, 6H, 2Me), 2.03 (s, 3H, Me), 2.06 (s, 6H, 2Me), 3.55 (app t, 1H, J_{1,2} =J_{1,6} = 9.0 Hz, H-1), 3.62 (t, 2H, H-7), 4.05 (t, 2H, H-9), 5.11-5.24 (m, 5H, H-inositol). **RMN ¹³C (CDCl₃**, **100.6 MHz):** δ (ppm) 20.4 (Me), 20.5 (2Me), 20.6 (2Me), 21.0 (Me), 29.4 (C-8), 61.1 (C-9), 69.4 (C-7), 70.0 (C-4), 70.4 (C-2 et C-6 ou C-3 et C-5), 71.5 (C-2 et C-6 ou C-3 et C-5), 78.1 (C-1), 169.3 (2CO), 169.4 (CO), 169.8 (2CO), 170.9 (CO). **HRMS** (*m*/*z,* ESI): calculé pour C₂₁H₃₀O₁₃Na, [M+Na]⁺ 513.1579. Trouvé: 513.1578.

### Exemple 48: Préparation du 2-O-(hydroxypropyl)-1,3,4,5,6-penta-O-acétyl-myo-inositol 64

Ce composé est préparé d'une manière similaire au composé 25 mais à partir d'une solution de **60** (1g, 1,45mmol) et FeCl₃ (1,01g, 6,8mmol) dans du CH₂Cl₂ anhydre (10mL) pour donner le composé **64** (455mg, 70% rdt) sous la forme d'un solide blanc.

| | |
|---|---|
| 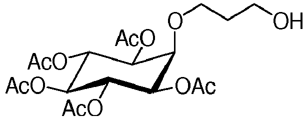 | Solide blanc |
| | **Formule** : C₁₉H₂₈O₁₂ |
| | **M. Mol.:** 448.42 g/mol |
| | **R_{f}** = 0.70 (AcOEt 1 00%) |
| | **pf** = 148-150°C |

**IR** film (v, cm⁻¹): 3448, 2944, 1755, 1229. **RMN ¹H (CDCl₃**, **400 MHz):** δ (ppm) 1.83 (qt, 2H, *J*_{8,7} = *J*_{8,9} = 6.0 Hz, H-8), 1.99 (s, 9H, 3Me), 2.07 (s, 6H, 2Me), 3.78 (t, 2H, H-7), 3.82 (t, 2H, H-9), 4.00 (app t, 1H, *J*_{2,1} = *J*_{2,3} = 2.5 Hz, H-2), 4.97 (dd, 2H, *J*_{3,4} = *J*_{1,6} = 9.5 Hz, *J*_{3,2} = *J*_{1,2} = 2.5 Hz, H-1 et H-3), 5.13 (app t, 1H, *J*_{5,4} = *J*_{5,6} = 9.5 Hz, H-5), 5.23 (app t, 2H, H-4 et H-6). **RMN ¹³C (CDCl₃**, **100.6 MHz):** δ (ppm) 20.6 (Me), 20.7 (2Me), 20.8 (2Me), 32.6 (C-8), 60.2 (C-9), 69.9 (C-4 et C-6), 71.1 (C-1, C-3 et C-5), 71.7 (C-7), 76.4 (C-2), 169.9 (2CO), 169.9 (2CO), 169.9 (CO). **HRMS** (*m*/*z,* ESI): calculé pour C₁₉H₂₈O₁₂Na, [M+Na]⁺ 471.1473. Trouvé: 471.1473.

### Exemple 49: Préparation du 1-O-(hydroxypropyl)-2,3,4,5,6-penta-O-acétyl-scyllo-inositol 65

Ce composé est préparé d'une manière similaire au composé 25 mais à partir d'une solution de **62** (1g, 1,45mmol) et FeCl₃ (1,01g, 6,8mmol) dans du CH₂Cl₂ anhydre (10mL) pour donner **65** (474mg, 73% rdt) sous la forme d'un solide blanc.

| | |
|---|---|
| 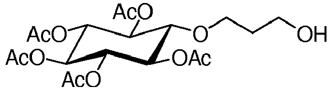 | Solide blanc |
| | **Formule** : C₁₉H₂₈O₁₂ |
| | **M. Mol.:** 448.42 g/mol |
| | **R_{f}** = 0.75 (AcOEt 100%) |
| | **pf** = 184-185°C |

**IR** film (v, cm⁻¹): 3472, 2941, 1747, 1226. **RMN ¹H (CDCl₃, 250 MHz):** δ (ppm) 1.74 (qt, *J*_{7,8} = *J*_{8,9} = 6.0 Hz, H-8), 2.02 (s, 3H, Me), 2.03 (s, 6H, 2Me), 2.10 (s, 6H, 2Me), 3.58 (app t, 1H, *J*_{1,2} = *J*_{1,6} = 10.0 Hz, H-1), 3.67 (t, 2H, H-9), 3.73 (t, 2H, H-7), 5.14-5.27 (m, 5H, H-inositol). **RMN ¹³C (CDCl₃, 62.9 MHz):** δ (ppm) 20.6 (Me), 20.6 (2Me), 20.8 (2Me), 32.7 (C-8), 60.2 (C-9), 70.2 (C-7), 10.5 (C-2 et C-6 ou C-3 et C-5), 70.7 (C-4), 71.5 (C-2 et C-6 ou C-3 et C-5), 78.3 (C-1), 169.5 (CO), 169.7 (2CO), 169.9 (2CO). **HRMS** (*m*/*z*, ESI): calculé pour C₁₉H₂₈O₁₂Na, [M+Na]⁺ 471.1473. Trouvé: 471.1471.

### Exemple 50: Préparation du 2-O-(tosyloxypropyl)-1,3,4,5,6-penta-O-acétyl-myo-inositol 66

Ce composé est préparé d'une manière similaire au composé 31 mais à partir d'une solution de **64** (100mg, 0,21 mmol), chlorure de tosyle (110mg, 0,56mmol) et triéthylamine (0,2mL, 0,4mmol) pour donner **66** (103mg, 77% rdt) sous forme de solide blanc.

| | |
|---|---|
| 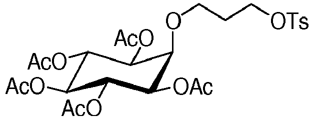 | huile |
| | **Formule** : C₂₆H₃₄O₁₄S |
| | **M. Mol.:** 602,60 g/mol |
| | **R_{f}** = 0.48 (Hex/AcOEt: 50/50) |

**IR** film (v, cm⁻¹): 2924, 1754, 1368, 1227. **RMN ¹H (CDCl₃, 250 MHz):** δ (ppm) 1.92 (qt, 2H, *J*_{7,8} = *J*_{8,9} = 6.0 Hz, H-8), 1.99 (s, 9H, 3Me), 2.04 (s, 6H, 2Me), 2.45 (s, 3H, Me-Ts), 3.68 (t, 2H, H-9), 3.92 (app t, 1H, *J*_{1,2} = *J*_{2,3} = 2.5 Hz, H-2), 4.22 (t, 2H, H-7), 4.96 (dd, 2H, *J*_{3,4} = *J*_{1,6} = 10.0 Hz, H-1 et H-3), 5.10 (app t, 1H, *J*_{4,5} = *J*_{5,6} = 10.0 Hz, H-5), 5.46 (app t, 2H, H-4 et H-6), 7.36 (d, 2H, *J* = 8.0 Hz, H-Ar), 7.80 (d, 2H, *J* = 8.0 Hz, H-Ar). **RMN ¹³C (CDCl₃, 62.9 MHz):** δ (ppm) 20.7 (5Me), 21.9 (Me-Ts), 29.8 (C-8), 67.4 (C-9), 69.7 (C-7), 70.0 (C-4 et C-6), 71.1 (C-1 et C-3), 71.4 (C-5), 76.9 (C-2), 128.1 (2C-Ar), 130.2 (2C-Ar), 134.2 (Cq-Ar), 145.0 (Cq-Ar), 169.8 (2CO), 169.9 (3CO). **HRMS** (*m*/*z,* ESI): calculé pour C₂₆H₃₄O₁₄SNa, [M+Na]⁺ 625.1561. Trouvé: 625.1548.

### Exemple 51: Préparation du 1-O-(tosyloxypropyl)-2,3,4,5,6-penta-O-acétyl-scyllo-inositol 67

Ce composé est préparé d'une manière similaire au composé 31 mais à partir d'une solution de **65** (100mg, 0,21 mmol), chlorure de tosyle (110mg, 0,56mmol) et triéthylamine (0,2mL, 0,4mmol) pour donner **67** (98mg, 73% rdt) sous forme de solide blanc.

| | |
|---|---|
| 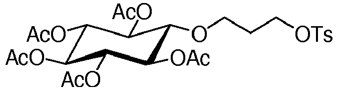 | Solide blanc |
| | **Formule** : C₂₆H₃₄O₁₄S |
| | **M. Mol.:** 602,60 g/mol |
| | **R_{f}** = 0.48 (Hex/AcOEt: 50/50) |
| | **pf** = 129-130°C |

**IR** film (v, cm⁻¹): 2928, 1747, 1232. **RMN ¹H (CDCl₃, 400 MHz):** δ (ppm) 1.79 (qt, 2H, *J*_{7,8} = *J*_{8,9} = 6.0 Hz, H-8), 1.98 (s, 3H, Me), 1.98 (s, 6H, 2Me), 2.03 (s, 6H, 2Me), 2.45 (s, 3H, Me-Ts), 3.47 (app t, 1H, *J*_{1,2} = *J*_{1,6} = 10.0 Hz, H-1), 3.62 (t, 2H, H-7), 4.00 (t, 2H, H-9), 5.06-5.13 (m, 5H, H-inositol), 7.35 (d, 2H, *J =* 8.0 Hz, H-Ar), 7.76 (d, 2H, H-Ar). **RMN ¹³C (CDCl₃, 100.6 MHz):** δ (ppm) 20.5 (Me), 20.6 (2Me), 20.7 (2Me), 21.8 (Me-Ts), 30.0 (C-8), 67.2 (C-9), 68.6 (C-7), 70.2 (C-4), 70.5 (C-2 et C-6 ou C-3 et C-5), 71.6 (C-2 et C-6 ou C-3 et C-5), 78.2 (C-1), 128.0 (2C-Ar), 130.1 (2C-Ar), 133.2 (Cq-Ar), 145.0 (Cq-Ar), 169.5 (CO), 169.5 (2CO), 169.8 (2CO). **HRMS** (*m*/*z,* ESI): calculé pour C₂₆H₃₄O₁₄SNa, [M+Na]⁺ 625.1561. Trouvé: 625.1574.

### Exemple 52: Préparation du 2-O-(fluoropropyl)-1,3,4,5,6-penta-O-acétyl-myo-inositol 68

Ce composé est préparé d'une manière similaire au composé 49 mais à partir d'une solution de **64** (50mg, 0,1mmol) dans du CH₂Cl₂ anhydre (8mL) est additionné du DAST (0,2mL) pour donner **68** (48mg, 95% rdt) sous la forme d'un solide blanc.

| | |
|---|---|
| 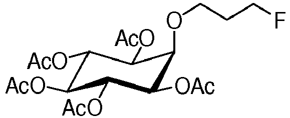 | Solide blanc |
| | **Formule** : C₁₉H₂₇FO₁₁ |
| | **M. Mol.:** 450.41 g/mol |
| | **R_{f}** = 0.30 (Hex/AcOEt: 50/50) |
| | **pf** = 156-157°C |

**IR** film (v, cm⁻¹): 2971, 1755, 1640, 1229. **RMN ¹H (CDCl₃**, **400 MHz):** δ (ppm) 1.95 (dqt, 2H, *J*_{H,F} = 27.0 Hz, *J*_{8,7} = *J*_{8,9} = 5.5 Hz, H-8), 1.99 (s, 3H, Me), 2.00(s, 6H, 2Me), 2.06 (s, 6H, 2Me), 3.73 (t, 2H, H-7), 3.99 (app t, 1H, *J*_{2,1} = *J*_{2,3} = 2.5 Hz, H-2), 4.63 (dt, 2H, *J*_{H,F} = 47.0 Hz, *J*_{9,8} = 5.5 Hz, H-9), 4.95 (dd, 2H, *J*_{3,4} = *J*_{1,6} = 10.0 Hz, *J*_{3,2} = *J*_{1,2} = 2.5 Hz, H-1 et H-3), 5.12 (app t, 1H, *J*_{5,4} = *J*_{5,6} = 10.0 Hz, H-5), 5.53 (app t, 2H, H-4 et H-6). **RMN ¹³C (CDCl₃, 100.6 MHz):** δ (ppm) 20.6 (Me), 20.7 (4Me), 31.3 (d, *J*_{C,F} = 20.0 Hz, C-8), 69.7 (d, *J*_{C,F} = 4.5 Hz, C-7), 69.9 (C-4 et C-6), 71.1 (C-1 et C-3), 71.2 (C-5), 76.8 (C-2), 80.4 (d, *J*_{C,F} = 164.0 Hz, C-9), 169.9 (2CO), 169.9 (CO), 170.0 (2CO). **RMN ¹⁹F (CDCl₃, 235 MHz):** δ (ppm) -223.3 (tt, *J*_{H,F} = 47.0 Hz, *J*_{H,F} = 27.0 Hz). **HRMS** (*m*/*z,* ESI): calculé pour C₁₉H₂₇FO₁₁Na, [M+Na]⁺ 473.1430. Trouvé: 473.1410.

### Exemple 53: Préparation du 1-O-(fluoropropyl)-2,3,4,5,6-penta-O-acétyl-scyllo-inositol 69

Ce composé est préparé d'une manière similaire au composé 49 mais à partir d'une solution de **65** (50mg, 0,1mmol) dans du CH₂Cl₂ anhydre (8mL) est additionné du DAST (0,2mL) pour donner **69** (46mg, 92% rdt) sous la forme d'un solide blanc.

| | |
|---|---|
| 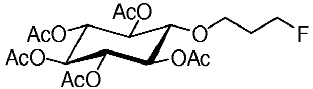 | Solide blanc |
| | **Formule** : C₁₉H₂₇FO₁₁ |
| | **M. Mol.:** 450.41 g/mol |
| | **R_{f}** = 0.33 (Hex/AcOEt: 50/50) |
| | **pf =** 199-200°C |

**IR** film (v, cm⁻¹): 2961, 1745, 1227. **RMN ¹H (CDCl₃, 400 MHz):** δ (ppm) 1.84 (dt, 2H, *J*_{H,F} = 27.0 Hz, *J*_{7,8} = *J*_{8,9} = 6.0 Hz, H-8), 2.01 (s, 3H, Me), 2.02 (s, 6H, 2Me), 2.07 (s, 6H, 2Me), 3.56 (app t, 1H, *J*_{1,2} = *J*_{1,6} = 9.0 Hz , H-1), 3.68 (t, 2H, H-7), 4.44 (dt, *J* _{H,F} = 47.0 Hz, 2H, H-9), 5.14-5.25 (m, 5H, H-inositol). **RMN ¹³C (CDCl₃, 100.6 MHz):** δ (ppm) 20.6 (Me), 20.6 (2Me), 20.7 (2Me), 31.1 (d, *J*_{C,F} = 20.0 Hz, C-8), 68.6 (d, *J*_{C,F} = 5.0 Hz, C-7), 70.3 (C-4), 70.5 (C-2 et C-6 ou C3 et C-5), 71.7 (C-2 et C-6 ou C-3 et C-5),78.2 (C-1), 80.3 (d, *J*_{C,F} = 163.0 Hz, C-9), 169.5 (CO), 169.6 (2CO), 169.9 (2CO). **RMN ¹⁹F (CDCl₃, 235 MHz):** δ (ppm) -223.5 (tt, *J*_{H,F} = 47.0 Hz, *J*_{H,F} = 27.0 Hz). **HRMS** (*m*/*z,* ESI): calculé pour C₁₉H₂₇FO₁₁Na, [M+Na]⁺ 473.1430. Trouvé: 473.1451.

### Exemple 54: Préparation du 2-O-(fluoropropyl)-myo-inositol 70

Ce composé est préparé d'une manière similaire au composé 37 mais à partir d'une solution de **68** (100mg, 0,22mmol) pour donner **70** (50mg, quantitatif) sous forme d'une huile.

| | |
|---|---|
| 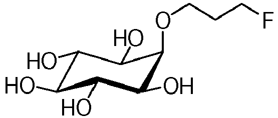 | huile |
| | **Formule :** C₉H₁₇FO₆ |
| | **M. Mol.:** 240.23 g/mol |
| | **R_{f} =** 0.43 (CH₃CN/H₂O: 90/10) |

**IR** film (v, cm⁻¹): 3400, 2924, 999. **RMN ¹H (D₂O, 400 MHz):** δ (ppm) 1.99 (dqt, 2H, *J*_{H,F} = 27.5 Hz, *J*_{8,7} = *J*_{8,9} = 6.0 Hz, H-8), 3.21 (app t, 1H, *J*_{5,4} = *J*_{5,6} = 9.5 Hz, H-5), 3.53 (dd, 2H, *J*_{3,4} = *J*_{1,6} = 9.5 Hz, *J*_{3,2} = *J*_{1,2} = 2.5 Hz, H-1 et H-3), 3.59 (app t, 2H, H-4 et H-6), 3.82 (app t, 1H, H-2), 3.85 (t, 2H, H-7), 4.61 (dt, 2H, *J*_{H,F} = 47.0 Hz, *J*_{9,8} = 6.0 Hz, H-9). **RMN ¹³C (D₂O, 100.6 MHz):** δ (ppm) 30.8 (d, *J*_{C,F} = 19.0 Hz, C-8), 70.4 (d, *J*_{C,F} = 6.0 Hz, C-7), 71.9 (C-4 et C-6), 73.0 (C-1 et C-3), 74.9 (C-5), 81.4 (C-2), 82.9 (d, *J*_{C,F} = 157.0 Hz, C-9). **RMN ¹⁹F (D₂O**, **235 MHz):** δ (ppm) -219.4 (tt, *J*_{H,F} = 47.0 Hz, *J*_{H,F} = 27.5 Hz). **HRMS** (*m*/*z,* ESI): calculé pour C₉H₁₇FO₆Na, [M+Na]⁺ 263.0901. Trouvé: 263.0913.

### Exemple 55: Préparation du 1-O-(fluoropropyl)-scyllo-inositol 71

Ce composé est préparé d'une manière similaire au composé 37 mais à partir d'une solution de **69** (100mg, 0,22mmol) pour donner **71** (50mg, quantitatif) sous forme d'une huile.

| | |
|---|---|
| 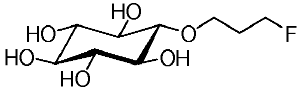 | Huile |
| | **Formule :** C₉H₁₇FO₆ |
| | **M. Mol.:** 240.23 g/mol |
| | **R_{f}** = 0.43 (CH₃CN/H₂O: 90/10) |

**IR** film (v, cm⁻¹): 3410, 2923,1002. **RMN ¹H (D₂O**, **400 MHz):** δ (ppm) 2.01 (dqt, 2H, *J*_{H,F} = 26.0 Hz, *J*_{7,8} = *J*_{8,9} = 6.0 Hz, H-8), 3.27 (app t, 1H, *J*_{1,2} = *J*_{1,6} = 9.0 Hz, H-1), 3.32-3.49 (m, 5H, H-inositol), 3.97 (t, 2H, H-7), 4.69 (dt, 2H, *J*_{H,F} = 47.0 Hz, H-9). **RMN ¹³C (D₂O, 100.6 MHz):** δ (ppm) 30.8 (d, *J*_{C,F} = 17.0 Hz, C-8), 69.5 (d, *J*_{C,F} = 5.5 Hz, C-7), 73.6 (C-2 et C-6 ou C3 et C-5), 73.8 (C-4), 73.9 (C-2 et C-6 ou C3 et C-5), 82.8 (d, *J*_{C,F} = 157.0 Hz, C-9), 82.8 (C-1). **RMN ¹⁹F (D₂O**, **235 MHz):** δ (ppm) -219.6 (tt, *J*_{H,F} = 47.0 Hz, *J*_{H,F} = 28.0 Hz). **HRMS** (*m*/*z,* ESI): calculé pour C₉H₁₇FO₆Na, [M+Na]⁺ 263.0901. Trouvé: 263.0907.

### Exemple 56: Préparation de la radiosvnthèse

### 1) Production de [¹⁸F]⁻

[¹⁸F]fluorures sont produits par un bombardement de protons sur de l'eau enrichie ¹⁸O-eau (>95%) via la réaction nucléaire ¹⁸O(p,n)¹⁸F. La cible d'argent a été remplie de 2,5mL d'eau et a été irradiée pendant quelques minutes (11µA.h à 40µA) avec 18MeV proton. A la fin de l'irradiation, les [¹⁸F]fluorures dans l'eau sont transférés par un tube en Teflon de 50 m jusqu'au laboratoire de recherche.

### 2) Activation de [18F]-

### Préparation of [n-DDTMA][¹⁸F]F⁻/DMSO:

100 mg de copolymère sorbant N-vinyl lactame (Waters Oasis HLB), chargé avec 20mg de n-dodecyl-triméthylammonium bromide ([n-DDTMA⁺][Br⁻]), est conditionné dans une cartouche (réservoir Isolute vide de 1mL, diamètre interne de 5-6mm) avec 500mL d'une solution de 1g de carbonate de potassium dans 1mL d'eau. La cartouche est rincée avec 3mL d'eau. La solution d'eau contenant les [¹⁸F]F⁻ est passée sur la cartouche d'extraction à 1mL/min en utilisant une pompe à seringue motorisée. La cartouche est séchée pendant 4min avec un flux d"azote (10L/min). La radioactivité est éluée de la cartouche avec 2mL de DMSO pour obtenir la solution [n-DDTMA⁺][¹⁸F]F⁻/DMSO (environ 85% de l'activité piégée a été éluée).

### Préparation of [¹⁸F]F⁻[P₂EtH]⁺:

Les [¹⁸F]fluorures dans l'eau sont piégés sur une cartouche Sep Pak light QMA. Après séchage avec du CH₃CN anhydre, les ¹⁸F-fluorures sont éluées du support avec 1mL d'une solution de CH₃CN contenant 5500ppm d'eau et 15µL de P₂Et. Dans ce cas 99% de l'activité est éluée dans environ 0,6mL de la solution.

### 3) Radiosynthèses

### Radiosynthèse du 1-C-([¹⁸F]-fluorométhyl)-scyllo-inositol [¹⁸F]4:

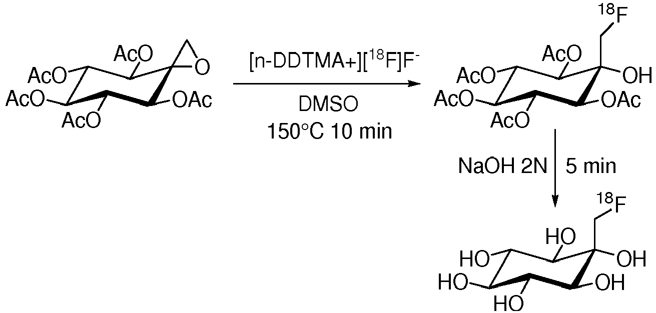

A 5,4mg de précurseur **4** (0,013mmol) dissout dans 300µL de DMF est additionné 200µL de [n-DDTMA⁺][¹⁸F]F⁻/DMSO (4,85mCi). Le mélange réactionnel est chauffé à 150°C pendant 10min. L'incorporation du [¹⁸F]F⁻ est vérifié par une radio-CCM (gel de silice, ACN/H₂O 90/10; Rf : [¹⁸F]fluorure : 0 ; **[¹⁸F]4'** : 0,95 ; pureté radiochimique : 28%). Le mélange est dilué avec 20mL d'eau et passé sur une cartouche de C18. Le produit-¹⁸F est fixé sur la cartouche puis l'hydrolyse avec NaOH 2N est effectuée pendant 5min, le produit est ensuite élué avec de l'eau. L'efficacité de la déprotection est vérifiée par radio-CCM (gel de silice, ACN/H₂O 90/10 ; Rf : [¹⁸F]fluorures : 0 ; **[¹⁸F]4"** : 0,50; pureté radiochimique : 26%). Le rendement de déprotection est de 32% et le rendement radiochimique total corrigé de la décroissance est de 9%.

### Radiosynthèse du 2-C-([18F]-fluoroéthyl)-1,2,3,4,5,6-hexa-O-acétyl-myo-inositol [18F] 72 :

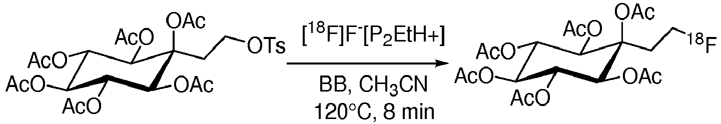

A 5,9mg de précurseur **48** (0,009mmol) dissout dans 400µL de CH₃CN est additionné 200µL de [¹⁸F]F⁻[P₂EtH]⁺ (1,95mCi) et 15µL de base de Barton. Le mélange réactionnel est chauffé à 120°C pendant 10min. L'incorporation du [¹⁸F]F⁻est vérifié par une radio-CCM (gel de silice, Hex/AcOEt : 50/50 ; Rf : [¹⁸F]fluorures : 0 ; **[¹⁸F]72** : 0,5). Le rendement radiochimique corrigé de la décroissance est de 1,2 %.

### Radiosynthèse du 1-C-([18F]-fluoropropyl)-scyllo-inositol [¹⁸F] 52 :

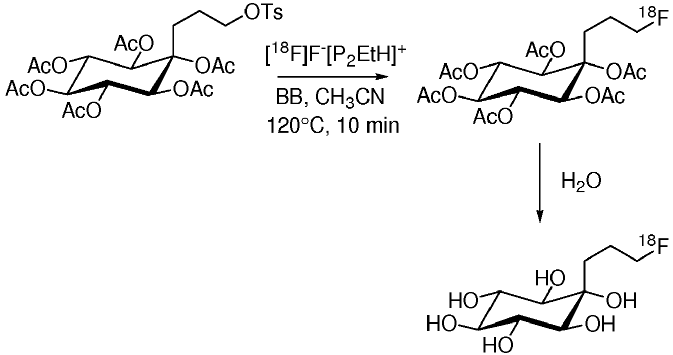

A 5,2mg de précurseur **45** (0,008mmol) dissout dans 400µL de CH₃CN est additionné 100µL de [¹⁸F]F⁻[P₂EtH]⁺ (3,12mCi) et 10µL de base de Barton. Le mélange réactionnel est chauffé à 120°C pendant 10min. L'incorporation du [¹⁸F]F⁻est vérifié par une radio-CCM (gel de silice, Hex/AcOEt 50/50 ; Rf : [¹⁸F]fluorures : 0; **[¹⁸F]49** : 0,4 ; pureté radiochimique : 45%). Le rendement radiochimique corrigé de la décroissance est de 39 %. Le mélange est dilué avec 5 mL d'eau et passé sur une cartouche de C18. Le produit **[¹⁸F]49** n'est pas fixé sur la cartouche car il a été déprotégé *in situ* en **[¹⁸F]52**. L'efficacité de la déprotection est vérifiée par radio-CCM (gel de silice, ACN/H₂O : 90/10 ; Rf : [¹⁸F]fluorures : 0 ; **[¹⁸F]52** : 0,48; pureté radiochimique : 26%). Le rendement de déprotection est de 68% et le rendement radiochimique total de **[¹⁸F]52** corrigé de la décroissance est de 26%.

### Radiosynthèse du 2-C-([18F]-fluoropropyl)-myo-inositol [¹⁸F] 51 :

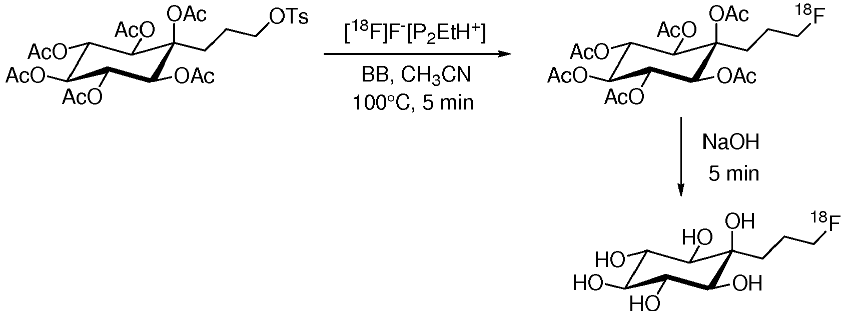

A 5,9mg de précurseur **46** (0,009mmol) dissout dans 400µL de CH₃CN est additionné 200µL de [¹⁸F]F⁻[P₂EtH]⁺ (1,714mCi) et 15µL de base de Barton. Le mélange réactionnel est chauffé à 120°C pendant 5 min. L'incorporation du [¹⁸F]F⁻est vérifié par une radio-CCM (gel de silice, Hex/AcOEt 50/50 ; Rf : [¹⁸F]fluorures : 0 ; **[¹⁸F]50**: 0,50 ; pureté radiochimique : 66%). Le rendement radiochimique corrigé de la décroissance est de 50 %. Le mélange est dilué avec 20mL d'eau et passé sur une cartouche de C18. Le produit **[¹⁸F]50** est fixé sur la cartouche puis l'hydrolyse avec NaOH 2N est effectuée pendant 5min, le produit est ensuite élué avec de l'eau. L'efficacité de la déprotection est vérifiée par radio-CCM (gel de silice, ACN/H₂O 90/10 ; Rf : [¹⁸F]fluorures : 0 ; **[¹⁸F]51** : 0.32). Le rendement de déprotection est de 36,5% et le rendement radiochimique total de **[¹⁸F]51** corrigé de la décroissance est de 18,5%.

### Exemple 57 : HPLC des composés inositols selon l'invention

### Mode opératoire :

Les temps de rétention ont été déterminés par utilisation d'un détecteur ELSD, ces molécules n'absorbant pas en UV. Les meilleurs résultats ont été obtenus sur des colonnes amino. Les conditions d'élution ont été optimisées en élution isochratique pour permettre la meilleure séparation possible avec des temps de rétention compatibles avec la radiochimie (< 20 min).

Les dérivés inositol suivants (se créant ou pouvant se créer lors de la radiosyntèse) ont été injectés dans l'éluant 70/30 ACN/H₂O avec un débit de 1 mL par minute. Ils présentent les temps de rétention suivants :

### Série myo-inositol :

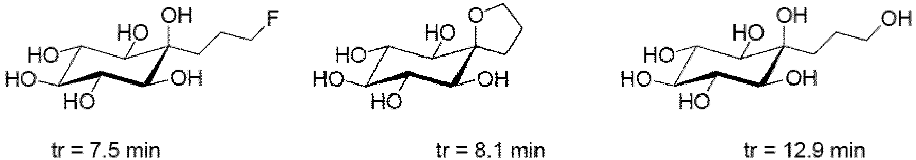

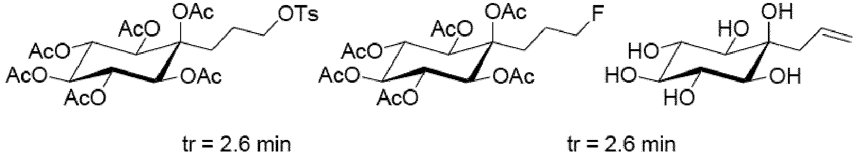

### Série scyllo-inositol :

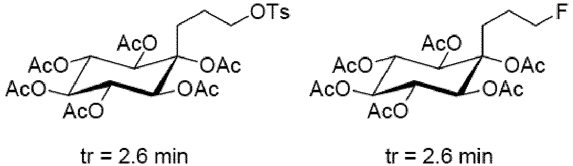

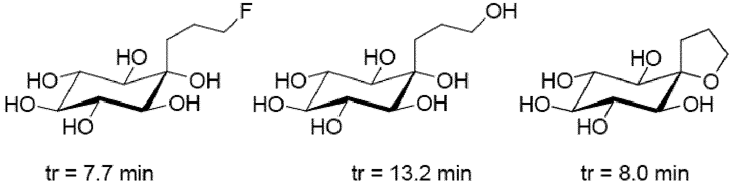

### Conclusion :

Ces dérivés montrent des temps de rétention inférieurs à 20 minutes (conditions nécessaires à la radiochimie), les deux références froides (équivalent du radiotraceur) éluent en 7.5 et 7.7 minutes et sont facilement séparables des autres composés de la même série, notamment des précurseurs de marquage correspondant qui ont un temps de rétention de 2.6 minutes.

Ces résultats permettent d'obtenir une bonne séparation de la référence froide et donc de purifier correctement le radiotraceur.

### Exemple 58: Radiosynthèse sur automate et obtention des images TEP

### Radiosvnthèse :

La radiosynthèse du radiotraceur s'effectue avec un automate All-In-One (Trasis).

Le [18F]F⁻ est piégé sur une cartouche Sep Pak light QMA, qui est ensuite éluée avec 1mL d'une solution CH₃CN/H₂O contenant du K₂₂₂ et du K₂CO₃. La solution est évaporée sous flux d'azote, le fluor est ainsi séché. Le précurseur est ensuite additionné dans de l'acétonitrile sec, puis la solution est chauffée à 95°C pendant 900 s. Le produit est ensuite déprotégé par une base forte (MeONa) puis la solution est neutralisée avec une solution d'HCl diluée. La solution est ensuite purifiée sur HPLC (colonne amino) avec un éluant H₂O/EtOH 80/20. Le rendement radiochimique final est de 11%.

### Préparation de l'animal, injection et obtention des images :

Après anesthésie générale par inhalation d'un mélange isoflurane-oxygène (2% - 1,5 v/v), et une mesure de la glycémie, un volume moyen de 0,4 mL, contenant 15 MBq de **[¹⁸F]69**, est injecté par voie intraveineuse (veine caudale) à une souris xénogreffée cancer du sein. Une acquisition statique d'une durée de 3 heures est débutée immédiatement après injection du traceur. Pendant cet enregistrement, la souris est maintenue sous anesthésie générale et placée en décubitus ventral.

A la fin de l'examen, et dans le but de corriger le phénomène d'atténuation, une acquisition de transmission d'une durée de 6 minutes est réalisée à l'aide de deux sources de cobalt-57 incluses dans l'appareil du MicroTEP. La reconstruction des images acquises est effectuée à partir des images obtenues lors de l'acquisition statique d'émission et de l'acquisition de transmission, et est réalisée selon les paramètres suivants: matrice 128*128, zoom 2, épaisseur de coupe 0,8 mm, aboutissant à des voxels de taille 0,4x0, 4x0, 8 mm et une résolution spatiale proche de 1 mm.

Les figures 1 et 2 représentent une image, respectivement axiale et dans le plan sagital, acquises au MicroTep après injection du composé **[¹⁸F]69** chez une souris Balbc portant un cancer du sein de 28 jours. Les flèches A et B montrent la fixation tumorale deux heures après injection du traceur.

## Revendications

1. Composé de formule (I) : 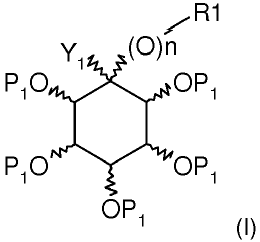 dans laquelle
- n est un entier égal à 0 ou 1,
- R1 représente
∘ quand n=0, une chaîne alkyle de 3 à 6 atomes de carbone de préférence linéaire, une chaîne alkényle, alkylène, une chaîne hydroxyalkyle linéaire de 2 à 6 atomes de carbone, la fonction hydroxyle étant éventuellement protégée ou l'oxygène de cette fonction étant substitué de manière à former un groupement partant, une chaîne halogénoalkyle, l'halogène étant radioactif ou non, de 2 à 6 atomes de carbone, ou un groupe fluorométhyle, le fluor étant radioactif ou non
∘ quand n=1, une chaîne monohydroxyalkyle de 1 à 6 atomes de carbone, la fonction hydroxyle, en position terminale, étant éventuellement protégée ou l'oxygène de cette fonction étant substitué de manière à former un groupement partant, ou monohalogénoalkyle, l'halogène, en position terminale, étant radioactif ou non, de 1 à 6 atomes de carbone, l'halogène étant différent du brome
- P₁ représente
∘ quand n=0 : chacun un hydrogène ou chacun un groupement protecteur identique de la fonction hydroxyle
∘ quand n=1 et R1 est une chaîne monohydroxyalkyle : chacun un hydrogène ou chacun un groupement protecteur identique de la fonction hydroxyle,
∘ quand n=1 et R1 est une chaîne monohalogénoalkyle : chacun un hydrogène ou chacun un groupement protecteur identique de la fonction hydroxyle
- Y1 représente :
∘ quand n=0, un groupement OP₁, où P₁ est tel que défini ci-avant, ou,
∘ quand n=1, un atome d'hydrogène,
à l'exception des composés 2-fluorométhyl-myo-inositols non radiomarqués pour lesquels P₁, identique ou différent, représente un atome d'hydrogène ou un groupement protecteur benzyle.

2. Composé selon la revendication 1 de formule (II) : 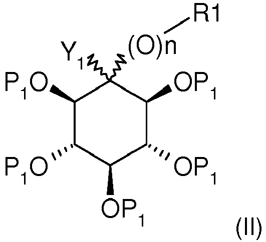 dans laquelle n, R1, P₁ et Y₁ sont tels que définis dans la revendication 1.

3. Composé selon la revendication 1 ou 2, dans lequel R1 est une chaîne fluoroalkyle de préférence de 2 à 6 atomes de carbone, le fluor étant en position terminale sur la chaîne alkyle et non radioactif.

4. Composé selon la revendication 1 ou 2, dans lequel R1 est une chaîne fluoroalkyle, de préférence de 2 à 6 atomes de carbone, le fluor étant en position terminale sur la chaîne alkyle et radioactif.

5. Composé selon l'une quelconque des revendications 1 à 4, dans lequel P₁ est un hydrogène.

6. Composé selon la revendication 1 ou 2, dans lequel R1 est une chaîne monohydroxyalkyle de 1 à 6 atomes de carbone dans laquelle la fonction hydroxyle est terminale et l'oxygène de cette fonction est substitué de manière à former un groupement partant.

7. Composé selon l'une quelconque des revendications 2 à 6, dans lequel le groupement (O)ₙ-R1 est en position axiale.

8. Composé selon l'une quelconque des revendications 2 à 6, dans lequel le groupement (O)ₙ-R1 est en position équatoriale.

9. Procédé de synthèse d'un composé de formule (XI) : 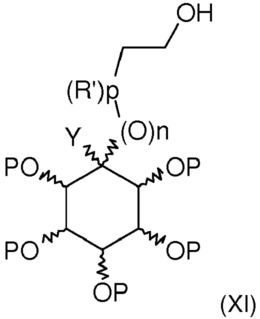 par hydroxylation d'un composé de formule (XII) 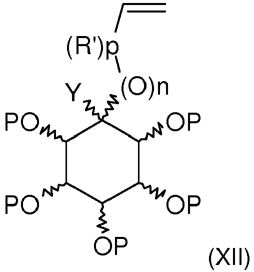 dans lesquelles :
- n est un entier égal à 0 ou 1,
- Y représente :
∘ quand n=0, un groupement OH, ou,
∘ quand n=1, un atome d'hydrogène,
- R' représente une chaîne alkyle de 1 atome de carbone,
- p est un entier compris entre 0 et 1, et
- P représente un groupement protecteur.

10. Procédé de synthèse d'un composé de formule (XII), 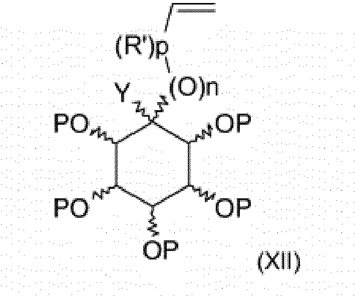 dans laquelle
- n=0, Y est un groupement OH,
- R' est une chaîne alkyle de 1 atome de carbone,
- p, un entier compris entre 0 et 1 et P désigne un groupement protecteur, par réaction d'un composé de formule (XV) ou (XV') : 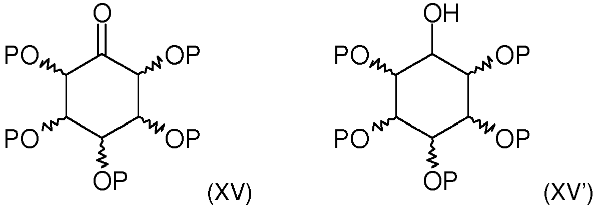
dans laquelle P est tel que décrit ci-avant, avec un organomagnésien de formule CH₂=CH-(R')p-MgBr pour le composé (XV), ou avec un bromure d'alkényle de formule CH₂=CH-(R')p-Br pour le composé (XV').

11. Procédé de synthèse d'un composé de formule (XXIII) : 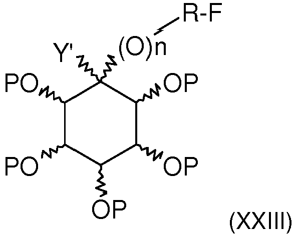 par fluoration d'un composé de formule (XXIV) : 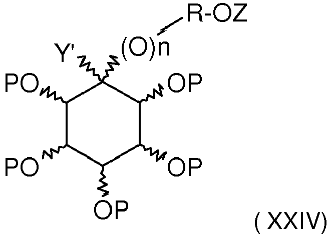 dans lesquelles :
- n est un entier égal à 0 ou 1,
- Y' représente :
∘ quand n=0, un oxygène portant un groupement protecteur, ou,
∘ quand n=1, un atome d'hydrogène,
- R représente une chaîne alkyle de 1 à 6 atomes de carbone,
- F est un atome de fluor non radioactif,
- P représente un groupement protecteur, et
- Z représente un groupement tosyle ou un atome d'hydrogène.

12. Procédé de synthèse d'un composé de formule (XXVII) : 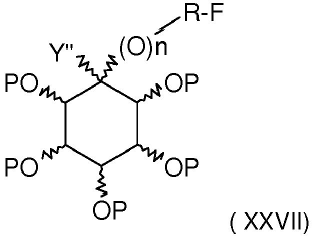 par fluoration d'un composé de formule (XXVIII), 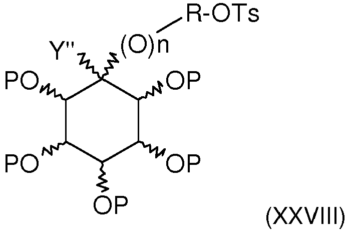 dans lesquelles :
- n est un entier égal à 0 ou 1,
- Y" représente :
∘ quand n=0, un groupement OP ou,
∘ quand n=1, un atome d'hydrogène,
- R représente une chaîne alkyle de 1 à 6 atomes de carbone,
- F est un atome de fluor radioactif,
- P représente un groupement protecteur, et
- Ts représente un groupement tosyle.

13. Procédé de synthèse d'un composé de formule (XXXI) à l'exception des composés 2-fluorométhyl-myo-inositols non radiomarqués pour lesquels P₁, identique, représente un atome d'hydrogène: 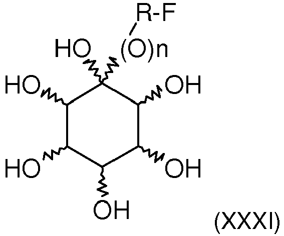 par déprotection d'un composé de formule (XXIII) 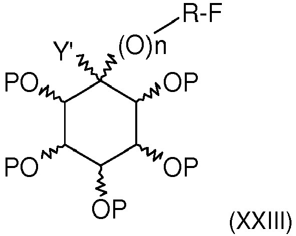 ou (XXVII) 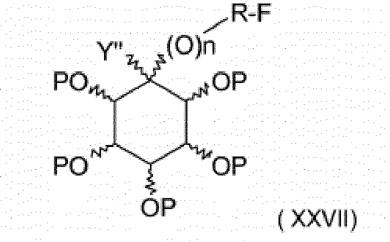 , dans lesquelles :
- n est un entier égal à 0 ou 1,
- Y' représente :
∘ quand n=0, un oxygène portant un groupement protecteur, ou,
∘ quand n=1, un atome d'hydrogène,
- Y" représente :
∘ quand n=0, un groupement OP ou,
∘ quand n=1, un atome d'hydrogène,
- R est une chaîne alkyle de 1 à 6 atomes de carbone,
- F est un atome de fluor radioactif ou non et
- P désigne un groupement protecteur.

14. Composé de formule (I) 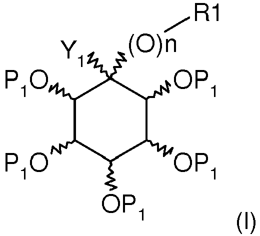 dans laquelle
- n est un entier égal à 0 ou 1,
- R1 représente
∘ quand n=0, une chaîne alkyle de 3 à 6 atomes de carbone, de préférence linéaire, une chaîne alkènyle, alkylène, une chaîne hydroxyalkyle linéaire de 2 à 6 atomes de carbone, la fonction hydroxyle étant éventuellement protégée ou l'oxygène de cette fonction étant substitué de manière à former un groupement partant, une chaîne halogénoalkyle, l'halogène étant radioactif ou non, de 2 à 6 atomes de carbone, ou un groupe fluorométhyle, le fluor étant radioactif ou non,
∘ quand n=1, une chaîne monohydroxyalkyle de 1 à 6 atomes de carbone, la fonction hydroxyle étant éventuellement protégée ou l'oxygène de cette fonction étant substitué de manière à former un groupement partant, ou monohalogénoalkyle, l'halogène étant radioactif ou non, de 1 à 6 atomes de carbone, l'halogène étant différent du brome,
- P₁ représente
∘ quand n=0 : chacun un hydrogène ou chacun groupement protecteur identique de la fonction hydroxyle
∘ quand n=1 et R1 est une chaîne monohydroxyalkyle : chacun un hydrogène ou chacun un groupement protecteur identique de la fonction hydroxyle,
∘ quand n=1 et R1 est une chaîne monohalogénoalkyle : chacun un hydrogène ou chacun un groupement protecteur identique de la fonction hydroxyle
- Y₁ représente :
∘ quand n=0, un groupement OP₁, où P₁ est tel que défini ci-avant, ou,
∘ quand n=1, un atome d'hydrogène
pour une utilisation comme médicament.

15. Composé de formule (I) 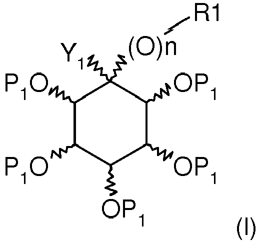 dans laquelle
- n est un entier égal à 0 ou 1,
- R1 représente
∘ quand n=0, une chaîne fluoroalkyle de 1 à 6 atomes de carbone, le fluor étant en position terminale sur la chaîne alkyle et radioactif,
∘ quand n=1, une chaîne fluoroalkyle de 1 à 6 atomes de carbone, le fluor étant en position terminale sur la chaîne alkyle et radioactif
- P₁ représente un groupement protecteur,
- Y₁ représente :
∘ quand n=0, un groupement OH, ou,
∘ quand n=1, un atome d'hydrogène,
pour une utilisation comme radiotraceur pour diagnostiquer en particulier les cancers.

## Patentansprüche

1. Verbindung der Formel (I): 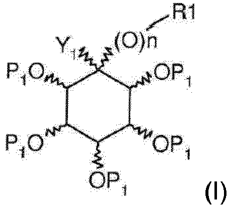 wobei:
- n eine Ganzzahl gleich 0 oder 1 ist,
- R1 darstellt:
∘ wenn n = 0, eine vorzugsweise lineare Alkylgruppe mit 3 - 6 Kohlenstoffatomen, eine Alkenyl-, Alkylen- oder lineare Hydroxyalkylgruppe mit 2 - 6 Kohlenstoffatomen, wobei der Hydroxylanteil ggf. geschützt ist oder der Sauerstoff dieses Anteils derart substituiert ist, dass sich eine Abgangsgruppe, eine Halogenoalkylgruppe mit 2 - 6 Kohlenstoffatomen, wobei das Halogen radioaktiv ist oder nicht, oder eine Fluormethylgruppe darstellt, wobei der Fluor radioaktiv ist oder nicht;
∘ wenn n = 1, eine Monohydroxyalkylgruppe mit 1 - 6 Kohlenstoffatomen, wobei der Hydroxylanteil in terminaler Position ggf. geschützt ist oder der Sauerstoff dieses Anteils derart substituiert ist, dass sich eine Abgangsgruppe bildet, oder eine Monohalogenoalkylgruppe mit 1 - 6 Kohlenstoffatomen, wobei das Halogen in terminaler Position radioaktiv ist oder nicht, wobei sich das Halogen vom dargestellten Brom Pi unterscheidet;
∘ wenn n = 0 : 2. jeweils einen Wasserstoff oder jeweils eine identische Schutzgruppe des Hydroxylanteils
∘ wenn n = 1 und R1 eine Monohydroxyalkylgruppe ist: jeweils einen Wasserstoff oder jeweils eine identische Schutzgruppe des Hydroxylanteils
∘ wenn n = 1 und R1 eine Monohalogenoalkylgruppe ist: jeweils einen Wasserstoff oder jeweils eine identische Schutzgruppe des Hydroxylanteils
- Y1 darstellt:
∘ wenn n = 0, eine OP15-Gruppe, wie sie oben definiert wird, oder
∘ wenn n = 1, ein Wasserstoffatom,
mit Ausnahme der nicht radioaktiv markierten 2-FluormethylmyoinositolVerbindungen, bei denen P1 identisch oder unterschiedlich ist und ein Wasserstoffatom oder eine Benzylschutzgruppe darstellt.

2. Verbindung der Formel (II) nach Anspruch 1, 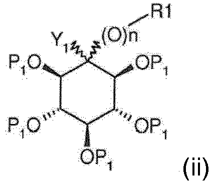 wobei n, R1, P1 und Y1 den jeweiligen Definitionen im Anspruch 1 entsprechen.

3. Verbindung nach Anspruch 1 oder 2, wobei R1 eine Fluoralkylgruppe, vorzugsweise mit 2 - 6 Kohlenstoffatomen, ist, wobei sich der Fluor in der terminalen Position an der Alkylgruppe befindet und nicht radioaktiv ist.

4. Verbindung nach Anspruch 1 oder 2, wobei R1 eine Fluoralkylgruppe, vorzugsweise mit 2 - 6 Kohlenstoffatomen, ist, wobei sich der Fluor in der terminalen Position an der Alkylgruppe befindet und radioaktiv ist.

5. Verbindung nach einem der Ansprüche 1 - 4, wobei P ein Wasserstoff ist.

6. Verbindung nach Anspruch 1 oder 2, wobei R1 eine Monohydroxyalkylgruppe mit 1 - 6 Kohlenstoffatomen, wobei der Hydroxylanteil terminal ist und der Sauerstoff dieses Anteils derart substituiert ist, dass sich eine Abgangsgruppe ergibt.

7. Verbindung nach einem der Ansprüche 2 - 6, wobei die Gruppe (O)n-R1 sich in der axialen Position befindet.

8. Verbindung nach einem der Ansprüche 2 - 6, wobei die Gruppe (O)n-R1 sich in der äquatorialen Position befindet.

9. Verfahren zur Synthese einer Verbindung der Formel (XI): 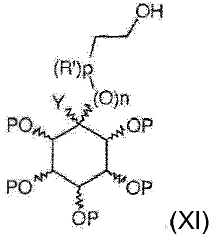 durch Hydroxylieren einer Verbindung der Formel (XII) 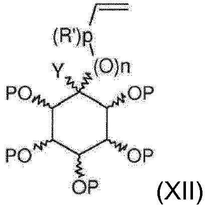 wobei:
- n eine Ganzzahl gleich 0 oder 1 ist,
- Y darstellt:
∘ wenn n = 0, eine OH-Gruppe oder,
∘ wenn n = 1, ein Wasserstoffatom,
- R1 eine Alkylgruppe mit 1 Kohlenstoffatom darstellt,
- p eine Ganzzahl zwischen 0 und 1 ist und
- P eine Schutzgruppe darstellt.

10. Verfahren zur Synthese einer Verbindung der Formel (XII): 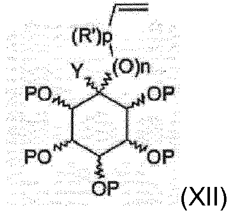 wobei:
- n = 0, Y eine OH-Gruppe ist,
- R' eine Alkylgruppe mit 1 Kohlenstoffatom ist,
- p eine Ganzzahl zwischen 0 und 1 ist und
- P eine Schutzgruppe darstellt, durch Umsetzen einer Verbindung der Formel (XV) oder (XV'): 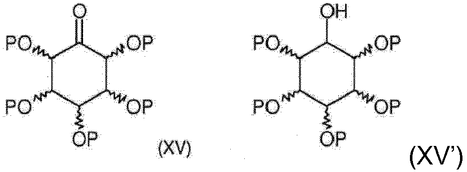
wobei P der obigen Beschreibung entspricht, mit einem Organomagnesium der Formel CH₂=CH-(R')p-MgBr für die Verbindung (XV), oder mit einem Alkenylbromid der Formel CH₂=CH-(R')p-Br für die Verbindung (XV').

11. Verfahren zur Synthese einer Verbindung der Formel (XXIII): 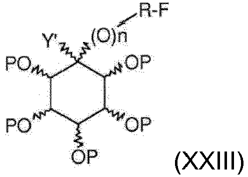 durch Fluorieren einer Verbindung der Formel (XII) 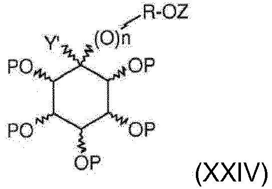 wobei:
- n eine Ganzzahl gleich 0 oder 1 ist,
- Y' darstellt:
∘ wenn n = 0, einen Sauerstoff mit einer Schutzgruppe oder,
∘ wenn n = 1, ein Wasserstoffatom,
- R eine Alkylgruppe mit 1 - 6 Kohlenstoffatomen darstellt,
- F ein nicht radioaktives Fluoratom ist,
- P eine Schutzgruppe darstellt und
- Z eine Tosylgruppe oder ein Wasserstoffatom darstellt.

12. Verfahren zur Synthese einer Verbindung der Formel (XXVII): 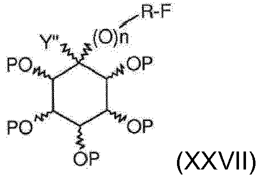 durch Fluorieren einer Verbindung der Formel (XXVII) 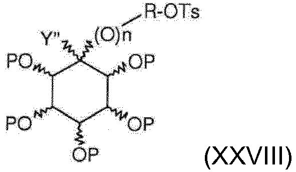 wobei:
- n eine Ganzzahl gleich 0 oder 1 ist,
- Y" darstellt:
∘ wenn n = 0, eine OP-Gruppe oder,
∘ wenn n = 1, ein Wasserstoffatom,
- R eine Alkylgruppe mit 1 - 6 Kohlenstoffatomen darstellt,
- F ein radioaktives Fluoratom ist,
- P eine Schutzgruppe darstellt und
- Ts eine Tosylgruppe darstellt.

13. Verfahren zur Herstellung einer Verbindung der Formel (XXXI) mit Ausnahme der nicht radioaktiv markierten 2- Fluormethylmyoinositolverbindungen bei denen P₁ identisch ist und ein Wasserstoffatom darstellt: 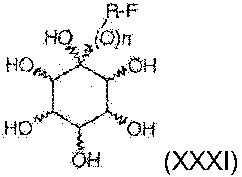 durch Entschützen einer Verbindung der Formel (XXIII) 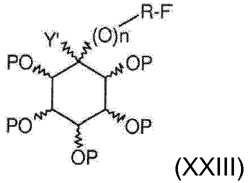 oder (XXVII) 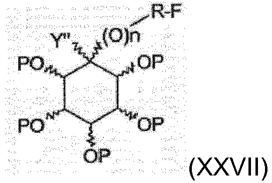 wobei:
- n eine Ganzzahl gleich 0 oder 1 ist,
- Y' darstellt:
∘ wenn n = 0, einen Sauerstoff mit einer Schutzgruppe oder,
∘ wenn n = 1, ein Wasserstoffatom,
- Y" darstellt:
∘ wenn n = 0, eine OP-Gruppe oder,
∘ wenn n = 1, ein Wasserstoffatom,
- R eine Alkylgruppe mit 1 - 6 Kohlenstoffatomen darstellt,
- F ein radioaktives oder nicht radioaktives Fluoratom und
- P eine Schutzgruppe darstellt.

14. Verbindung der Formel (I): 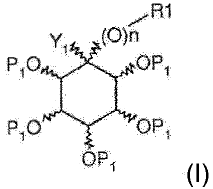 wobei:
- n eine Ganzzahl gleich 0 oder 1 ist,
- R1 darstellt:
∘ wenn n = 0, eine vorzugsweise lineare Alkylgruppe mit 3 - 6 Kohlenstoffatomen, eine Alkenyl-, Alkylen- oder lineare Hydroxyalkylgruppe mit 2 - 6 Kohlenstoffatomen, wobei der Hydroxylanteil ggf. geschützt ist oder der Sauerstoff dieses Anteils derart substituiert ist, dass sich eine Abgangsgruppe, eine Halogenoalkylgruppe mit 2 - 6 Kohlenstoffatomen, wobei das Halogen radioaktiv ist oder nicht, oder eine Fluormethylgruppe darstellt, wobei der Fluor radioaktiv ist oder nicht;
∘ wenn n = 1, eine Monohydroxyalkylgruppe mit 1 - 6 Kohlenstoffatomen, wobei der Hydroxylanteil ggf. geschützt ist oder der Sauerstoff dieses Anteils derart substituiert ist, dass sich eine Abgangsgruppe bildet, oder eine Monohalogenoalkylgruppe mit 1 - 6 Kohlenstoffatomen, wobei das Halogen radioaktiv ist oder nicht, wobei sich das Halogen vom dargestellten Brom unterscheidet;
- P₁ darstellt
∘ wenn n = 0: jeweils einen Wasserstoff oder jeweils eine identische Schutzgruppe des Hydroxylanteils
∘ wenn n = 1 und R1 eine Monohydroxyalkylgruppe ist: jeweils einen Wasserstoff oder jeweils eine identische Schutzgruppe des Hydroxylanteils
∘ wenn n = 1 und R1 eine Monohalogenoalkylgruppe ist: jeweils einen Wasserstoff oder jeweils eine identische Schutzgruppe des Hydroxylanteils
- Y₁ darstellt:
∘ wenn n = 0, eine OP1-Gruppe darstellt oder P1 der obigen Definition entspricht oder,
∘ wenn n = 1, ein Wasserstoffatom zur Verwendung als Arzneimittel.

15. Verbindung der Formel (I): 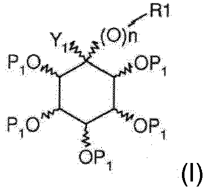 wobei:
- n eine Ganzzahl gleich 0 oder 1 ist,
- R1 darstellt:
∘ wenn n = 0, eine Fluoralkylgruppe mit 1 - 6 Kohlenstoffatomen, wobei der Fluor sich in der terminalen Position an der Alkylgruppe befindet und radioaktiv ist,
∘ wenn n = 1, eine Fluoralkylgruppe mit 1 - 6 Kohlenstoffatomen, wobei der Fluor sich in der terminalen Position an der Alkylgruppe befindet und radioaktiv ist
- und P1 eine Schutzgruppe darstellt,
- Y1 darstellt:
∘ wenn n = 0, eine OH-Gruppe oder,
∘ wenn n = 1, ein Wasserstoffatom,
zur Verwendung als Radionuklid zur Diagnose insbesondere von Krebserkrankungen.

## Claims

1. Compound of formula (I): 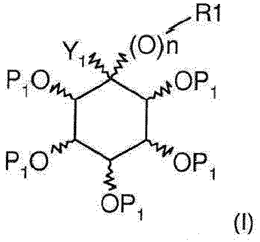 in which
- n is an integer equal to 0 or 1,
- R1 represents
∘ when n=0, an alkyl chain of 3 to 6 preferably linear carbon atoms, an alkenyl or alkylene chain, a linear hydroxyalkyl chain of 2 to 6 carbon atoms, wherein the hydroxyl function is optionally protected or the oxygen of this function is substituted in order to form a leaving group, a haloalkyl chain, wherein the halogen is radioactive or not and of 2 to 6 carbon atoms, or a fluoromethyl group, wherein the fluorine is radioactive or not
∘ when n=1, a monohydroxyalkyl chain of 1 to 6 carbon atoms, wherein the hydroxyl function, in the terminal position, is optionally protected or the oxygen of this function is substituted in order to form a leaving group, or monohaloalkyl, wherein the halogen, in the terminal position, is radioactive or not and of 1 to 6 carbon atoms, wherein the halogen is different from bromine
- P₁ represents
∘ when n=0: respectively a hydrogen atom or respectively an identical protective group of the hydroxyl function
∘ when n=1 and R1 is a monohydroxyalkyl chain: respectively a hydrogen atom or respectively an identical protective group of the hydroxyl function,
∘ when n=1 and R1 is a monohaloalkyl chain: respectively a hydrogen atom or respectively an identical protective group of the hydroxyl function
- Y₁ represents:
∘ when n=0, a OP₁ group, wherein P₁ is as defined above, or,
∘ when n=1, a hydrogen atom,
with the exception of non-radiolabeled 2-fluoromethyl-myo-inositols compounds for which an identical or different P₁ represents a hydrogen atom or a benzyl protecting group.

2. Compound according to claim 1 of formula (II): 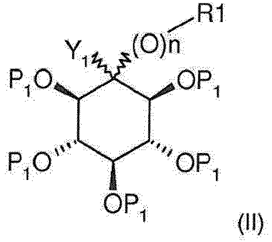 in which n, R1, P₁ and Y₁ are as defined in claim 1.

3. Compound according to claim 1 or 2, wherein R1 is a fluoroalkyl chain preferably of 2 to 6 carbon atoms, wherein the fluorine is in the terminal position on the alkyl chain and non-radioactive.

4. Compound according to claim 1 or 2, wherein R1 is a fluoroalkyl chain preferably of 2 to 6 carbon atoms, wherein the fluorine is in the terminal position on the alkyl and radioactive chain.

5. Compound according to any one of the claims 1 to 4 wherein P₁ is a hydrogen atom.

6. Compound according to claim 1 or 2, wherein R1 is a monohydroxyalkyl chain of 1 to 6 carbon atoms, wherein the hydroxyl function is in the terminal position and the oxygen of this function is substituted to form a leaving group.

7. Compound according to any one of the claims 2 to 6, wherein the (O)ₙ-R1 group is in the axial position.

8. Compound according to any one of the claims 2 to 6, wherein the (O)ₙ-R1 group is in the equatorial position.

9. Method for synthesizing a compound of formula (XI): 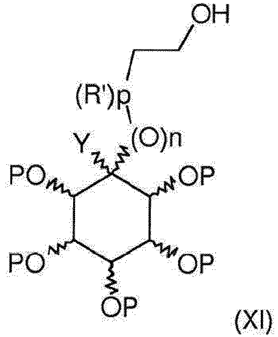 by hydroxylation of a compound of formula (XII): 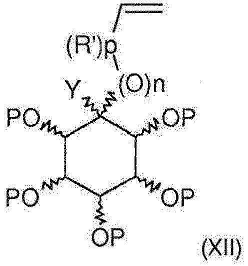 in which:
- n is an integer equal to 0 or 1,
- Y represents:
∘ when n=0, an OH group, or,
∘ when n=1, a hydrogen atom,
- R' represents an alkyl chain of 1 carbon atom,
- p is an integer between 0 and 1, and
- P represents a protective group.

10. Method for synthesizing a compound of formula (XII), 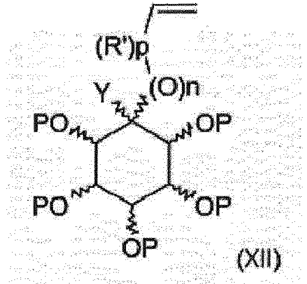 in which
- when n=0, Y is an OH group,
- R' is an alkyl chain of 1 carbon atom,
- P is an integer between 0 and 1, and P denotes a protective group, by reaction of a compound of formula (XV) or (XV'): 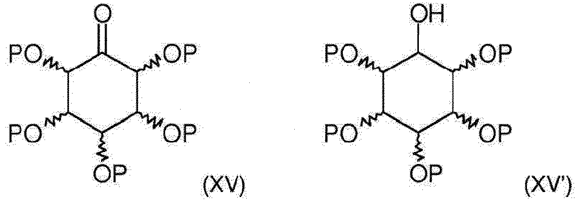
in which P is as described above, with an organomagnesium of formula CH₂=CH-(R')p-MgBr for the compound (XV), or with an alkenyl bromide of formula CH₂=CH-(R')p-Br for the compound (XV').

11. Method for synthesizing a compound of formula (XXIII): 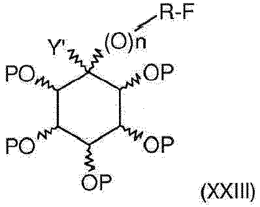 by fluorination of a compound of formula (XXIV): 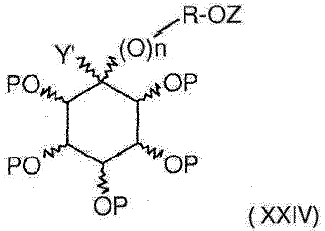 in which:
- n is an integer equal to 0 or 1,
- Y' represents:
∘ when n=9, an oxygen atom carrying a protective group, or,
∘ when n=1, a hydrogen atom,
- R represents an alkyl chain of 1 to 6 carbon atoms,
- F is a non-radioactive fluorine atom,
- P represents a protective group, and
- Z represents a tosyl group or a hydrogen atom.

12. Method for synthesizing a compound of formula (XXVII): 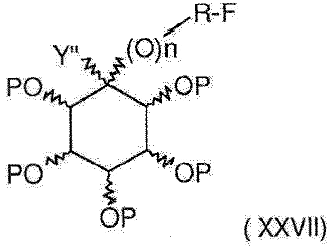 by fluorination of a compound of formula (XXVIII), 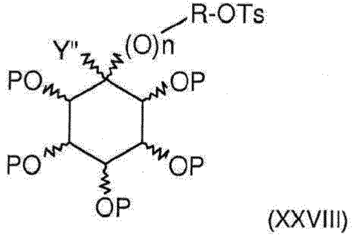 in which:
- n is an integer equal to 0 or 1,
- Y" represents:
∘ when n=0, an OP group or,
∘ when n=1, a hydrogen atom,
- R represents an alkyl chain of 1 to 6 carbon atoms,
- F is a radioactive fluorine atom,
- P represents a protective group, and
- Ts represents a tosyl group.

13. Method for synthesizing a compound of formula (XXXI) with the exception of non-radiolabeled 2-fluoromethyl-myo-inositols compounds for which P₁, identical, represents a hydrogen atom: 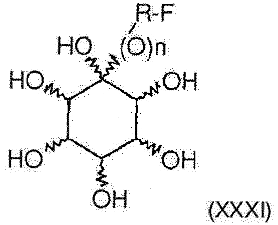 by deprotection of a compound of formula (XXIII) 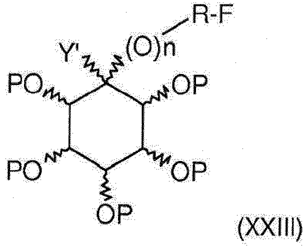 or (XXVII) 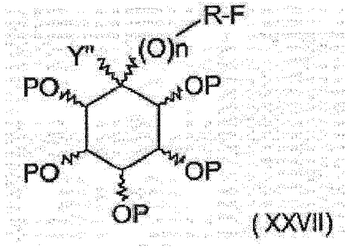 in which:
- n is an integer equal to 0 or 1,
- Y' represents:
∘ when n=0, an oxygen atom carrying a protective group, or,
∘ when n = 1, a hydrogen atom,
- Y" represents:
∘ when n=0, an OP group, or
∘ when n=1, a hydrogen atom,
- R is an alkyl chain of 1 to 6 carbon atoms,
- F is a radioactive fluorine atom or not, and
- P denotes a protective group.

14. Compound of formula (1): 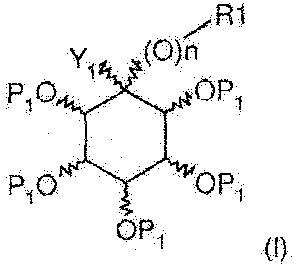 in which
- n is an integer equal to 0 or 1,
- R1 represents
∘ when n=0, an alkyl chain of 3 to 6 carbon atoms, preferably linear, an alkenyl or alkylene chain, a linear hydroxyalkyl chain of 2 to 6 carbon atoms, wherein the hydroxyl function is optionally protected or the oxygen of this function is substituted in order to form a leaving group, a haloalkyl chain, wherein the halogen is radioactive or not and of 2 to 6 carbon atoms, or a fluoromethyl group, wherein the fluorine is radioactive or not,
∘ when n=1, a monohydroxyalkyl chain of 1 to 6 carbon atoms, wherein the hydroxyl function is optionally protected or the oxygen of this function is substituted in order to form a leaving group, or monohaloalkyl, wherein the halogen is radioactive or not and of 1 to 6 carbon atoms, wherein the halogen is different from bromine,
- P₁ represents
∘ when n=0: respectively a hydrogen atom or respectively a protective group that is identical to the hydroxyl function
∘ when n=1 and R1 is a monohydroxyalkyl chain: respectively a hydrogen atom or respectively an identical protective group of the hydroxyl function,
∘ when n=1 and R1 is a monohaloalkyl chain: respectively a hydrogen atom or respectively an identical protective group of the hydroxyl function
- Y₁ represents:
∘ when n=0, an OP₁ group, wherein P₁ is as defined above, or,
∘ when n=1, a hydrogen atom
for use as a medicine.

15. Compound of formula (I): 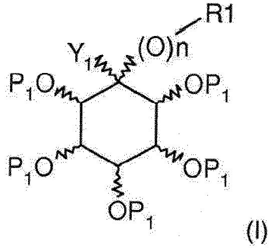 in which
- n is an integer equal to 0 or 1,
- R1 represents
∘ when n=0, a fluoroalkyl chain of 1 to 6 carbon atoms, wherein the fluorine is in the terminal position on the alkyl and radioactive chain,
∘ when n=1, a fluoroalkyl chain of 1 to 6 carbon atoms, wherein the fluorine is in the terminal position on the alkyl and radioactive chain
- P₁ represents a protective group,
- Y₁ represents:
∘ when n=0 an OH group, or,
∘ when n=1, a hydrogen atom,
for use as a radiotracer for diagnosing cancers in particular.
